(19) Europäisches Patentamt
European Patent Office
Office européen des brevets

(11) EP 1 624 059 A2

(12) **EUROPEAN PATENT APPLICATION**

(43) Date of publication:
**08.02.2006 Bulletin 2006/06**

(21) Application number: **03257914.6**

(22) Date of filing: **16.12.2003**

(51) Int Cl.:
*C12N 15/11* (2006.01) *C12P 19/34* (2006.01)
*C07H 21/00* (2006.01)

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **18.12.2002 US 324409**

(71) Applicant: **Agilent Technologies, Inc.**
**Palo Alto, CA 94306 (US)**

(72) Inventors:
- **Sampson, Jeffrey R.**
**San Francisco, CA 94127 (US)**
- **Ach, Robert A.**
**San Francisco, CA 94131 (US)**
- **Wolber, Paul**
**Los Altos, CA 94011 (US)**

(74) Representative: **Tollett, Ian et al**
**Williams Powell**
**Morley House**
**26-30 Holborn Viaduct**
**London EC1A 2BP (GB)**

# (54) Method of producing nucleic acid molecules with reduced secondary structure

(57) The present invention provides a system for generating nucleic acid molecules having a reduced ability to hybridize to form intermolecular and intramolecular base pairs between regions of substantial complementarity as compared with nucleic acid molecules having the same nucleotide sequence containing naturally-occurring nucleotides. Furthermore, nucleic acid molecules of the present invention are characterized by the ability to form intermolecular base pairs with other nucleic acid molecules of choice.

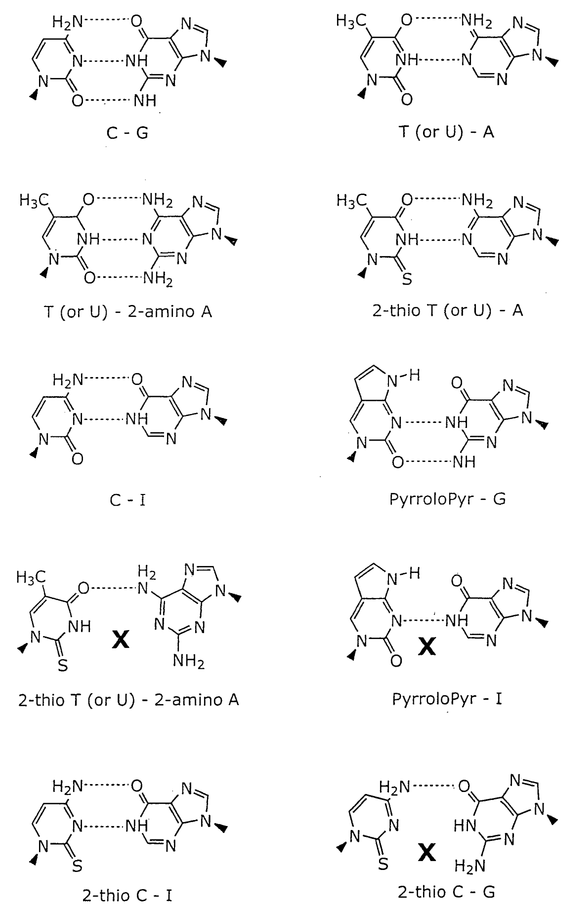

C - G

T (or U) - A

T (or U) - 2-amino A

2-thio T (or U) - A

C - I

PyrroloPyr - G

2-thio T (or U) - 2-amino A

PyrroloPyr - I

2-thio C - I

2-thio C - G

FIG. 1

EP 1 624 059 A2

## Description

The present invention relates to nucleic acid molecules having reduced secondary structure and to a method of generating said nucleic acid molecules.

The present application claims priority to co-pending application USSN 09/632,639, filed July 31,2000, and USSN 09/358,141, filed July 20, 1999. Each of these patent applications is incorporated herein by reference in its entirety.

Naturally occurring ribonucleic acid (RNA) and deoxyribonucleic (DNA) acid molecules contain nucleotides which are capable of forming base pairs through hydrogen bond interactions with complementary nucleotides, according to the "Watson-Crick" scheme. In naturally occurring RNA, these nucleotides are adenine (rA), uracil (rU), guanine (rG) and cytosine (rC). In naturally occurring DNA, these nucleotides are adenine (dA), thymine (dT), guanine (dG) and cytosine (dC). According to the Watson-Crick scheme, adenine nucleotides in a nucleic acid molecule form base pairs through two hydrogen bonds with thymine nucleotides in DNA (A/T) and uracil nucleotides in RNA (A/U). For G/C base pairs, guanine nucleotides form base pairs through three hydrogen bonds with cytosine nucleotides (G/C) in both DNA and RNA.

Where the nucleotide sequences permit, base pairing can form between two nucleic acid molecules (intermolecular) resulting in a double-helical structure. This can occur between two molecules of DNA, two molecules of RNA or between one molecule of DNA and one molecule of RNA. In addition, base pairs can form between two regions within a single molecule of DNA or RNA (intramolecular secondary structure) where the two regions contain complementary or substantially complementary sequences that permit the formation of base pairs. If two regions of complementarity in one molecule containing nucleotide sequences between them, hybridization of the two regions together results in the formation of a loop. Base-pairing hybridization between two regions of complementarity results in a hairpin structure if there are few or no nucleotides between the two regions. In addition, nucleic acid molecules can form three-way junctions and four-way junctions.

Loops, duplexes, hairpin structures, three-way junctions, and four-way junctions are a few examples of secondary structures in nucleic acid molecules. Secondary structure in a nucleic acid molecule can effectively block hybridization of a nucleic acid molecule having substantially complementary sequence. As a result, secondary structure limits the sensitivity, specificity and utility of applications that rely upon hybridization of probes used to analyze nucleic acid molecules of a particular sequence. Such applications include solution hybridization methods (e.g. mRNA fluorescence in situ hybridization; FISH) and surface-bound oligonucleotide arrays (e.g. DNA array technology) for sequence analysis (Pease, A.C., *et al.,* Proc. Natl. Acad. Sci USA, 11, 502205026 (1994), Dramanac, S., *Nature Biotechnology,* 16, 54-58 (1998) and US patents; US520223, US5667972, US6270961, US6270961 and US6018041) mutation detection (Chee, M., *et al.,* (1996) *Science* 274, 610-614, Wang, et. al., *Science,* 280, 1077-1082) gene expression assays (Lockart, D., *et al.,* (1996) *Nat. Biotech.* 14, 1675-1680), and comparative genomic hybridization (see Sudar, D. *et al., Science,* 258 (5083), 818-821 (1992); Pinkel, *D. et al., Nature Genetics,* 20(2), 207-211 (1998), and US Patent No. 5,830,645).

The problem of target secondary structure is particularly acute for those methods that utilize relatively short oligonucleotide probes since the shorter probes are less effective in competing with the intramolecular base-pairing interactions (e.g. US Pat 5,605,798, PCT publications WO92/15712, WO97/35033). These methods include but are not limited to polymerase extension assays (PEA; US Pat 5,595,890; US Pat 5,534,424; US Pat 5,605,798) and oligonucleotide ligation assays (US Pat 4,988,617; US Pat 5,494,810; WO 95/04160).

Solutions to the problem of utilizing oligonucleotide probes to study nucleic acid molecules having secondary structure have been met with only limited success. One attempt to overcome this problem entails performing the hybridization of the oligonucleotide probe to the region having secondary structure in the presence of mild denaturants and/or at elevated temperatures. However, these treatments also result in decreasing the stability of the interaction between the target nucleic acid molecule and the probe (and therefore, overall sensitivity of the assay) due to the decrease hydrogen bond interactions in the entire system.

Although employing longer oligonucleotide probes can compensate for a lowered duplex stability, the use of longer probes can lead to a decrease in sequence specificity which is undesirable. One problem is that the decrease in specificity severely limits the ability of a longer probe to distinguish between polynucleotides that may differ in sequence by only one nucleotide. Since many genetic diseases are caused by single nucleotide mutations, the inability of a longer probe to be highly sequence specific severely limits assays where the goal is to detect single nucleotide changes within the target sequence.

Fragmentation of nucleic acid molecules can also reduce secondary structures that involve intramolecular base pairing between two regions that are separated by large distances (Lockart, D., *et al.,* (1996) *Nat. Biotech.* 14, 1675-1680). However, this does not eliminate secondary structure in the form of stable hairpins resulting from short-range base-pairing interactions. Moreover, because the detection labels are generally incorporated at multiple internal positions within the target sequence, fragmentation of the target will effectively reduce the target's overall label specific activity ([label]:[target]) thereby reducing the detection limit of the assay.

Other attempts to reduce the secondary structure in nucleic acid molecules involve the use of chemical modifications to the molecule. For example, methylmercuric hydroxide reacts with the imino bonds of C and G making them unable to base pair with their natural unmodified complements. However, clearly such modifications will also prevent hybridization of the target with its complementary oligonucleotide probe. In addition, chemical modifications of nucleic acids require the use of volatile and toxic reagents which makes the use of this method undesirable.

Numerous methodologies in molecular biology, biochemistry and biophysics rely on the hybridization of a nucleic acid molecule to another nucleic acid molecule. These methods include Southern and Northern blot hybridization, fluorescence in situ hybridization (FISH), gene-chip array technologies, and polymerase chain reactions (PCR). One goal of these techniques is to determine the presence and/or amounts of nucleic acid molecules containing nucleotide sequences of interest. In general, nucleic acid molecules are labeled with a detectable marker such as a radioactive or a fluorescent marker. After sequence-specific hybridization of two molecules (target and probe), the presence and/or levels of a sequence of interest is measured using the radioactive or fluorescent marker.

Error in the measurement of the amounts of nucleic acid molecules having sequences of interest occurs when target-target or probe-probe intermolecular *cross-hybridization* affects the measurement of target-to-probe hybridization. The problem of intermolecular cross-hybridization is the undesirable binding of target-to-target or probe-to-probe nucleic acid molecules to each other, and affects all analytical methods that are based upon the specific binding of complementary nucleic acid sequences. For example, in DNA chip technology as depicted in Figure 23, sample nucleic acids (targets) can bind to surface-bound or solution probe sequences indirectly through hybridization to an intermediate molecule resulting in the incorrect identification of the probe sequence. Examples of intermolecular *cross-hybridization* include binding between two nucleic acid molecules with a low degree of complementary sequences and binding of a target sequence to another target sequence which already bound to a probe. Therefore, intermolecular *cross-hybridization* can lead to inaccurate measurements resulting from a target binding to a probe through an intermediate molecule instead of hybridizing directly to the probe.

The control of intermolecular cross-hybridization is particularly important for methods that employ massively parallel arrays of hybridization probes (DNA microarray methods). Such arrays depend solely upon hybridization for specificity since there is no enzyme-based proofreading of duplexes as in methods based upon Sanger dideoxy sequencing or the polymerase chain reaction. In addition, the large number of probes reduces the ability to verify the specificity of all probe-target interactions.

Intermolecular cross-hybridization to nearly-complementary nucleic acid sequences can be controlled by using standard sequence homology search tools (e.g. BLAST) to avoid probe sequences that are close matched to many sequences potentially present in the sample of interest. However, the problem of indirect intermolecular cross-hybridization in DNA microarray experiments has only recently been recognized, and is not solvable by homology methods. Indirect intermolecular cross-hybridization arises when the intended target of a given probe serves as a bridge to another, unintended target (Figure 23). This phenomenon can occur at more than one point along the target nucleic acid chain, and gives rise to an intermolecular cross-hybridization signal that is a nearly constant fraction of the specific target signal. Unfortunately, methods to reduce intermolecular *cross-hybridization* such as homology searching cannot ameliorate the problems associated with this form of cross-hybridization since such *cross-hybridization* depends upon the sequence of the target, and not the probe.

As for fragmentation of secondary structures that involve intramolecular base-pairing, fragmentation of the target reduces intermolecular *cross-hybridization* to some degree since shorter targets offer fewer secondary sites for binding by another target molecule. However, fragmentation also decreases the signal for detection of target molecules (e.g. if the target has been randomly labeled with a reporter molecule), and increases the complexity of sample preparation protocols. Fragmentation is particularly problematic in differential expression studies where two samples labeled with different reporter molecules must be reproducibly and identically fragmented.

Therefore, there is a need for methods that reduce intramolecular and intermolecular hybridization that affect the measurement of levels target-to-probe hybridization of nucleic acid molecules.

The present invention provides a system for the production of nucleic acid molecules with reduced levels of intramolecular base pairing (secondary structure) and intermolecular base pairing by generating unstructured nucleic acids (UNAs). The inventive system allows for the production of nucleic acid molecules with reduced levels of intramolecular or intermolecular base pairing as compared with nucleic acid molecules of substantially identical sequence comprising all naturally-occurring nucleotides. Preferably, nucleic acid molecules of the present invention having reduced levels of secondary structure are capable of hybridizing to other nucleic acid molecules having substantially complementary nucleotide sequences or regions of substantially complementary sequences. In addition, the nucleic acids having reduced levels of secondary structure have reduced levels of *cross-hybridization* to sequences that are not the sequence of interest. The present invention also provides methods of using of UNAs.

In one aspect, the present invention provides methods of producing nucleic acid molecules with reduced levels of intramolecular base pairing (UNAs) by incorporating pairs of complementary nucleotides (A and T or G and C (or both)) that are unable to form stable base pairs. The nucleotides of each pair may include one natural nucleotide and one modified nucleotide or two modified nucleotides. In another aspect, the present invention provides methods for producing nucleic acids that have reduced base-pairing stability with another UNA molecule. Preferably, the UNA pairs are positioned in nucleic acid molecules of the present invention in sequence elements of substantially complementary sequence to reduce intramolecular or intermolecular base pairing. Also preferably, the UNA molecules are able to form stable base pairs and therefore stable intermolecular hybrids with other types of nucleic acid molecules composed of defined natural and/or modified nucleotides.

Nucleic acid molecules with reduced levels of intramolecular base pairing or intermolecular base pairing are produced by any method, e.g., by chemical synthesis or enzymatic synthesis. Preferably, nucleic acid molecules of the present invention are ribonucleotides or deoxyribonucleotides. Preferably, the nucleic acid molecules of the present invention are produced enzymatically. For example, RNA molecules with reduced levels of secondary structure are produced by an RNA polymerase, a reverse transcriptase, a ribozyme or a self-replicating RNA molecule, and DNA molecules with reduced levels of secondary structure are produced by a DNA polymerase. In one aspect of the invention, UNAs are synthesized enzymatically to produce UNA molecules having lengths greater than UNAs synthesized by current conventional chemical methodologies. UNAs have a reduced ability to hybridize to other UNAs as compared to the levels of hybridization between natural nucleic acid molecules having the same nucleotide sequence. Moreover, UNAs of the present invention maintain the ability to hybridize to non-UNA molecules, preferably naturally-occurring nucleic acid molecules. Therefore, intermolecular hybridization between UNA molecules is reduced, but intermolecular hybridization between a UNA and a naturally-occurring nucleic acid molecule (or a non-UNA molecule) is maintained or even increased. In another aspect of the present invention, UNAs are synthesized enzymatically with a reduce ability to hybridize to itself through formation of intramolecular hydrogen-bonded base pairs. These UNAs therefore have reduced levels of secondary structure. Thus, UNAs according to the present invention include UNAs that have both reduced levels of secondary structure and reduced levels of intermolecular hybridization between UNAs. UNAs as referred to herein, include both RNA UNAs and DNA UNAs.

In a preferred embodiment, nucleic acid molecules of the present invention are produced by incorporating at least one modified purine nucleotide and at least one natural pyrimidine nucleotide; at least one natural purine nucleotide and at least one modified pyrimidine nucleotide; or at least one modified purine and at least one modified pyrimidine nucleotide such that at least one complementary purine/pyrimidine base pair does not form a stable hydrogen bonded base pair. Preferably, modified nucleotides are positioned in nucleic acid molecules of the present invention in sequence elements of substantially complementary sequence to reduce intramolecular or intermolecular base pairing. As described herein, a sequence element is part or all of a polynucleotide molecule consisting of at least one nucleotide. According to the invention, complementary sequence elements in different nucleic acid molecules or the same nucleic acid molecule may hybridize to one another.

In another preferred embodiment wherein the UNAs are produced enzymatically, nucleic acid molecules are produced by incorporating the deoxy- or ribo versions of nucleotides 2-aminoadenosine, 2-thiothymidine, inosine (I), pyrrolo-pyrimidine (P), 2-thiocytidine, adenine (A), thymine (T), guanine (G), cytosine (C) and combinations thereof such that at least two complementary nucleotides that are not capable of forming a base pair are present. Any form of the deoxy- or ribonucleotides that can be enzymatically incorporated is within the scope of the present invention. Preferably the deoxy- or ribo- nucleotides are nucleotide triphosphates.

In yet another preferred embodiment, nucleic acid molecules ofthe present invention are produced by incorporating the deoxy- or ribo- versions of nucleotides 2-aminoadenosine, 2-thiothymidine, guanine (G), cytosine (C) and combinations thereof such that 2-aminoadenosine is present in a first sequence element and 2-thiothymidine is present in a second sequence element substantially complementary to the first sequence element.

In yet another preferred embodiment, nucleic acid molecules of the present invention are produced by incorporating the deoxy- or ribo- versions of nucleotides inosine (I), pyrrolo-pyrimidine (P), adenine (A), thymine (T), and combinations thereof such that inosine is present in a first sequence element and pyrrolo-pyrimidine is present in a second sequence element substantially complementary to the first sequence element.

In yet another preferred embodiment, nucleic acid molecules of the present invention are produced by incorporating the deoxy- or ribo- nucleotides 2-thiocytidine, guanine (G), adenine (A), thymine (T), and combinations thereof such that 2-thiocytidine is present in a first sequence element and guanine is present in a second sequence element substantially complementary to the first sequence element.

In yet another preferred embodiment, nucleic acid molecules of the present invention are produced by incorporating the deoxy- or ribo- versions of nucleotides 2-aminoadenosine, 2-thiothymidine, inosine (I), pyrrolo-pyrimidine (P), and combinations thereof such that complementary sequence elements are unable to form stable base pairs.

In yet another preferred embodiment, nucleic acid molecules of the present invention are produced by incorporating the deoxy- or ribo- versions of nucleotides 2-aminoadenosine, 2-thiothymidine, 2-thiocytidine, guanine (G), and combinations thereof such that complementary sequence elements are unable to form stable base pairs.

The present invention also provides for deoxy- or ribo- nucleic acid having reduced secondary structure relative to a deoxy- or ribo- nucleic acid of substantially identical nucleotide sequence having naturally occurring bases, wherein the unstructured nucleic acid has at least two sequence elements that are substantially complementary, wherein the substantially complementary sequence elements do not form intramolecular base pairs, wherein at least one sequence element of at least two complementary sequence elements is capable of hybridizing to a substantially complementary sequence in another nucleic acid molecule.

The present invention also provides for unstructured nucleic acid molecules (DNA UNAs or RNA UNAs) synthesized enzymatically having reduced levels of intermolecular *cross-hybridization* and/or intramolecular hybridization relative to nucleic acid molecules of substantially identical nucleotide sequence having naturally occurring nucleotides, wherein the UNA has at least one sequence element that is substantially complementary to a second sequence element in a second nucleic acid molecule or in itself, wherein the substantially complementary sequence elements have reduced levels of hybridization, and wherein at least one of the two sequence elements is capable of hybridizing to a substantially complementary sequence in a non-UNA, a naturally-occurring nucleic acid molecule, or a UNA having modified nucleotides that specifically permit hybridization.

In a preferred embodiment, unstructured nucleic acids includes nucleotides such as 2-aminodeoxyadenosine 5'-triphosphate, 2-thiodeoxythymidine 5'-triphosphate, deoxyinosine 5'-triphosphate, deoxypyrrolopyrimidine 5'-triphosphate, 2-thiodeoxycytidine 5'-triphosphate, deoxyguanosine 5'-triphosphate, deoxycytidine 5'-triphosphate, deoxyadenosine 5'-triphosphate, deoxythymidine 5'-triphosphate, and combinations thereof.

In yet another preferred embodiment, unstructured nucleic acids of the present invention are at least 40 nucleotides in length.

In yet another preferred embodiment, unstructured nucleic acids of the present invention are at least 100 nucleotides in length.

In yet another preferred embodiment, unstructured nucleic acids of the present invention are at least 500 nucleotides in length.

In yet another preferred embodiment, unstructured nucleic acid molecules of the present invention are used in applications including but not limited to solution, bead-based and array-based ligase assays, solution, bead-based and array-based polymerase or primer extension assays, and solution, bead-based and array-based hybridization assays.

The present invention also provides for a method of producing an unstructured nucleic acid, comprising the steps of i) providing a nucleic acid template including a first nucleic acid sequence and a second nucleic acid sequence substantially complementary to the first nucleic acid sequence; ii) providing nucleotide triphosphates to produce the unstructured nucleic acid which is complementary with the first and second nucleic acid sequences, the nucleotide triphosphates being unable to hybridize with complementary nucleotide triphosphates; and iii) contacting the nucleic acid template and the nucleotide triphosphates with an enzyme under conditions such that the unstructured nucleic acid is produced.

The present invention also provides for a method described in the preceding paragraph wherein the nucleotide triphosphates comprise at least two nucleotides capable of being incorporated enzymatically into a polynucleotide, and wherein the at least two nucleotide triphosphates are unable to form intramolecular base pair but can form intermolecular base pairs.

The present invention also provides for a method described in the two preceding paragraphs, wherein the sequences complementary to the first nucleic acid sequence and the one another.

The present invention also provides for a method described in the three preceding paragraphs, wherein step ii) comprises providing a) 2-aminodeoxyadenosine 5'-triphosphate, 2-thiodeoxythymidine 5'-triphosphate, deoxyinosine 5'-triphosphate, deoxypyrrolopyrimidine 5'-triphosphate, 2-thiodeoxycytidine 5'-triphosphate, deoxyguanosine 5'-triphosphate, deoxycytidine 5'-triphosphate, deoxyadenosine 5'-triphosphate, deoxythymidine 5'-triphosphate; or b) 2-aminodeoxyadenosine 5'-triphosphate, 2-thiodeoxythymidine 5'-triphosphate, deoxyinosine 5'-triphosphate, deoxypyrrolopyrimidine 5'-triphosphate; or c) 2-aminodeoxyadenosine 5'-triphosphate, 2-thiodeoxythymidine 5'-triphosphate, deoxyguanosine 5'-triphosphate, 2-thiodeoxycytidine 5'-triphosphate; or d) 2-aminodeoxyadenosine 5'-triphosphate, 2-thiodeoxythymidine 5'-triphosphate, deoxyguanosine 5'-triphosphate, deoxycytidine 5'-triphosphate; or e) deoxyinosine 5'-triphosphate, deoxypyrrolopyrimidine 5'-triphosphate, deoxyadenosine 5'-triphosphate, deoxythymidine 5'-triphosphate; or f) 2-thiodeoxycytidine 5'-triphosphate, deoxyguanosine 5'-triphosphate, deoxyadenosine 5'-triphosphate, deoxythymidine 5'-triphosphate; or g) mixtures thereof.

The present invention also provides for a method described in the four preceding paragraphs, wherein the enzyme is selected from the group consisting of: RNA polymerase, DNA polymerase, reverse transcriptase, ribozymes, self-replicating RNA molecules and mixtures thereof.

The present invention also provides for an unstructured nucleic acid with reduced secondary structure relative to a nucleic acid of substantially identical nucleotide sequence having naturally occurring nucleotides, wherein the unstructured nucleic acid has at least two sequence elements that are complementary, wherein the complementary sequence elements do not form intramolecular base pairs, wherein at least one sequence element of at least two complementary sequence elements is capable of hybridizing to a substantially complementary sequence in another nucleic acid.

The present invention also provides for an unstructured nucleic acid described in the preceding paragraph, wherein the nucleic acid is synthesized by an enzyme, optionally a enzyme such as RNA polymerase, DNA polymerase, reverse transcriptase, ribozymes and self replicating RNA molecules.

The present invention also provides for an unstructured nucleic acid described in the preceding two paragraphs, wherein the nucleic acid is at least 40 nucleotides in length, alternatively at least 100 nucleotides in length or alternatively at least 500 nucleotides in length.

The present invention also provides for an unstructured nucleic acid described in the preceding three paragraphs, wherein the nucleic acid comprises 2-aminodeoxyadenosine 5'-triphosphate, 2-thiodeoxythymidine 5'-triphosphate, deoxyinosine 5'-triphosphate, deoxypyrrolopyrimidine 5'-triphosphate, 2-thiodeoxycytidine 5'-triphosphate, deoxyguanosine 5'-triphosphate, deoxycytidine 5'-triphosphate, deoxyadenosine 5'-triphosphate, deoxythymidine 5'-triphosphate, or mixture thereof.

The present invention also provides for an unstructured nucleic acid described in any of the preceding four paragraphs producible by the methods described in any of the preceding nine paragraphs.

The present invention also provides nucleotides or nucleosides that are labeled with a detectable chemical moiety. According to the present invention, the labeled nucleotide or nucleoside can be a modified or a naturally occurring nucleotide.

In yet another aspect, the present invention provides kits comprising nucleotide triphosphates, at least one enzyme capable of polymerizing the nucleotide triphosphates into polynucleotide molecules, buffer solutions, and optionally containing nucleic acid arrays. In a preferred embodiment, the kit comprises the nucleotide triphosphates 2-aminodeoxyadenosine 5'-triphosphate, 2-thiodeoxythymidine 5'-triphosphate, deoxyinosine 5'-triphosphate, deoxypyrrolopyrimidine 5'-triphosphate, 2-thiodeoxycytidine 5'-triphosphate, deoxyguanosine 5'-triphosphate, deoxycytidine 5'-triphosphate, deoxyadenosine 5'-triphosphate, deoxythymidine 5'-triphosphate, and combinations thereof; a DNA polymerase, or an RNA polymerase; buffer solutions and optionally a nucleic acid array.

## Definitions

Naturally occurring nucleotides or nucleosides are defined for the purposes of the present invention as adenine (A), thymine (T), guanine (G), cytosine (C), and uracil (U). The structures of A, T, G and C are shown in Figure 1. For RNA, uracil (U) replaces thymine. Uracil (structure not shown) lacks the 5-methyl group of T. It is recognized that certain modifications of these nucleotides or nucleosides occur in nature. However, for the purposes of the present invention, modifications of A, T, G, C, and U that occur in nature that affect hydrogen bonded base pairing are considered to be non-naturally occurring. For example, 2-aminoadenosine is found in nature, but is not a "naturally occurring" nucleotide or nucleoside as that term is used herein. Other non-limiting examples of modified nucleotides or nucleosides that occur in nature that do not affect base pairing and are considered to be naturally occurring are 5-methylcytosine, 3-methyladenine, O(6)-methylguanine, and 8-oxoguanine.

Nucleotides or nucleosides may be defined for purposes of the present invention as nitrogenous nucleotides or nucleosides derived from purine or pyrimidine. Modified nucleotides or nucleosides (excluding A, T, G, C, and U) include for example, nucleotides or nucleosides having a structure derived from purine or pyrimidine (i.e. nucleotide or nucleoside analogs). For example without limitation, a modified adenine may have a structure including a purine with a nitrogen atom covalently bonded to C6 of the purine ring as numbered by conventional nomenclature known in the art. In addition, it is recognized that modifications to the purine ring and/or the C6 nitrogen may also be included in a modified adenine. A modified thymine may have a structure comprising at least a pyrimidine, an oxygen atom covalently bonded to the C4 carbon, and a C5 methyl group. Again, it is recognized by those skilled in the art that modifications to the pyrimidine ring, the C4 oxygen and/or the C5 methyl group may also be included in a modified adenine. Derivatives of uracil may have a structure comprising at least a pyrimidine, an oxygen atom convalently bonded to the C4 carbon and no C5 methyl group. For example without limitation, a modified guanine may have a structure comprising at least a purine, and an oxygen atom covalently bonded to the C6 carbon. A modified cytosine has a structure including a pyrimidine and a nitrogen atom covalently bonded to the C4 carbon. Modifications to the purine ring and/or the C6 oxygen atom may also be included in modified guanine nucleotides or nucleosides. Modifications to the pyrimidine ring and/or the C4 nitrogen atom may also be included in modified cytosine nucleotides or nucleosides.

Some examples of modified nucleotides include, without limitation, 2'-deoxynucleotides include 2'-deoxyadenosine-monophosphate (dAMP), 2'-deoxyguanosine-monophosphate (dGMP), 2'-deoxycytidine-monophosphate (dCMP), 2'-deoxythymidine-monophosphate (thymidine) (dTMP or TMP), 2-amino-2'-deoxyadenosine-monophosphate, 2-thio-2'deoxythymidine-monophosphate (or 2-thiothymidine-monophosphate), 2-thio-2'-deoxycytidine-monophosphate, 2'-deoxyinosine-monophosphate, 2'-deoxypyrrolopyrimidine-monophosphate (dPMP).

Analogs may also be derivatives of purines without restrictions to atoms covalently bonded to the C6 carbon. These analogs would be defined as purine derivatives. Analogs may also be derivatives of pyrimidines without restrictions to atoms covalently bonded to the C4 carbon. These analogs would be defined as pyrimidine derivatives. The present invention includes purine analogs having the capability of forming stable base pairs with pyrimidine analogs without limitation to analogs of A, T, G, C, and U as defined. The present invention also includes purine analogs not having the capability of forming stable base pairs with pyrimidine analogs without limitation to analogs of A, T, G, C, and U.

Complementary nucleotides are defined according to the Watson-Crick definition for base pairing. Adenine nucleotide is complementary to thymine nucleotide and forms a stable base pair. Guanine nucleotide is complementary to cytosine nucleotide and forms a stable base pair. The base-pairing scheme is depicted in Figure 1. Complementation of modified nucleotide analogs is defined according to the parent nucleotide. Complementation of modified nucleotides does not require the ability to form stable hydrogen bonded base pairs. In other words, two modified nucleotides may be complementary according to the identity of the modified nucleotide but may not form a stable base pair. Complementation of nucleotide analogs which are not considered derivatives of A, T, G, C or U is defined according to an ability to form a stable base pair with a nucleotide or analog thereof. For example, a particular derivative of C (i.e. 2-thiocytosine) may not form a stable base pair with G, but is still considered complementary.

In addition to purines and pyrimidines, modified nucleotides or analogs, as those terms are used herein, include any compound that can form a hydrogen bond with one or more naturally occurring nucleotides or with another nucleotide analog. Any compound that forms at least two hydrogen bonds with T (or U) or with a derivative of T or U is considered to be an analog of A or a modified A. Similarly, any compound that forms at least two hydrogen bonds with A or with a derivative of A is considered to be an analog ofT (or U) or a modified T or U. Similarly, any compound that forms at least two hydrogen bonds with G or with a derivative of G is considered to be an analog of C or a modified C. Similarly, any compound that forms at least two hydrogen bonds with C or with a derivative of C is considered to be an analog of G or a modified G. It is recognized that under this scheme, some compounds will be considered for example to be both A analogs and G analogs (purine analogs) or both T/U analogs and C analogs (pyrimidine analogs).

A stable natural base pair is defined as two bases that can interact through the formation of at least two hydrogen bonds. Alternatively or additionally, a stable base pair may be defined as two bases that interact through at least one, preferably two, hydrogen bonds that promote base stacking interactions and therefore, promotes duplex stability. The present invention also considers base-pairing interactions that are stabilized by forces other than hydrogen bonding, which promote base stacking interactions and therefore, promotes duplex stability. These base-pairing interactions include hydrophobic interactions (Wu, Y.Q., J. American Chemical Society, 122, 7621-7632 (2000)), metal-mediated interactions (Atwell, S., J. American Chemical Society, 123, 12364-12367 (2001)), and other non-polar interactions (Guckian K.M., *Nature Structural Biology,* 5:954-959 (1998)).

A sequence element is defined as part or all of a polynucleotide molecule consisting of at least one nucleotide. Preferably, a sequence contains at least three contiguous nucleotides. More preferably, a sequence element contains at least six contiguous nucleotides. Nucleic acid sequence is defined by the identity of the nucleotides in a polynucleotide molecule. A single nucleic acid may have multiple sequence elements. Substantially complementary sequences within a nucleic acid molecule may hybridize to form intramolecular base pairs resulting in a secondary structure, such as a loop. Substantially complementary sequence elements between different nucleic acid molecules may also hybridize to form intermolecular base pairs resulting in a double helical structure.

For purposes of the present invention, two sequence elements are considered substantially complementary if at least 50% of the nucleotides in the two elements can form stable hydrogen bonds. Preferably, sequence elements are considered substantially complementary if at least 75% of the nucleotide sequence in the two elements are complementary. More preferably, sequence elements are considered substantially complementary if at least 85% of the nucleotide sequence in the two elements are complementary. Most preferably, sequence elements are considered substantially complementary if at least 95% of the nucleotide sequence in the two elements are complementary.

A label, according to the present invention, is a chemical moiety that is used to uniquely identify a nucleic acid molecule. In preferred embodiments, a label is a chemical moiety added onto a nucleotide or nucleoside. A label can be a nucleotide or nucleoside, a nucleic acid fragment, such as a DNA or RNA. In certain preferred embodiments, it may be preferable for the label be distinguishable from all other labels used in a particular application. The discrimination from other chemical moieties can be based on the chromatographic behavior of the label, its spectroscopic or potentiometric properties, or some combination thereof.

In certain preferred embodiments, the label may be cleavable by chemical means or by light. Chemically cleavable labels include those that are cleavable by an acid or by a nucleotide or nucleoside. Photo-cleavable labels include those that are cleavable by a wavelength of light. Other method of cleavage include oxidation, reduction, enzymatic, electrochemical, heat, and the like.

In other preferred embodiments, a label may be capable of terminating a primer extension reaction. In certain preferred embodiments, the terminating nature of the label may be due to the nature of the label itself, for example, the structure of the label, e.g., a label that is sufficiently bulky in its structure so that it prevents addition of any additional nucleotides or nucleosides to the extension product. Alternatively or additionally, the terminating nature of the label may be due to the placement of the label of the nucleotide or nucleoside. For example, the label may be attached to the nucleotide or nucleoside so that when the nucleotide or nucleoside is added to the growing 3'end by additional nucleotides or nucleosides cannot be added once a labeled nucleotide or nucleoside has been added.

The characteristics of a variety of well known labels that are amenable to attachment to the nucleotides or nucleosides and nucleic acid molecules of the invention are described in U.S. Patent No. 6,027,890, incorporated by reference. Such labels are detectable, once cleaved from the extended nucleotide or nucleoside, by fluorometry, mass spectrometry, infrared spectrometry, ultraviolet spectrometry, or potentiostatic amperometry.

"Complementary nucleotide triphosphates with a reduced ability to form base pairs" as used herein means that at least one of the nucleotides in a base pair is a derivative of a naturally occurring nucleotide that reduces the number of hydrogen bonds formed in the base pair. A "reduced ability to form a base pair" includes in its meaning "no ability to form a base pair."

Genomic DNA is used herein to mean the DNA which is found in an organism's genome and passed on to offspring as information necessary for survival.

Hybridization, as used herein, means the process in which single-stranded nucleic acid molecules are allowed to interact with each other to form double-stranded complexes wherein the two strands of a double-stranded duplex have substantially complementary sequences. For UNAs of the present invention, a reduced ability to hybridize means that two nucleic acid molecules having substantially complementary sequences do not maintain a double-stranded complex in a solution defined by a desired level of stringency. Hybridization may also occur between complementary sequence elements of the same nucleic acid molecule.

Preferably, an ability of a single-stranded nucleic acid to hybridize to another single-stranded nucleic acid molecule having substantially complementary sequence to form a duplex means that the duplex is maintained under low-stringency conditions. More preferably, the duplex is maintained under moderate (or medium) stringency conditions. Most preferably, the duplex is maintained under high-stringency conditions. Therefore, a "reduced ability to hybridize" means that two complementary sequences that were capable of hybridization to form a duplex under a desired level of stringency, are therefore not capable of duplex formation at the same level (or lower level) of stringency after introduction of modified nucleotides or nucleosides according to the teachings of the present invention. In addition, regions of UNAs having a reduced level of hybridization may be capable of hybridization under lower levels of stringency. Regions of UNAs having a reduced level of hybridization may also be unable to form duplexes under any level of stringency.

In performing hybridization assays discussed in the present teachings, suitable hybridization conditions are well known to those of ordinary skill in the art. In addition, those skilled in the art are capable of adjusting hybridization conditions to achieve a desired level of hybridization between nucleic acid molecules. More experimental details are available by reviewing Ausubel *et al. Current Protocols in Molecular Biology.* John Wiley & Sons. New York. 1999; Sambrook *et al., Molecular Cloning: A Laboratory Manual,* Cold Spring Harbor Laboratory Press, Cold Spring Harbor, NY, 1989.

As non-limiting examples of solution conditions that define stringency, the concentrations of SSC (sodium chloride, sodium citrate) and SDS (sodium dodecyl sulfate) can be adjusted and generally range from 6x SSC to 0.1x SSC (from low to high stringency) and 2% SDS to 0.1% SDS (also from low to high stringency). Also increasing the temperature of hybridization will increase the stringency of the hybridization. Temperatures for hybridization generally range from 42 degrees C (low stringency) to 70 degrees C (high stringency).

Preferred conditions for hybridization at varying stringency are provided as examples and are not limited to these conditions.

Low-stringency (6X SSC, 0.1% SDS) preferably at 42 degrees Celsius.

Moderate-stringency (0.5X SSC, 0.1% SDS) preferably at 55 degrees Celsius.

High-stringency (0.1X SSC, 0.1% SDS) preferably at 65 degrees Celsius.

Messenger RNA (mRNA) is defined in the present application as single-stranded RNA molecules that specify the amino acid sequence of one or more polypeptide chains. Synthesis of mRNA is carried out by an RNA polymerase from DNA and can be in vivo or in vitro. The information encoded by mRNA is translated during protein synthesis when ribosomes bind to the mRNA

Nucleotide triphosphates, as used herein, means a nucleotide monomer that can be used to form a nucleic acid polymer. The process of forming a nucleic acid polymer can be chemical or enzymatic. Preferably the process is enzymatic. Preferred nucleotide triphosphates are nucleotide triphosphates that can be incorporated by a polymerase to form an oligonucleotide or polynucleotide molecule.

Some non-limiting examples of deoxynucleotides include 2'-deoxyadenosine-5'-triphosphate (dATP), 2'-deoxyguanosine-5'-triphosphate (dGTP), 2'-deoxycytidine-5'-triphosphate (dCTP), 2'-deoxythymidine-5'-triphosphate (thymidine-5'-triphosphate) (TTP), 2-amino-2'-deoxyadenosine-5'-triphosphate, 2-thio-2'deoxythymidine-5'-triphosphate (or 2-thiothymidine-5'-triphosphate), 2-thio-2'-deoxycytidine-5'-triphosphate, 2'-deoxyinosine-5'-triphosphate (dITP), 2'-deoxypyrrolopyrimidine-5'-triphosphate (dPTP).

Some non-limiting examples of ribonucleotides include adenosine-5'-triphosphate (ATP), guanosine-5'-triphosphate (GTP), cytidine-5'-triphosphate (CTP), uridine-5'-triphosphate (UTP), 2-amino-adenosine-5'-triphosphate, 2-thiouridine-5'-triphosphate, 2-thiocytidine-5'-triphosphate, inosine-5'-triphosphate (ITP), pyrrolopyrimidine-5'-triphosphate (PTP).

Plasmid DNA as used herein means an independently replicating piece of DNA that can be transferred from one organism to another. Plasmid DNA can be linear or circular DNA molecules found in both prokaryotic cells and eukaryotic cells capable of autonomous replication. Plasmid DNA includes artificially constructed DNA.

A template, as used herein, means a nucleic acid molecule that can be used by a nucleic acid polymerase to direct the synthesis of a nucleic acid molecule that is complementary to the template according to the rules of Watson-Crick base pairing. For example, DNA polymerases utilized DNA to synthesize another DNA molecule having a sequence complementary to a strand of the template DNA. RNA polymerases utilize DNA as a template to direct the synthesis of RNA having a sequence complementary to a strand of the DNA template. DNA reverse transcriptases utilize RNA to direct the synthesis of DNA having a sequence complementary to a strand of the RNA template.

Preferred embodiments of the present invention are described below, by way of example only, with reference to the accompanying drawings:

Figure 1. Base pairing schemes for natural and modified nucleotide pairs. The bold X indicates the disruption or destabilization of the natural hydrogen-bonding interaction.

Figure 2. A) DNA primer and template sequence used for the polymerase extension reaction. B) Phosphorimage of the 10% denaturing PAGE analysis of the polymerase extension reactions. The dNTP composition are indicated for each reaction and abbreviated as follows: A; 2'-deoxyadenosine-5'-triphosphate, D; 2-amino-2'-deoxyadenosine-5'-triphosphate, G; 2'-deoxyguanosine-5'-triphosphate, C; 2'-deoxycytidine-5'-triphosphate, T; thymidine-5'-triphosphate, S; 2-thiothymidine-5'-triphosphate. The polymerase present in each reaction is indicated. The positions of the $^{32}$P-labeled primer (SEQ ID NO: 1) and 30-mer products (SEQ ID NO: 2) are indicated by arrows.

Figure 3. A) The 6-mer DNA primer and template (SEQ ID NO: 3) sequence used to test the incorporation of the ; 2-amino-2'-deoxyadenosine-5'-triphosphate in a polymerase extension reaction. B) Phosphorimage of the 20% denaturing PAGE analysis of the polymerase extension reactions. The dATP and 2-amino-2'-deoxyadenosine-5'-triphosphate (illustrated as dDTP) concentrations present in each reaction are indicated. The positions of the $^{32}$P-labeled DNA 6-mer and 7-mer products are indicated by arrows. C) Graphic representation of the percentage of 6-mer DNA primer converted to 7-mer DNA product as a function of dNTP concentration.

Figure 4. A) The 6-mer DNA primer and template sequence (SEQ ID NO: 3) used to test the incorporation of the 2-thiothymidine triphosphate in a polymerase extension reaction. B) Phosphorimage of the 20% PAGE analysis of the polymerase extension reactions. The dTTP and 2-thiothymidine-5'-triphosphate (illustrated as 2-thioTTP) concentrations present in each reaction are indicated. The positions of the $^{32}$P-labeled DNA 6-mer and 7-mer products are indicated by arrows. C) Graphic representation of the percentage of 6-mer DNA primer converted to 7-mer product as a function of dNTP concentration.

Figure 5. A) The 6-mer DNA primer and template sequences (SEQ ID NO: 3) used to test the incorporation of the 2-amino-2'-deoxyadenosine and 2-thiothymidine triphosphate in the polymerase extension reaction. B) MALDI mass spectra of the polymerase extension reactions containing the indicated dNTP. Then m/z values for the 6-mer and 7-mer extension products are indicated. C) Table summarizing the predicted and measured m/z values for the 6-mer and 7-mer extension products.

Figure 6. The scheme for generating single-stranded polynucleotides using a primer (SEQ ID NO: 4)/template (SEQ ID NO: 5)- dependent polymerase extension reaction (producing SEQ ID NO: 6) followed by digestion of the template DNA with λ exonuclease. Bold letters designate the TTT and AAA repeated sequence in each sequence.

Figure 7. Analysis by 10% denaturing PAGE of the single-stranded polynucleotides containing the indicated nucleotides generated according to the scheme outlined in Figure 6. The positions of size marker dyes are indicated with arrows.

Figure 8. Predicted secondary structures for three related 56-polynucleotide sequences containing either the four natural (A, G, C, T) nucleotides (SEQ ID NO: 7, SEQ ID NO: 9, and SEQ ID NOS: 11) or the 2-amino-2'-deoxyadenosine and 2-thiothymidine (illustrated as S) nucleotide substitutions (SEQ ID NO: 8, SEQ ID NO: 10, and SEQ ID NOS: 12).

Figure 9. Ultra-violet absorption spectra of the six purified polynucleotides depicted in Figure 8.

Figure 10. Analysis by 10% denaturing PAGE of the six polynucleotides containing either the four natural (indicated as A T) nucleotides or the 2-amino-2'-deoxyadenosine and 2-thiothymidine (illustrated as S) nucleotide substitutions (indicated as D S). 0.1 and 0.2 micrograms of the templated DNA for the HP21 polymerase extension reactions were run as standards. The size marker dyes are indicated with arrows. The gel was stained with Stains-All®.

Figure 11. The DNA primer and template (SEQ ID NO: 13, SEQ ID NO: 14, SEQ ID NO: 6, and SEQ ID NO: 15) sequences used to test the effect of the polynucleotide secondary structure on the polymerase extension reaction. The arrows indicate the direction of the polymerase extension reaction. Sequences in bold in the first three templates are derived from the primer shown in Figure 6 for the polymerization reaction used to generate each single-stranded template.

Figure 12. Phosphorimages of DNA products resolved by 20% denaturing PAGE resuming from polymerase extension reactions depicted in Figure 11. The primers used, templates used, reaction products, and reaction times for each reaction are indicated.

Figure 13. A quantitative graphic representation of the polymerase extension reactions shown in Figure 12. The graphs indicate the percentage of primers converted to the reaction products.

Figure 14. A flow chart representation of a direct labeling synthesis method for generating a single-stranded labeled UNA comprising the 2-aminoadenosine and 2-thiothymidine UNA pair using mRNA as the template. A labeled UNA strand is synthesized using a polymerase extension reaction directly from the polyadenylated mRNA strand primed with an oligo-dT primer. After UNA synthesis is complete, the mRNA template is degraded with RNAse A. (2-amino-2'-deoxyadenosine-5'-triphosphate=dDTP and 2'-deoxyinosine-5'-triphosphate=ITP)

Figure 15. A flow chart representation of a linear amplification method for generating labeled UNA comprising the 2-aminoadenosine and 2-thiothymidine UNA pair using a mRNA as the template. A first strand of DNA comprising the natural nucleotides dAMP, dGMP, dCMP and TMP is synthesized using an oligo-dt primed polymerase extension reaction directly from the polyadenylated mRNA. The RNA strand of the resulting DNA/RNA duplex is then nicked with RNaseH to generate random RNA primers to initiate second strand DNA synthesis. After the second strand synthesis is complete, the dNTPs are inactivated with a heat labile phosphatase. The phosphatase is inactivated and an oligo-dT primer is annealed by heating and cooling the reaction mixture. A first strand of UNA comprising the 2-aminoadenosine and 2-thiothymidine UNA pair is synthesized by the addition of the appropriate dNTPs (one labeled) and a thermostable polymerase. Multiple copies of UNA can be generated by cycling the temperature of the reaction mixture to repeat the last two steps of the process.

Figure 16. A flow chart representation of a method for generating labeled UNA comprising the 2-aminoadenosine and 2-thiothymidine, and the guanosine and 2-thiocytidine UNA pairs using a mRNA as the template. A first strand of DNA comprising the natural nucleotides dAMP, dGMP, dCMP and TMP is synthesized using an oligo-dT primed polymerase extension reaction directly from the polyadenylated mRNA. The dNTPs are subsequently inactivated using phosphatase followed by inactivation of the phosphatase with heat. The RNA strand of the resulting DNA/RNA duplex is then nicked with RNaseH to generate random RNA primers to initiate second strand DNA synthesis. Second strand synthesis is then carried out in the presence of dATP, dITP, dCTP and TTP. After inactivation of the dNTPs with the phosphatase, the phosphatase is inactivated and an oligo-dT primer is annealed to the DNA/DNA duplex by heating and cooling the reaction mixture. The labeled UNA comprising the 2-aminoadenosine and 2-thiothymidine, and the guanosine and 2-thiocytidine UNA pairs is then synthesized by the addition of the appropriate dNTPs (one labeled) and a DNA polymerase.

Figure 17. A flow chart representation of linear amplification method for generating a labeled UNA comprising the 2-aminoadenosine and 2-thiothymidine, and the guanosine and 2-thiocytidine UNA pairs using a mRNA as the template. A first strand of DNA comprising the natural nucleotides dAMP, dGMP, dCMP and TMP is synthesized using an oligo-dT primed polymerase extension reaction directly from the polyadenylated mRNA. The dNTPs are subsequently inactivated using phosphatase followed by inactivation of the phosphatase with heat. The RNA strand of the resulting DNA/RNA duplex is then nicked with RNaseH to generate random RNA primers to initiate second strand DNA synthesis. Second strand synthesis is then carried out in the presence of dATP, dITP, dCTP and TTP. After inactivation of the dNTPs with a phosphatase, the phosphatase is inactivated and an oligo-dT primer is annealed to DNA/INA duplex by heating and cooling the reaction mixture. The labeled UNA comprising the 2-aminoadenosine and 2-thiothymidine, and the guanosine and 2-thiocytidine UNA pairs is then synthesized by the addition of the appropriate dNTPs (one labeled) and a thermostable DNA polymerase. Multiple copies of the UNA are generated by cycling the temperature of the reaction mixture to repeat the last two steps of the process.

Figure 18. A flow chart representation of a linear amplification method for generating labeled ribo-UNA comprising the 2-aminoadenosine and 2-thiouridine UNA pair. A first strand of DNA comprising the natural nucleotides dAMP, dGMP, dCMP and TMP is synthesized by a polymerase extension reaction using a polyadenylated mRNA template and a primer comprising a phage RNA polymerase promoter sequence directly 5' to a poly-dT sequence. The RNA strand of the resuming DNA/RNA duplex is then nicked with RNaseH to generate random RNA primers to initiate second strand DNA synthesis. After the second strand synthesis is complete, synthesis of the ribo-UNA is initiated by the addition of the appropriate NTPs and phage RNA polymerase (e.g. T7 RNA polymerase).

Figure 19. A flow chart representation of linear amplification method for generating labeled ribo-UNA comprising the 2-aminoadenosine and 2-thiouridine, and the guanosine and 2-thiocytidine UNA pair. A first strand of DNA comprising the natural nucleotides dAMP, dGMP, dCMP and TMP is synthesized by a polymerase extension reaction using a polyadenylated mRNA template and a primer comprising a hairpin loop structure corresponding to the duplex sequence of a phage RNA polymerase promoter followed by a 3' single-stranded poly-dT sequence. The dNTPs are inactivated using phosphatase followed by inactivation of the phosphatase with heat. The RNA strand of the resulting DNA/RNA duplex is then nicked with RNaseH to generate random RNA primers to initiate second strand DNA synthesis. Second strand synthesis is then carried out in the presence of dATP, dITP, dCTP and TTP. After the second strand synthesis is complete, the nick between 5'-terminal thiophosphate of the hairpin primer and the completed second strand of DNA may be closed using DNA ligase. Synthesis of the ribo-UNA is then initiated by the addition of the appropriate NTPs and phage RNA polymerase (e.g. T7 RNA polymerase).

Figure 20. A flow chart representation of a method for generating amplified labeled UNA comprising the 2-aminoadenosine and 2-thiothymidine pair from double-stranded DNA using PCR and a linear amplification reaction. The double-stranded DNA target is first amplified using standard two-primer PCR methods. The dNTPs are inactivated using phosphatase which in turn is inactivated by heating. Additional forward primer is added and annealed to the PRC generated DNA/DNA duplex. UNA is synthesis is initiated by the addition of the appropriate dNTPs (one labeled) and a thermostable DNA polymerase. Multiple copies of UNA are generated by cycling the temperature to repeat the last two steps of the process.

Figure 21. A flow chart representation of a method for generating amplified labeled single-stranded UNA comprising the 2-aminoadenosine and 2-thiothymidine UNA pair from a PCR amplified double-stranded DNA. The double-stranded DNA target is first amplified using standard two-primer PCR methods. In this case, the two PCR primers have a 5'-terminal phosphate to initiate digestion by the λ Exonuclease. The dNTPs are inactivated using phosphatase which in turn is inactivated by heating. A forward primer lacking a 5'-terminal phosphate is then annealed to the PRC generated DNA/DNA duplex. UNA is synthesis is initiated by the addition of the appropriate dNTPs (one labeled) and a DNA polymerase. The template DNA strand having the 5'-terminal phosphate is then digested using λ Exonuclease.

Figure 22. A flow chart representation of a method for generating amplified labeled single-stranded UNA comprising the 2-aminoadenosine and 2-thiothymidine, and the guanosine and 2-thiocytidine UNA pair from a PCR amplified double-stranded DNA. The double-stranded DNA target is first amplified using standard two-primer PCR methods. In this case, the two PCR primers have a 5'-terminal phosphate to initiate digestion by the λ Exonuclease. The dNTPs are inactivated using phosphatase which in turn is inactivated by heating. Additional reverse primer and a dNTP mixture where dGTP is replaced with dITP is then added to initiate the synthesis of second strands having IMP. The dNTPs are once again inactivated using phosphatase which in turn is inactivated by heating. A forward primer lacking a 5'-terminal phosphate is then added and annealed to the PRC generated DNA/INA duplex. UNA is synthesis is initiated by the addition of the appropriate dNTPs (one labeled) and a DNA polymerase. The template INA strand having the 5'-terminal phosphate is then digested using λ Exonuclease.

Figure 23. A flow chart representation of an isothermal linear amplification method for generating single-stranded UNA comprising the 2-aminoadenosine and 2-thiothymidine UNA pair from messenger RNA. A first strand of DNA comprising the natural nucleotides dAMP, dGMP, dCMP and TMP is synthesized by a primer dependent polymerase extension reaction using polyadenylated mRNA as the template. The primer comprises a 5'-terminal sequence corresponding to the recognition site for the nicking enzyme BstNB I followed by a 3'-terminal oligo-dT sequence. The RNA strand of the resulting DNA/RNA duplex is then nicked with RNaseH to generate random RNA primers to initiate second strand DNA synthesis. After the second strand synthesis is complete, the dNTPs are inactivated with a heat labile phosphatase. The phosphatase is inactivated and the BstNB I enzyme is added to generate a nick to initiate first strand synthesis. First strand UNA comprising the 2-aminoadenosine and 2-thiothymidine UNA pair is synthesized by the addition of the appropriate dNTPs (one labeled) and a DNA polymerase. Multiple copies of UNA are then generated by the regeneration of the primer site through the continued nicking of the UNA/DNA products by BstNB I.

Figure 24. A flow chart representation of a direct labeling method for generating a single-stranded labeled UNA comprising the 2-aminoadenosine and 2-thiothymidine UNA pair using messenger RNA as the template. The labeled UNA strand is synthesized by a polymerase extension reaction using randomly primed mRNA templates. The primer mixture may comprise a complete set ofNmer sequences or subset ofNmers ranging in length from 6 to 10 nucleotides in length. After UNA synthesis is complete, the mRNA template is degraded with RNAse A.

Figure 25. The problem of cross-hybridization between two target nucleic acid molecules is depicted. A nucleic acid molecule (long dashes) with sequence specificity to a probe hybridizes to another nucleic acid molecule (short dashes) without sequence specificity to the probe resulting a false signal provided by the indirect hybridization of the second target (short dashes) to the probe.

Figure 26. The nucleotide sequence of a DNA hairpin is depicted with natural nucleotides. The same DNA hairpin having modified nucleotides is also shown. The theoretical thermal melting temperature for the DNA hairpin with A, T, C, G is shown for 1 M NaCl. The modified nucleotides are D (2-aminoadenosine) and 2-thiothymidine (illustrated as S).

Figure 27. A) The thermal melting profile of DNA having A, T, C, G shown in Figure 26 is shown. The concentration of DNA is 3.3 micromolar in I x SSPE (sodium salt, phosphate buffer, EDTA). B) The thermal melting profile of the hairpin nucleic acid molecule shown in Figure 26 having modified nucleotides is shown. The concentration of the "unstructured nucleic acid" (UNA) is 2.6 micromolar in 1x SSPE.

Figure 28 A and B. The first derivatives of the thermal melting profile from Figures 16 and 17 are shown at two concentrations of nucleic acids (a, 3 micromolar; b, 0.45 micromolar).

Figure 29. A table summarizing the thermal melting profiles of Figures 15-18 are shown.

Figure 30. A) Depicts is the specificity of 2-thio-2'-deoxycytidine triphosphate (2-thio-dCTP) incorporation by Bst DNA polymerase when either a single G or I was presented in the DNA template directly downstream of the primer binding site. B) Depicts efficient conversion of the 12-mer primer to the 13-mer extension product obtained with template G in the presence of dCTP but not in the presence of dsCTP

Figure 31. A) Synthesis of a UNA having all natural base pairing interactions destabilized, using two primed DNA templates, one containing G at multiple positions and the other containing I at the analogous position. B) Full-length product is generated for template G2 in the presence of the four natural nucleotides (AGCT), the single I for G substitution (AICT) and the triple substitution containing 2-aminoadenosine (illustrated as D), I and 2-thiothymidine (illustrated as S) (DICS)

Figure 32. A) The genes for the penicillin binding protein from *E. coli* (pBp-1), the chlorophyll A/B binding protein from *Arabidopsis thaliana* (Cab), and nucleotides 5-335 of the Hepatitis C virus (HCV) genome, which correspond to the three T7-RNA transcripts. B) Depiction of the expected lengths of the cUNAs generated from the three polyadenylated RNAs using an oligo-dT primer is 1194, 729 and 360 nucleotides respectively.

Figure 33. This figure shows that full-length products are generated for all three template RNAs in the presence of the four natural dNTPs (A,G,C,T); for the three template RNAs when the UNA nucleotide triphosphate analogues 2-amino-2'-deoxyadenosine (illustrated as D) and 2-thiothymidine (illustrated as S) are substituted for dATP and TTP respectively; and for the reaction mixtures that contain the additional substitution of ITP (I) for GTP.

Figure 34. Full-length fluorescently labeled product is generated in one hour in the presence of either the four natural dNPTs (A,G,C,T), when the UNA nucleotide triphosphate analogues 2-amino-2'-deoxyadenosine (D) and 2-thiothymidine (S) are substituted for dATP and TTP respectively, and also for the reaction mixtures that contain the additional substitution of ITP (I) for GTP.

Figure 35. A) Depiction of the T7 promoter linked to the double-stranded 138 base pair segment of the human retinoblastoma gene. B) The full-length product generated in the presence of either the four natural nucleotide triphophates or when the 2-aminoadenosine-5'-triphosphate and 2-thiouridine-5'-triphosphate are substituted for ATP and UTP respectively.

The present invention provides a system for generating nucleic acid molecules having reduced levels of intermolecular and/or intramolecular hybridization between substantially complementary regions, or sequence elements, compared to nucleic acid molecules of the same nucleotide sequence containing only naturally-occurring nucleotides. Such molecules are referred to herein as "unstructured nucleic acids" (UNAs). UNAs of the present invention include nucleic acid molecules that have reduced levels of secondary structure. UNAs of the present invention also include nucleic acid molecules that have a reduced ability to hybridize to another UNA having a region of sequence complementarity. UNAs maintain an ability to hybridize to a nucleic acid molecule of choice. A nucleic acid molecule of choice can be a naturally-occurring nucleic acid, a nucleic acid molecule with modified nucleotides or nucleosides, sugars and/or phosphate backbone, or a UNA molecule having a sequence designed specifically to hybridize to another UNA.

In certain preferred embodiments, the UNAs have reduced levels of secondary structure because of their reduced ability to form intramolecular hydrogen bond base pairs between regions of substantially complementary sequence. Such UNAs of the present invention are further characterized by the ability to hybridize to other nucleic acid molecules of interest. In other preferred embodiments, UNAs have reduced levels of *cross-hybridization* because of their reduced ability for form intermolecular hydrogen bonds between regions of substantially complementary sequence.

UNAs of the present invention contain regions of complementarity (sequence elements) such that the sequence elements of substantial complementarity have a reduced ability or inability to hybridize and form intramolecular or intermolecular hydrogen bond base pairs. Thus, the sequence elements may be within the same or between different nucleic acid molecules. For example, in non-UNA molecules, a sequence element X1 is capable of hybridizing to a substantially complementary sequence element Y1. In a UNA, one or both sequence elements contain modified nucleotides such that the ability of X1 to hybridize to Y1 is reduced or abolished. Preferably, sequence elements X1 and Y1 are characterized by the ability to hybridize to other sequence elements of substantial complementarity comprising naturally occurring or modified nucleotides.

In a modified sequence element, one or both of the nucleotides that together form a complementary base pair is substituted with a nucleotide containing a nucleotide analog so that the base pair is no longer formed, or is only formed at a reduced level. Preferably, the reduced level of base pairing is no more than one hydrogen bond interaction. Preferably, the analog(s) is selected so that the sequence element retains the ability to hybridize with a third sequence element in a nucleic acid molecule of complementary or substantially complementary sequence.

The base-pairing concepts of the present invention are schematically depicted by the following formulas where A' ≠ T' and G' ≠ C' represent disallowed base-pairing schemes, with the symbol ≠ representing the inability to form a base pair. A second group of nucleotides represented by [A*, T*, G*, and C*] are capable of forming base pairs with A', T', G' and C' according to the general Watson-Crick base pair scheme of A=T and G=C, where = represents the ability to form a base pair. The same base-pairing rules apply for RNA where U replaces T. (The horizontal base-pairing symbols are not meant to represent the number of hydrogen bonds present in the base pair, but are meant only to indicate a stable base pair or lack of a stable base pair.)

$$(A' \neq T'; G' \neq C') \qquad (1)$$

$$(A'=T^*; T'=A^*; G'=C^*; C'=G^*) \qquad (2)$$

Formula 1 indicates that base pair analogs A'/T' and G'/C' are unable to form a stable base pair. However, as indicated in Formula 2, the nucleotides A' T' G' and C' are capable of forming stable base pairs with a second group of nucleotides (A* T* G* C*).

UNAs of the present invention may contain a mixture of nucleotide analogs and naturally-occurring nucleotides. UNAs of the present invention may also contain only nucleotide analogs. More specifically, in accordance with the base-pairing formulas outlined in Formula 1 and 2, nucleotides of the first group (A', T', G', C') and nucleotides of the second group (A*, T*, G*, and C*) may include combinations of natural nucleotides and modified nucleotides or include all modified nucleotides. For example, A' and T', which do not form a stable base pair, may be comprised of one nucleotide analog (A') and one natural nucleotide (T'). Alternatively, A' and T' may be comprised of two nucleotide analogs. Nucleotide pairs from the second group (e.g. A* and T*) may or may not form stable base pairs (A*=T* or A*≠T*).

To provide non-limiting schematic examples of UNAs that contain mixtures of modified and natural nucleotides, UNAs of the present invention may contain both A' and T' nucleotide analogs that do not form stable base pairs and also contain G and C nucleotides that do form stable base pairs and also contain A and T nucleotides that do form stable base pairs. Alternatively, UNAs may contain G' and C' nucleotide analogs that do not form stable base pairs and also contain A and T nucleotides that do form stable base pairs. UNAs of the present invention may also contain both sets of analogs that do not form stable base pairs (A'≠T' and G'≠C'). For the present invention, nucleotides from the first and second class (e.g. A' and A*) may be mixed in the same molecule. However, it is preferred that a single UNA molecule possess no more than one of each type of nucleotide (e.g. only A' T' G and C) which results in only one type of base-pairing scheme for each potential base pair.

In preferred embodiments, the present invention provides unstructured nucleic acids or mixtures of unstructures nucleic acids including the nucleotides A, A', G, G', T, T', C and C', wherein A' does not form a stable base pair with T', and G' does not form a stable base pair with C' wherein the nucleic acid can form stable base pairs with another nucleic acid comprising the nucleotides A, A*, G, G*, T, T*, C, and C* wherein A* can form a stable base pair with T' and G* can form a stable base pair with C' and T* can form a stable base pair with A' and C* can form a stable base pair with G'.

In other preferred embodiments, the present invention provides unstructured nucleic acids or mixtures of unstructured nucleic acids including the nucleotides A', G', T', and C', wherein A' does not form a stable base pair with T', and G' does not form a stable base pair with C' wherein the nucleic acid can form stable base pairs with another nucleic acid comprising the nucleotides A*, G*, T*, and C* wherein A* can form a stable base pair with T' and G* can form a stable base pair with C' and T* can form a stable base pair with A' and C* can form a stable base pair with G'.

In yet other preferred embodiments, the present invention provides unstructured nucleic acids or mixtures of unstructured nucleic acids including the nucleotides A', G, T', C, wherein A' does not form a stable base pair with T', wherein the nucleic acid can form stable base pairs with another nucleic acid comprising the nucleotides A*, G, T*, C, wherein A* can form a stable base pair with T' and T* can form a stable base pair with A'.

Further preferred embodiments provide unstructured nucleic acids or mixtures of unstructured nucleic acids including the nucleotides A, G', T, C', wherein G' does not form a stable base pair with C', wherein the nucleic acid can form stable base pairs with another nucleic acid comprising the nucleotides A, G*, T, C*, wherein G* can form a stable base pair with C' and C* can form a stable base pair with G'.

*Selection of Nucleotides for UNAs*

In accordance with the present invention, UNAs are produced such that substantially complementary sequence elements between two UNAs or within a single UNA have a reduced ability to hybridize with each other. Nucleotides for producing such sequence elements having a reduced ability to hybridize are selected such that a first nucleotide is not capable of forming a stable base pair with a nucleotide complement within the same, or in a different nucleic acid molecule. The two complementary nucleotides may have one naturally-occurring nucleotide and one nucleotide analog or may have two nucleotide analogs. The complementary nucleotides that are unable to form a stable base pair are used to produce UNAs with reduce the levels of intramolecular or intermolecular base pairing by reducing hybridization between sequence elements within the UNA or between the UNAs that are substantially complementary.

In addition, where the sequence elements are within a single nucleotide, it is preferable that the complementary nucleotides in a UNA that are unable to form stable base pairs are capable of forming stable base pairs with at least one nucleotide complement present in a second polynucleotide molecule. Preferably, the second polynucleotide molecule contains sequences elements substantially complementary to sequence elements in the UNA to allow hybridization of part or all of the second polynucleotide to the UNA. Complementary sequence elements of the second polynucleotide may contain naturally-occurring nucleotides or nucleotide analogs.

Alternatively, where the reduced secondary structure is desired between sequence elements in two different nucleic acid molecules, it is preferable that a UNA maintains the ability to hybridize to a desired nucleic acid molecule (e.g., "probe" in a DNA expression array system: DNA chips or DNA in solution). The region of substantial complementarity between a UNA and a probe may overlap with the region of the UNA with a reduced ability to cross hybridize to another UNA. The region of substantial complementarity between a UNA and a probe may also be in a different part of the UNA molecule than the region with a reduce ability to cross hybridize to another UNA.

*2-Aminoadenosine, 2-Thiothymidine, Inosine (I) and Pyrrolo-pyrimidine (P)*

In certain preferred embodiments of the present invention, the nucleotide analogs 2-aminoadenosine, 2-thiothymidine, inosine (I), and pyrrolo-pyrimidine (P) are used to generate nucleic acid molecules that are unable to form stable intra- or intermolecular base pairs, yet retain their ability to form Watson-Crick base pairs with oligonucleotides composed of the four natural nucleotides. In certain preferred embodiments the nucleotide analogs are used to generate single UNAs having substantially complementary sequence elements having a reduced ability to hybridize. In other preferred embodiments, the nucleotide analogs are used to generate a first nucleic acid molecule (UNA) that has a reduced ability to cross hybridize with a second nucleic acid molecule having sequence elements substantially complementary to sequence elements in the first nucleic acid molecule. Such UNAs, while being unable to cross hybridize with each other, maintain the ability to hybridize with nucleic acid molecules of choice ("probes''). The structures of the 2-amino adenosine/2-thiothymidine, I/P and the four natural base pairs along with various combinations of the natural and nucleotide analogs are shown in Figure 1.

Naturally occurring Watson-Crick base pairing is defined by specific hydrogen bonding interactions between the nucleotides of adenine and thymine (or uracil) and between guanine and cytosine (or uracil). Positioning of hydrogen-bond donors (e.g. amino groups) and hydrogen-bond acceptors (e.g. carbonyl groups) on purine and pyrimidine nucleotides place structural constraints on the ability of two nucleosides to form stable hydrogen bonds. Figure 1 shows the structures of the nucleotides and the relative orientations of the nucleotides to each other in a Watson-Crick base pair. In addition, an inosine:cytosine base pair is shown. The inosine-cytosine base pair is identical to a G-C base pair except that the I-C base pair lacks the hydrogen bond donor of the 2-amino group of guanine, which is missing in inosine.

Therefore, a first sequence element containing 2-aminoadenosine is unable to cross hybridize to a second sequence element containing 2-thiiothymidine. However, 2-aminoadenosine of the first sequence element is still capable of hybridizing to a nucleic acid molecule containing natural thymidine nucleotides. In addition, a first sequence element containing inosine is unable to cross hybridize to a second sequence element containing pyrroloyrimidine. However, inosine of the first sequence element is still capable of hybridizing to a sequence element containing natural guanosine nucleotides. These first and second sequence elements can form regions of the same or different UNA molecules creating the ability or inability to form stable intramolecular or intermolecular base pairs, respectively.

*2-Aminoadenosine, 2-Thiothymidine*

Without being limited by theory, a 2-aminoadenosine/2-thiothymidine base pair analog is prevented from forming a stable base pair presumably due to a steric clash between the 2-thio group of 2-thiothymidine and the exocyclic amino group of 2-aminoadenosine as a result of the larger atomic radius of the sulfur atom. This tilts the nucleotides relative to one another such that only one hydrogen bond is able to form. It is also known that thionyl sulfur atoms are poorer hydrogen-bonding acceptors than carbonyl oxygen atoms which could also contribute to the weakening of the 2-aminoadenosine/2-thiothymidine base pair.

However, the 2-aminoadenosine is capable of forming a stable base pair with thymidine (T) through three hydrogen bonds in which a third hydrogen bonding interaction is formed between the 2-amino group and the C2 carbonyl group of thymine. As a result, the 2-aminoadenosine/T base pair is more stable thermodynamically than an A/T base pair. In addition, 2-thiothymidine is capable of forming a stable hydrogen bonded base pair with adenosine (A) which lacks an exocyclic C2 group to clash with the 2-thio group.

Therefore, polynucleotide molecules with 2-aminoadenosine and 2-thiothymidine replacing A and T respectively are unable to form intramolecular and intermolecular 2-aminoadenosine/2-thiothymidine base pairs but are still capable of hybridizing to polynucleotides of substantially complementary sequence comprising A and T and lacking 2-amino adenosine and 2-thiothymidine. Without being limited by theory, the aforementioned proposed mechanisms regarding the factors responsible for stabilizing and disrupting the A/T and G/C analogue pairs are not meant in any way to limit the scope of the present invention and are valid irrespective of the nature of the specific mechanisms.

Gamper and coworkers (Kutyavin *et al. Biochemistry,* 35; 11170 (1996)) determined experimentally that short oligonucleotide duplexes containing D/T base pairs that replace A/T base pairs have melting temperatures (Tm) as much as 10°C higher than duplexes of identical sequence composed of the four natural nucleotides. This is due mainly to the extra hydrogen bond provide by the 2-amino group. However, the duplexes designed to form opposing 2-aminoadenosine/2-thiothymidine base pairs exhibited Tms as much as 25°C lower than the duplex of identical sequence composed of standard A/T base pairs. The authors speculate that this is mainly due to the steric clash between the 2-thio group and the 2-amino group which destabilizes the duplex. Deoxyribonucleotides in this study were synthesized using chemical methods.

Although the base-pairing selectivity for these analog pairs has been experimentally tested for only DNA duplexes, it is likely that these same rules will hold for RNA duplexes and DNA/RNA heteroduplexes as well. This would allow for RNA versions of UNAs to be generated by transcription of PCR or cDNA products using the ribonucleotide triphosphate forms of the UNA analog pairs and RNA polymerases.

*Inosine (I) and Pyrrolo-pyrimidine (P)*

The inosine (I) and pyrrolo-pyrimidine (P) I/P base pair analog is also depicted in Figure 1. Inosine, which lacks the exocyclic 2-amino group of guanine, forms a stable base pair with cytosine through two hydrogen bonds (vs. three for G/C). The other member of the I/P analog is pyrrolo-pyrimidine (P) which is capable of forming a stable base pair with guanine despite the loss of the 4-amino hydrogen bond donor of cytosine. Figure 1 shows that a P/G base pair is also formed through two hydrogen bonds. The N7 group of P is spatially confined by the pyrrole ring and is unable to form a hydrogen bond with the C6 carbonyl O of guanine. However, this does not prevent the formation of the other two hydrogen bonds between P/G. The I/P base pair is only capable of forming one hydrogen bond (as depicted in Figure 1) and is therefore not a stable base pair. As a result, polynucleotide molecules with I and P replacing G and C respectively are unable to form intramolecular and intermolecular I/P base pairs but are still capable of hybridizing to polynucleotides of substantially complementary sequence comprising G and C and lacking I and P.

Woo and co-workers (Woo *et al., Nucleic Acids Research,* 24; 2470 (1996)) showed that introducing either P or I into 28-mer duplexes to form P/G and I/C base pairs decreased the Tm of the duplex by -0.5 and -1.9°C respectively per modified base pair. These values reflect the slight destabilization attributable to the G/P pair and a larger destabilization due to the I/C pair. However, introducing P and I into the duplexes such that opposing I/P base pairs are formed reduced the Tm by -3.3°C per modified base pair. Therefore the I/P base pairs are more destabilizing.

*UNAs comprising 2-aminoadenosine, 2-thiothymidine, I, and P*

In accordance with the present invention, UNAs with reduced ability to form intramolecular or intermolecular base pairs are generated by performing primer dependent, template directed polymerase reactions using the nucleotide 5'-triphosphate forms of the appropriate analog pairs. These include; 2-amino-2'-deoxyadenosine-5'-triphosphate, 2-thiothymidine-5'-triphosphate, 2'-deoxyinosine-5'-triphosphate (dITP) and 2'-deoxypyrrolo-pyrimidine-5'-triphosphate (dPTP). For example, a reaction containing 2-amino-2'-deoxyadenosine-5'-triphosphate, 2-thiothymidine-5'-triphosphate, dCTP and dGTP will generate UNAs which are unable to form intramolecular and intermolecular 2-aminoadenosine/2-thiothymidine base pairs. Likewise, a reaction containing dATP, dTTP, dPTP and dITP will generate UNAs which are unable to form intramolecular and intermolecular P/I (modification of G/C) base pairs. A polymerization reaction containing both analog pairs, 2-amino-2'-deoxyadenosine-5'-triphosphate, 2-thiothymidine-5'triphosphate; and dPTP, dITP will generate UNAs that have no predicted intramolecular or intermolecular base-pairing interactions. However, since 2-aminoadenosine, 2-thiothymidine, pyrrolopyrimindine, and inosine are still capable of forming stable base pairs with thymidine, adenosine, cytidine and guanosine respectively, all three types of UNAs should be able to specifically hybridize intermolecularly to oligonucleotides composed of the four natural bases.

In yet another preferred embodiment, it is recognized that UNAs of the present invention may contain various levels of intramolecular and intermolecular secondary structure. For example, UNAs may contain only G/C intramolecular base pairs and not A/T intramolecular base pairs. Alternatively, for UNAs having A, T, pyrrolo-pyrimidine, and inosine, these UNAs may contain only A/T intramolecular or intermolecular base pairs and not G/C intramolecular or intermolecular base pairs. As described in Examples 1 and 2 but without limitations to only those experimental conditions, UNAs potentially containing only G/C intramolecular or intermolecular base pairs are generated by enzymatically incorporating the triphosphate forms of 2-aminoadenosine, 2-thiothymidine, guanosine, and cytosine into a polynucleotide. The resulting UNA polynucleotide is not capable of forming intramolecular and intermolecular A/T base pairs, but is still capable of forming intramolecular and intermolecular G/C base pairs. The aforementioned mechanisms, which may account for the observed disruption of the A/T and G/C analogue pairs is not meant in anyway to limit the scope of the present invention and is valid irrespective of the nature of the specific mechanisms.

*UNAs comprising 2-aminoadenosine, 2-thiothymidine, 2-thiocytosine, and G*

In yet another preferred embodiment of the present invention, the nucleotide analogs 2-amioadenosine/2-thiothymidine and 2-thiocytosine/guanosine (2-thiocytosine/G) are used in primer dependent polymerase reactions to generate nucleic acid molecules that are unable to form stable intramolecular or intermolecular base pairs with each other yet retain their ability to form Watson-Crick base pairs with oligonucleotides composed of the four natural nucleotides and inosine or containing inosine as a substitute for G. 2-thiocytosine and G are unable to form a stable base pair. The presence of a 2-thiol exocyclic group in cytosine replacing the C2 carbonyl group effectively removes the hydrogen bond acceptor at that position and causes a steric clash due to the large ionic radius of sulfur as compared to oxygen. As a result, 2-thiocytosine/G is only capable of forming a single hydrogen bond and is thus not a stable base pair. However, 2-thiocytosine and I are capable of forming a stable base pair through two hydrogen bonds since the removal of the 2-amino exocyclic group of guanine that results in inosine effectively removes the steric clash between the C2 sulfur of 2-thiocytosine and the 2-amino group of guanine.

Therefore, UNA polynucleotide molecules with reduced levels of intramolecular or intermolecular hybridization are generated enzymatically using the 5'-triphosphate forms of the base pair analogs. These include; 2-amino-2'-deoxyadenosine-5'-triphosphate, 2-thiothymidine-5'-triphosphate, 2'-deoxyguanosine-5'-triphosphate (dGTP) and 2-thio-2'-deoxycytidine-5'-triphosphate. For example, a reaction with 2-thio-2'-deoxycytidine-5'-triphosphate, dGTP, dATP, dTTP will generate UNAs that can form only A/T base pairs. A polymerization reaction containing both analog pairs, 2-thio-2'-deoxycytidine-5'-triphosphate/dGTP, and 2-amino-2'-deoxyadenosine-5'-triphosphate/2-thiothymidine-5'-triphosphate will generate UNAs that have no predicted intramolecular or intermolecular base-pairing interactions. However, since 2-aminoadenosine, 2-thiothymidine, 2-thiocytidine and guanosine are still capable of forming stable base pairs with thymidine, adenosine, inosine and cytidine respectively, UNAs comprising (A, T, 2-thiocytosine, G) or (2-aminoadenosine, 2-thiothymidine, 2-thiocytosine, G) should be able to specifically hybridize to oligonucleotides (i.e., probes) composed of the appropriate nucleotides according to the base-pairing rules discussed.

The 2-thiocytosine/G base pair analog provides an example of a base pair analog comprising a natural nucleotide and an analog which can not form a stable base pair. As previously stated, polynucleotides containing 2-thiocytidine and guanosine cannot form intramolecular or intermolecular 2-thiocytosine/G base pairs. However, these polynucleotides can form base pairs with polynucleotides of substantially complementary sequences through 2-thiocytosine/I and C/G base pairs. Therefore, UNAs comprising 2-thiocytosine/G are capable of hybridizing to polynucleotide molecules also containing base analogs (inosine).

*Methods of Producing UNAs*

It is an aspect of the present invention that polynucleotides with reduced levels of intramolecular or intermolecular base pairing and preferably also with the ability to hybridize to other nucleotide molecules of substantially complementary sequence are produced by any method that incorporates nucleotides having naturally occurring and/or modified nucleotides. Preferably, UNAs according to the present invention are produced enzymatically. Alternatively, the UNAs of the invention are produced chemically.

Oligonucleotides and polynucleotides containing nucleotide or nucleoside, ribose and phosphate backbone modifications have been chemically synthesized using methods known in the art (Beaucage and Caruthers, *Tetrahedron Letters,* 22; 1859-1862 (1981)). Current limitations in the chemistry of the nucleic acid synthesis, such as yield and purity, restrict the size of oligonucleotides synthesized chemically to approximately 100 nucleotides in length. However, the present invention does not preclude the chemical synthesis of UNAs greater than 100 nucleotides in length.

In a preferred embodiment, UNAs of the present invention are produced enzymatically. Polymerization methodologies that utilize template-dependent DNA or RNA polymerases are preferred methods for copying genetic material of known or unknown sequence from biological sources for subsequent sequence and expression analyses. However, it is recognized that preferred UNAs of the present invention may be produced by any method and their production is not limited to enzymatic methods. Thus UNAs, which are produced preferably by enzymatic methods, are well suited for generating oligonucleotides and polynucleotides for subsequent genetic and expression analysis. Moreover, since preferred UNAs of the present invention are generated using DNA and RNA polymerases, the invention is able to generate oligonucleotides and polynucleotides anywhere from 8 to several thousand nucleotides in length. Preferably, UNAs of the present invention are at least 40 nucleotides in length. More preferably, UNAs of the present invention are at least 100 nucleotides in length. Most preferably, UNAs of the present invention are at least 500 nucleotides in length.

Any enzyme capable of incorporating naturally-occurring nucleotides, nucleotide analogs, or combinations thereof into a polynucleotide may be utilized in accordance with the present invention. A key to forming nucleic acids with reduced levels of intramolecular base pairing is the selection of nucleotides for the polymerase reaction, such that certain first and second complementary nucleotides in the growing nucleotide strands have a reduced ability to form an intramolecular base pair but can form an intermolecular base pair. Similarly, a key to forming nucleic acids with a reduced ability to form intermolecular base pairs with a second nucleic acid molecule, is the selection of nucleotides for the polymerase reaction such that certain first and second complementary nucleotides in the growing nucleotide strands have a reduced ability to form an intermolecular base pair with certain other modified nucleic acids (e.g., other UNAs) but can form an intermolecular base pair with a natural nucleic acid molecule.

According to certain preferred embodiments wherein intramolecular base pairing between two sequence elements is reduced, one method of synthesizing an unstructured nucleic acid includes the steps of first providing a nucleic acid template strand including a first template sequence element and a second template sequence element that is substantially complementary to the first template sequence element. Such a template nucleic acid strand is likely to form hairpin loop structures by forming intramolecular base pairs between the substantially complementary first and second elements. A collection of nucleotide precursors (e.g., nucleotide triphosphates) is provided that is sufficient to synthesize a nucleic acid strand complementary to at least a portion of the template nucleic acid strand, which portion includes the first and second template sequence elements. The collection includes first and second complementary nucleotides, wherein the first and second complementary nucleotides have a reduced ability to form an intramolecular base pair, but can form an intermolecular base pair. According to the present invention, the collection of nucleotide triphosphates includes naturally occurring nucleotides and or modified nucleotides. In certain preferred embodiments, the collection of nucleotide triphosphates includes labeled nucleotides that are naturally occurring or modified nucleotides.

Synthesis of the complementary strand occurs by contacting the template and nucleotide triphosphates with a nucleic acid polymerase enzyme capable of polymerizing the nucleotides under conditions and for a time sufficient for incorporation of the nucleotides to synthesize the UNA so that the first complementary sequence element and the second complementary sequence element of the UNA do not interact with one another. That is, the incorporated nucleotides prevent formation of intramolecular base pairs between the first and second sequence elements, which are substantially complementary. This further prevents the formation of hairpin loop structures in single-stranded nucleic acid molecules.

As mentioned above, the nucleic acid polymerase enzyme selected can be any nucleic acid polymerase that will incorporate naturally occurring and modified nucleotides into a growing nucleic acid template. In general, these include any nucleic acid polymerases that are capable of incorporating natural nucleotides. Without limitation, the enzyme can be a primer/DNA template-dependent DNA polymerase, a primer/RNA template-dependent reverse transcriptase, a single-stranded DNA dependent terminal transferase or self replicating RNA molecule, or a promoter-dependent RNA polymerase. Non-limiting examples of DNA polymerases include *E. coli* DNA polymerase I, *E. coli* DNA polymerase I Large Fragment (Klenow fragment), phage T7 DNA polymerase, and Phi-29 DNA polymerase. The polymerase can be a thermophilic polymerase such as *Thermus aquaticus* (Taq) DNA polymerase, *Thermus flavus* (Tfl) DNA polymerase, *Thermus Thermophilus* (Tth) DNA polymerase, *Thermococcus litoralis* (Tli) DNA polymerase, *Pyrococcus furiosus* (Pfu) DNA polymerase, Vent$^{TM}$ DNA polymerase, or *Bacillus stearothermophilus* (Bst) DNA polymerase. Non-limiting examples of reverse transcriptases include AMV Reverse Transcriptase, MMLV Reverse Transcriptase and HIV-1 reverse transcriptase. Non-limiting examples of RNA polymerases suitable for generating RNA version of UNAs include the bacteriophage RNA polymerases from SP6, T7 and T3. Furthermore, any molecule capable of using a DNA or an RNA molecule as a template to synthesize another DNA or RNA molecule can be used in accordance with the present invention (e.g. self replicating RNA).

Primer/DNA template-dependent DNA polymerases, primer/RNA template-dependent reverse transcriptases and promoter-dependent RNA polymerases incorporate nucleotide triphosphates into the growing polynucleotide chain according to the standard Watson and Crick base-pairing interactions (see for example; Johnson, Annual Review in *Biochemistry,* 62; 685-713 (1993), Goodman *et al.,* Critical Review in *Biochemistry and Molecular Biology,* 28; 83-126 (1993) and Chamberlin and Ryan, *The Enzymes, ed.* Boyer, Academic Press, New York, (1982) pp 87-108): Some primer/DNA template-dependent DNA polymerases and primer/RNA template-dependent reverse transcriptases are capable of incorporating non-naturally occurring nucleotide triphosphates into polynucleotide chains when the correct complementary nucleotide is present in the template sequence. For example, Klenow fragment and AMV reverse transcriptase are capable of incorporating the nucleotide analogue iso-guanosine opposite iso-cytidine residues in the template sequence (Switzer *et al., Biochemistry* 32; 10489-10496 (1993). (Nucleic acids containing the nucleotide analogs iso-guanosine or isocytidine are referred to herein as INAs.) Similarly, Klenow fragment and HIV-1 reverse transcriptase are capable of incorporating the nucleotide analogue 2,4-diaminopyrimidine opposite xanthosine in a template sequence (Lutz *et al., Nucleic Acids Research* 24; 1308-1313 (1996)).

UNAs may also be generated using one of a number of different methods known in the art. These include but are not limited to nick translation for generating labeled target molecules (Feinberg and Vogelstein, *Analytical Biochemistry,* 132; 6-13 (1983) and Feinberg and Vogelstein, *Analytical Biochemistry,* 137; 266-267 (1984)), asymmetric PCR methods (Gyllensten and Erlich, *Proc. Natl. Acad. Sci.* USA. 85; 7652-7656(1988)) that utilize a single primer or a primer having some chemical modification that results in the synthesis of strands of unequal lengths (Williams and Bartel, *Nucleic Acids Research,* 23; 4220-4221 (1995) and affinity purification methods that utilize either magnetic beads (Hultman *et al., Nucleic Acids Research,* 17; 4937-4946 (1989)) or streptavidin-induced electrophoretic mobility shifts (Nikos, *Nucleic Acids Research,* 24; 3645-3646 (1996)).

The asymmetric PCR method would be performed using a single target-specific primer and either a single-stranded or double-stranded DNA template in the presence of a thermophilic DNA polymerase or reverse transcriptase and the appropriate UNA nucleotide triphosphates. The reaction mixture would be subjected to temperature cycle a defined number of times depending upon the degree of amplification desired. The limitation of the amplification to this type of linear mode is inherent to the designed base-pairing properties of UNAs. Unlike nucleic acids generated from the four standard nucleotides, the UNA replication products are generated from non-complementary pairs of nucleotides and thus cannot serve as templates for subsequent replication events. However the invention does not preclude the use of PCR to amplify the target prior to generation of UNAs by the invention.

UNAs can also be generated using a polymerase extension reaction followed by a strand-selective exonuclease digestion (Little *et al., J Biol Chem.* 242, 672 (1967) and Higuchi and Ochamn, *Nucleic Acids Research,* 17; 5865-(1989)). For example, a target-specific primer is extended in an isothermal reaction using a DNA polymerase or reverse transcriptase in the presence of the appropriate UNA nucleotide triphosphates and a 5'-phosphorylated DNA template. The DNA template strand of the resulting duplex is then specifically degraded using the 5'-phosphoryl-specific lambda exonuclease. A kit for performing the latter step is the Strandase Kit™ currently marketed by Novagen (Madison, WI). Such methods are exemplified in Figures 21 and 22.

In other preferred embodiments, the UNAs generated by the present invention are labeled UNAs. Those skilled in the art will appreciate that many different labels are available for detecting nucleic acid molecules, e.g., radioactive labels, fluorescent labels, photocleavable or photoactivatable labels, dye labels, etc, (see Sambrook *et al., Molecular Cloning: A Laboratory Manual,* Cold Spring Harbor Press, N.Y.; Ausubel *et al., Current Protocols in Molecular Biology,* Green Publishing Associates, New York, V. 1&2 1996, each of which are incorporated herein by reference). Labeled nucleotides or nucleosides of the invention include labeled naturally occurring nucleotides or nucleosides, and labeled modified nucleotides or nucleosides.

For example, labeled single-stranded UNAs can be generated from mRNA templates as shown in Figure 14, which illustrates the generation of a single-stranded UNA using direct labeling synthesis. A primer extension reaction is carried out on a template mRNA, wherein the nucleotides incorporated into the growing nucleic acid strand include modified nucleotides, a naturally occurring nucleotide, and a labeled naturally occurring nucleotide. Once the primer extension reaction is complete, an RNAse degrades the template strand leaving the single-stranded UNA. Specifically, an extension reaction is carried out by annealing a primer (e.g., oligo dTTP) to the mRNA template and incubating the annealed primer/template with one or more DNA polymerase (or reverse transcriptase) and a combination of four nucleotides that include modified and natural nucleotides and labeled nucleotides (e.g., 2-amino-2'-deoxyadenosine-5'-triphosphate (illustrated as dDTP), dGTP (or ITP), dCTP•("•" = labeled nucleotide), dsTTP). Once the labeled UNA strand is synthesized, the RNA template strand is degraded with RNAse A.

Single-stranded mRNA can also be used as a template for linear amplification of UNAs. Figure 15 illustrates a linear amplification and labeling synthesis reaction where mRNA is incubated with a nucleic acid polymerase (e.g., DNA polymerase or reverse transcriptase) and four naturally occurring dNTPs (dATP, dGTP, dCTP, dTTP). Once strand extension is complete, resulting in the generation of a DNA/mRNA hybrid molecule, the RNA strand is nicked with RNAse H and second strand DNA synthesis is initiated using DNA polymerase (or reverse transcriptase) and four dNTPs. This step results in the generation of a DNA/DNA molecule. Those skilled in the art will appreciate that prior to proceeding to the synthesis of a labeled UNA any unincorporated dNTPs are preferably first inactivated with a phosphatase enzyme (e.g., shrimp alkaline phosphatase). Generation ofthe UNA molecule is initiated by first inactivating the phosphatase enzyme with increased temperature, then annealing a primer to the DNA/DNA template and incubating the primer/template with DNA polymerase or reverse transcriptase and a mixture of natural and modified nucleotides and labeled nucleotides. In Figure 15, the mixture of nucleotides includes 2-amino-2'-deoxyadenosine-5'-triphosphate (illustrated as dDTP), dGTP•, dCTP, and 2-thio-dTTP. However, those skilled in the art will appreciate that any mixture of nucleotides, selected from modified nucleotides, naturally occurring nucleotides, and labeled modified and naturally occurring nucleotides, may be used. Those skilled in the art will further appreciate that the labeling synthesis reaction can be repeated any number of times to generate many labeled UNA molecules. Furthermore, it will also be recognized that an exonuclease digestion can optionally be performed to degrade the template strand, to generate a single-stranded UNA.

UNA molecules having a 2-thio-cytidine nucleotide to reduce G-C base pair stability can be generated using an inosine-containing template (INA). Instead of generating a DNA duplex molecule from a mRNA template, as shown in Figure 15, a DNA heteroduplex molecule having a first strand containing 2'-deoxyinosine-5'-triphosphate (dITP) nucleotides (INA strand) and a second strand containing dNTP nucleotides, is generated. For example, as shown in Figure 16, the DNA/mRNA hybrid molecule is used as a template for a primer extension reaction in the presence of a nucleic acid polymerase and a mixture of nucleotides that includes a deoxyinosine nucleotide (e.g., dATP, dITP, dCTP, dTTP, as shown in Figure 16). The labeled UNA strand is then generated off the INA template strand, as described above, using a mixture of UNA and labeled nucleotides, e.g., 2-amino-2'-deoxyadenosine-5'-triphosphate (illustrated as dDTP), dGTP•, 2-thio-dCTP, and 2-thio-dTTP. The labeling synthesis reaction can be performed off the DNA template any number of times to generate many labeled UNA molecules (Figure 17).

Single-stranded ribonucleotide (RNA) versions of UNAs can be synthesized using *in vitro* transcription methods which utilize phage promoter-specific RNA polymerases such as SP6 RNA polymerase, T7 RNA polymerase and T3 RNA polymerase (see for example Chamberlin and Ryan, *The Enzymes, ed.* Boyer, Academic Press, New York, (1982) pp87-108, and Melton *et al., Nucleic Acids Research,* 12; 7035 (1984)). For these methods, a double-stranded DNA corresponding to the target sequence is generated using PCR methods known in the art in which a phage promoter sequence is incorporated upstream of the target sequence. This double-stranded DNA is then used as the template in an *in vitro* transcription reaction containing the appropriate phage polymerase and the ribonucleotide triphosphate UNA analogues. Alternatively, a single-stranded DNA template prepared according to the method of Milligan and Uhlenbeck, *(Methods in Enzymology,* 180A, 51-62 (1989)) can be used to generate RNA versions of UNAs having any sequence. A benefit of these types *of in vitro* transcription methods is that they can result in a 100 to 500 fold amplification of the template sequence.

Labeled polynucleotides containing ribonucleotides can be synthesized from mRNA templates using an RNA polymerase (e.g., T7 RNA polymerase, SP6 polymerase, or T3 polymerase). A DNA/DNA template may be synthesized that includes a promoter sequence for RNA polymerase. In certain preferred embodiments, this is accomplished by providing an mRNA template and subjecting the mRNA template to primer extension using a primer that includes an RNA polymerase promoter. For example, Figure 18 illustrates one strategy in which a primer containing a T7 promoter is used in a primer extension reaction on the mRNA template with a DNA polymerase (or reverse transcriptase) and four dNTPs to generate a DNA/mRNA hybrid molecule. After second DNA strand synthesis is complete, the resulting DNA/DNA molecule, now containing a T7 promoter, is incubated with T7 RNA polymerase and a mixture of rUNAs, labeled rNTP bases, and unlabeled rNTP bases (e.g., 2-aminoadenosine-5'-triphosphate (illustrated as rDTP), rGTP, rCTP•, 2-thio-rUTP). Other examples of ribonucleotides that might be used to synthesize a UNA include adenosine-monophosphate (AMP), guanosine-monophosphate (GMP), cytidine-monophosphate (CMP), uridine-monophosphate (UMP), 2-aminoadenosine-monophosphate, 2-thiouridine-monophosphate, 2-thiocytidine-monophosphate, inosine-monophosphate, pyrrolopyrimidine-monophosphate (PMP). The resulting molecule contains a first (newly synthesized) strand, which contains ribonucleotides and labeled rNTP nucleotides.

Those skilled in the art will appreciate that a primer containing an RNA polymerase promoter sequence can be any type of oligonucleotide primer that initiates polymerization. As shown in Figure 19, the primer can form a hairpin-loop structure. Those skilled in the art will also appreciate that any combination of nucleotides can be used in the polymerase reaction at any stage of the linear synthesis, or linear amplified labeling synthesis process. For example, as shown in Figure 19, a dITP can be used in the second round of primer extension to generate a DNA/INA heteroduplex, instead of a dNTP as shown in Figure 18 (also see Figure 15 and Figure 16). It will also be recognized that any combination of nucleotides can be used to generate labeled UNAs, whether they be dNTP or rNTP, labeled or unlabeled nucleotides. Any base pair combination can be selected, wherein certain nucleotides are modified nucleotides and certain nucleotides are naturally occurring nucleotides, as long as the desired UNA molecule having reduced secondary structure is generated. In certain embodiments, the UNA base may be labeled. For example, a combination of 2-aminoadenosine-5'-triphosphate, rGTP, rCTP, r2-thio-rUTP• (illustrated as rsUTP•) may be used to generate a UNA, wherein •represents the label, as shown in Figure 19 as a star. However, the combination of 2-aminoadenosine-5'-triphosphate, rGTP, rCTP•, and 2-thio-rUTP may also be used to generate the UNA as shown in Figure 19. However, the combination of 2-am ino-2'-deoxriboyadenos ine-5'-tri phosphate, rGTP, 2-thio-rCTP•, and 2-thio-rUTP may also be used to generate the UNA, as shown in Figure 18, using a labeled naturally occurring nucleotide. Other combinations would be obvious to one skilled in the art based on the teachings provided herein.

Other methods of generating labeled single-stranded and double-stranded UNA molecules include methods of coupling PCR and linear amplification labeling. One method of generating labeled double-stranded UNA-containing molecules is to perform a first round of amplification with natural dNTP nucleotides and performing a second round of amplification with a mixture of nucleotides that includes a mixture of natural dNTP, labeled dNTP and modified nucleotides. This results in labeled double-stranded UNA, as shown in Figure 20.

Yet other methods involve generating labeled single-stranded UNA molecules, as illustrated in Figures 21 and 22. In Figure 21, double-stranded DNA molecules are generated using a standard PCR reaction where both primers possesses a 5'-terminal phosphate moiety. The resulting product serves as template for a nucleotide polymerase (e.g., DNA polymerase or reverse transcriptase) which extends an unphosphorylated primer in the presence of UNA and labeled and unlabeled dNTPs. To isolate a labeled single-stranded UNA molecule, the DNA template having 5'-phosphorylated termini is digested with a DNA exonuclease (e.g., λ exonuclease).

Another method, illustrated in Figure 22, includes the step of generating a DNA/INA heteroduplex by carrying out two separate primer extension reactions after the amplification of the double-stranded DNA molecule is complete. The first primer extension reaction incorporates dITP into the growing strand, generating the DNA/INA heteroduplex. The DNA/INA heteroduplex is then used in a second primer extension reaction, wherein the INA serves as the template for the growing UNA strand. Exposure of the UNA/INA heteroduplex to an exonuclease yields a labeled single-stranded UNA.

Yet another method of generating UNAs is illustrated in Figure 23. This method uses an isothermal linear amplification procedure for generating single-stranded UNA comprising the 2-aminoadenosine and 2-thiothymidine UNA pair from messenger RNA. In this method, a first strand of DNA comprising the natural nucleotides dAMP, dGMP, dCMP and TMP is synthesized by a primer dependent polymerase extension reaction using polyadenylated mRNA as the template. In this method, the primer comprises a 5'-terminal sequence corresponding to the recognition site for a nicking enzyme, such as BstNB I, followed by a 3'-terminal oligo-dT sequence capable of hybridizing to the poly-A sequence of the messenger RNA. After first strand synthesis is complete, the RNA strand of the resulting DNA/RNA duplex is nicked with RNaseH to generate random RNA primers for second strand DNA synthesis. Those skilled in the art will appreciate that prior to proceeding with UNA synthesis, any unincorporated dNTPs must be inactivated with a phosphatase. The phosphatase is then inactivated and the BstNB I enzyme is added to generate a nick to initiate first strand synthesis. First strand UNA comprising the 2-aminoadenosine and 2-thiothymidine UNA pair is synthesized by the addition of the appropriate dNTPs (one labeled) and a DNA polymerase. Multiple copies of UNA are then generated by the regeneration of the primer site through the continued nicking of the UNA/DNA products by BstNB I. It should be appreciated by those skilled in the art that UNAs can also be generated using the appropriate dNTPs and the strand displacement method first proposed by Walker et al in 1992 (G. T. Walker, M. C. Little, J. G. Nadeau and D. D. Shank (1992) Proc. Natl. Acad. Sci 89, 392-396).

It should also be noted that the direct labeling method for generating a single-stranded labeled UNA can be carried out using a mixture of primers designed to hybridize at random positions along the target sequence. As illustrated in Figure 24, a UNA comprising the 2-aminoadenosine and 2-thiothymidine pair is generated using a polymerase extension reaction like that described in Figure 14 using messenger RNA as the template and a mixture ofNmers primers. The Nmer primer mixture may comprise a complete set of Nmer sequences or subset of the Nmers ranging in length from 6 to 10 nucleotides in length. For example, the Nmer mixture may be the complete set or subset of 4,096 6-mer sequences, a complete set or subset of 16,384 7-mer sequences or complete set or subset of 65,536 8-mer sequences. After UNA synthesis is complete, the mRNA template is degraded with RNAse A.

*Structural Modifications to Nucleotides*

According to the present invention, any form of a nucleotide analog that may be incorporated enzymatically or chemically into a polynucleotide may be used in accordance with the present invention. Preferably, but without limitation, the nucleotides and nucleotide analogues are in the 5'-triphosphate (NTP) form, including nucleoside 5'-triphosphates.

Nucleotide (or nucleoside) analogues having fewer structural changes can also be efficient substrates for DNA polymerase reactions. For example, a number of polymerases can specifically incorporate inosine across cytidine residues (Mizusawa *et al., Nucleic Acids Research,* 14; 1319 (1986). The analogue 2-aminoadenosine triphosphate can also be efficiently incorporated by a number of DNA polymerases and reverse transcriptases (Bailly and Waring, *Nucleic Acids Research,* 23; 885 (1996). In fact, 2-aminoadenosine is a natural substitute for adenosine in S-2L cyanophage genomic DNA. However, for the present invention 2-aminoadenosine is defined as a non-naturally occurring nucleotide. The 2-aminoadenosine ribonucleotide-5'-triphosphate is a good substrate for *E. coli* RNA polymerase (Rackwitz and Scheit, *Eur. J. Biochem.,* 72, 191 (1977)). The adenosine analogue 2-aminopurine can also be efficiently incorporated opposite T residues by *E. coli* DNA polymerase (Bloom *et al., Biochemistry* 32; 11247-11258 (1993) but can mispair with cytidine residues as well (Law *et al., Biochemistry* 35; 12329-12337 (1996)).

Any structural modifications to a nucleotide that do not inhibit the ability of an enzyme to incorporate the nucleotide analogue (or only slightly reduces the ability of an enzyme to incorporate the nucleotide analogue) is acceptable in accordance with the present invention if the modifications do not result in a violation of the nucleotide or nucleoside pairing rules set forth in the present invention. Modifications include but are not limited to structural changes to the nucleotide or nucleoside moiety (e.g. C5-bromouridine, C5-fluorouridine, C5-iodouridine, C5-propynyl-uridine, C5-propynyl-cytidine, C5-methylcytidine, 2-aminoadenosine, 7-deazaadenosine, 7-deazaguanosine, 8-oxoadenosine, 8-oxoguanosine), changes to the ribose ring (e.g. 2'-hydroxyl, 2'-fluro), and changes to the phosphodiester linkage (e.g. phosphorothioates and 5' -N-phosphoamidite linkages). It is recognized by those of ordinary skill in the art that the efficiency or rate of incorporation of modified nucleotide triphosphates by an enzyme may be affected by numerous factors included but not limited to reaction buffers containing varying concentrations of ethanol, DMSO, betaine, and divalent cations such as magnesium.

Watson-Crick base-pairing schemes can accommodate a number of modifications to the ribose ring, the phosphate backbone and the nucleotides or nucleosides (Saenger, Principles of Nucleic Acid Structure, Springer-Verlag, New York, NY. 1983). Certain modified nucleotides such as inosine, 7-deazaadenosine, 7-deazaguanosine and deoxyuridine decrease the stability of base-pairing interactions when incorporated into polynucleotides. The dNTP forms of these modified nucleotides are efficient substrates for DNA polymerases and have been used to reduce sequencing artifacts that result from target and extension product secondary structures (Mizusawa *et al., Nucleic Acids Research,* 14; 1319. 1986). Other modified nucleotides, such as 5-methylcytidine, C-5 propynyl-cytidine, C-5 propynyl-uridine and 2-aminoadenosine increase the stability of duplex when incorporated into polynucleotides (Wagner *et al., Science,* 260; 1510. 1993) and have been used to increase the hybridization efficiency between oligonucleotide probes and target sequences.

*Methods of Utilizing UNAs*

The ability to generate nucleic acids that retain their genetic information content, yet have a reduced ability to form intramolecular or intermolecular base pairs and hybridize to nucleic acid molecules of choice, has many advantages. Methods used in molecular biology and nucleic acids chemistry that rely on the hybridization of single-stranded nucleic acid probes to single-stranded nucleic acid targets of substantially complementary sequence can utilize UNAs in accordance with the present invention (for general protocols, see *Ausubel et al. Current Protocols in Molecular Biology.* John Wiley & Sons, Inc. 1998; Sambrook *et al., Molecular Cloning: A Laboratory Manual,* Cold Spring Harbor Laboratory Press, Cold Spring Harbor, NY, 1989; both of which are incorporated herein by reference). By way of examples and not limitations to the present invention, these methods include methods of *in situ* hybridization (*e.g.* mRNA fluorescence in situ hybridization; FISH), surface-bound oligonucleotide arrays for mutation detection (*e.g.* gene chip technology, Chee, M., *et al.,* (1996) *Science* 274, 610-614, Wang, *et. al., Science,* 280, 1077-1082), gene expression assays (Lockart, D., *et al.,* (1996) *Nat. Biotech.* 14, 1675-1680), the polymerase chain reaction (PCR), RNA (e.g. Northern blot) and DNA (e.g. Southern blot) blot hybridization, DNA sequencing including high-throughput (e.g., see Cheng, ''High speed DNA sequence analysis," *Prog. Biochem Biophys,* 22?223-227, June 1995; Ramsay, "DNA chips: State of the Art," *Nature Biotechnology,* 16:40-44, January 1998; Nanopore Sequencing; Deamer and Akeson, *Trends in Biotech,* 18:147-151, 2000), primer extension analysis, screening recombinant DNA libraries, polymerase extension assays, and X-mer ligase assays.

Methods used in molecular biology and cell biology that rely on the competitive hybridization of labeled genomic DNA to a fragment of immobilized DNA can also utilize UNAs as the labeled nucleic acid in accordance with the present invention. One such example includes comparative genomic hybridization (CGH), which allows rapid detection of DNA amplification anywhere in a genome and can additionally map the amplified region to a defined location on the chromosomes. CGH is based on competitive *in situ* hybridization of differentially labeled DNA samples to a normal chromosomal metaphase spread. Regions of amplified sequences are seen as an increased color ratio of tow fluorochromes used in the labeled DNAs (Kallioniemi *et al., Semin. Cancer Biol.* 4, 41-46 (1993) and Sudar *et al. Science,* 258(5083), 818-821 (1992) and U.S. Patent Nos. 5,665,549 and 6,159,685).

Techniques employing ligase assays and polymerase extension assays are useful for determining whether a mutation is present at a defined location in an otherwise known target nucleic acid sequence (see for example; Haff and Smirnov, *Genome Research,* 7; 378-388 (1997), Landegren *et al., Genome Research,* 8; 769-776 (1998), Shumaker *et al., Human Mutation,* 7; 346-354 (1996), Pastinen *et al., Genome Research,* 7: 606-614 (1997), and Nikiforov *et al., Nucleic Acids Research,* 22; 4167-4175 (1994)). U.S. Patent No. 4,988,617, for example, discloses a method for determining whether a mutation is present at a defined location in an otherwise known target nucleic acid sequence by assaying for the ligation of two natural oligonucleotides that are designed to hybridize adjacent to one another along the target sequence. U.S. Patent No. 5,494,810 discloses a method that utilizes a thermostable ligase and the ligase chain reaction (LCR) to detect specific nucleotide substitutions, deletions, insertions and translocations within an otherwise known target nucleic acid sequence using only natural nucleic acids. U.S. Patent No. 5,403,709 discloses a method for determining the nucleotide sequence by using another oligonucleotide as an extension and a third, bridging oligonucleotide to hold the first two together for ligation, and WO 97/35033 discloses methods for determining the identity of a nucleotide 3' to a defined primer using a polymerase extension assay.

U.S. Patent Nos. 5,521,065, 4,883,750 and 5,242,794 disclose methods of testing for the presence or absence of a target sequence in a mixture of single-stranded nucleic acid fragments. The method involves reacting a mixture of single-stranded nucleic acid fragments with a first probe that is complementary to a first region of the target sequence and with a second probe that is complementary to a second region of the target sequence. The first and second target regions are contiguous with one another. Hybridization conditions are used in which the two probes become stably hybridized to their associated target regions. Following hybridization, any of the first and second probes hybridized to contiguous first and second target regions are ligated, and the sample is subsequently tested for the presence of expected probe ligation product.

US patent application Serial Number 09/112437 discloses a generic methods and reagents for analyzing the nucleotide sequence of nucleic acids using high-throughput mass spectrometry (incorporated herein by reference in its entirety). The disclosed methods and reagents utilize complex mixtures of short (e.g. 6-mer and 7-mer) oligonucleotides (X-mers) and polymerase extension and ligation reactions to generate a complex mixture of oligonucleotide products that reflect the sample's DNA sequence. Because the methods rely on hybridization between the short X-mers and the target polynucleotide, UNAs would serve as superior targets for both the polymerase extension assay (PEA) and X-mer ligase assays (XLA). This is because the X-mers would not have to compete with intramolecular target sequences for their complementary binding site. Minimizing the effect of target structure makes the problem of equalizing the hybridization rates and subsequent polymerase extension reaction rates for each X-mer more straight-forward.

*Array Technology*

The present invention is particularly applicable to gene expression assays (e.g. DNA chip technology; Ramsay. "DNA chips:State of the Art." *Nature Biotechnology.* 16:40-44, Jan 1998; also see for example DNA chips made by Affymetrix, Santa Clara, CA). The current art requires considerable probe design and probe redundancy to ensure that the probes are targeted to regions of the mRNA species (or cDNA) which are not involved in the intramolecular structures and therefore capable of hybridizing with the probes. In addition, current technology produces potential *cross-hybridization* between targets resulting in false positives when attempting to detect desired probe nucleic acid sequences. Employing UNAs as the target would minimize if not eliminate the need for target design, would likely increase the overall sensitivity of the gene expression assays, and would reduce signals resulting from false positives due to cross-hybridization oftargets. Importantly, UNAs would greatly facilitate the use of short oligonucleotide arrays for *de novo* sequence, re-sequencing, mutation scanning and detection since these applications, by definition, require that all regions of the target sequence be accessible for hybridization by the interrogating probes.

The ability to generate nucleic acids that retain their information content yet possess little or no secondary or inter-target structure would improve DNA microarray expression profiling results in two ways. First, as previously noted, UNAs are incapable of indirect cross-hybridization. Therefore, differential expression ratios measured in microarray experiments would not be degraded by this mechanism. Second, the inability of UNAs to form intra-molecular secondary structure would produce the same benefits derived from target fragmentation (reduction of target secondary structure and indirect cross-hybridization potential) without the accompanying disadvantages (signal loss and irreproducibility of the degree of fragmentation).

Techniques employing hybridization to surface-bound DNA probe arrays are useful for analyzing the nucleotide sequence of target nucleic acids. These techniques rely upon the inherent ability of nucleic acids to form duplexes via hydrogen bonding according to Watson-Crick base-pairing rules. In theory, and to some extent in practice, hybridization to surface-bound DNA probe arrays can provide a relatively large amount of information in a single experiment. For example, array technology has identified single nucleotide polymorphisms within relatively long (1,000 residues) sequences (Kozal, M., *et al., Nature Med.* 7:753-759, July 1996). In addition, array technology is useful for some types of gene expression analysis, relying upon a comparative analysis of complex mixtures of mRNA target (Lockart, D., *et al., Nat. Biotech.* 14, 1675-1680. 1996). Although array technologies offer the advantages to being reasonably sensitive and accurate when developed for specific applications and for specific sets of target sequences, they lack a generic implementation that can simultaneously be applied to multiple and/or different applications and targets. This is in large part due to the need for relatively long probe/target duplexes. Moreover, this use of relatively long probes makes it difficult to interrogate single nucleotide differences due to the inherently small thermodynamic difference between the perfect complement and the single mismatch within the probe/target duplex. In addition, detection depends upon solution diffusion properties and hydrogen bonding between complementary target and probe sequences. Therefore, utilizing UNAs of the present invention as nucleic acid targets will enable the use short oligonucleotide probes for differentiating between nucleic acid molecules differing in sequence by a few or even a single nucleotide.

UNA targets could also be used in conjunction with arrays for performing genomic hybridization (CGH) (see Pinkel, D. et al., Nature Genetics, 20(2), 207-211 (1998) and U.S. Patent No. 5,830,645). In this method, amplified regions of DNA within a genome can be identified by generating labeled UNAs using the normal and disease genomic DNA as templates and performing the competitive hybridization with the labeled UNAs on an array comprising either oligonucleotide probes or cDNAs corresponding to defined genes or regions of the chromosome. The use of UNA targets could minimize or eliminate indirect cross-hybridization in which one target sequence binds to another target sequence, which is already bound to a defined probe. Thus, any potential for erroneously high signals resulting from indirect cross-hybridization would be minimized or eliminated making the CGH measurement more quantitative.

Other uses of UNAs include utilizing the inability of UNAs to serve as PCR templates in the presence of their complementary nucleotides. UNAs therefore could be used as the basis of a linear DNA amplifier analogous to the linear RNA amplifiers used to amplify and label cDNA samples prior to application to DNA microarrays.

It is appreciated by those of ordinary skill in the art that other modified nucleotides, nucleic acid polymerization enzymes and methods for generating UNAs not described in the specification may be used in accordance with the present invention. The following Examples are meant to provide experimental detail to the present invention and are not meant to limit the scope of the present invention.

**Examples**

*Example 1: Materials Preparation of the 2-amino-2'-deoxyadenosine-.5'-triphosphate and 2-thiothymidine-5'-triphosphate*

The 2-amino-2'-deoxyadenosine and 2-thiothymidine nucleosides were purchased from Chemgenes (Waltham MA) and Berry & Associates (Dexter MI) respectively. The nucleoside 5'-triphosphates were prepared by phosphorylation of the unprotected nucleosides using anhydrous phosphoryl chloride ($POCl_3$) and purified by chromatography on DEAE-sephadex according to the method of Seela and Gumbiowski *(Helvetica Chimica Acta,* (1991) 74, 1048). The purified nucleoside 5-triphosphates were >95% pure as determined by $^{31}P$ NMR and HPLC. The extinction coefficients ($mol^{-1}$ $cm^{-1}$) for the 2-amino-2'-deoxyadenosine-5'-triphosphate and 2-thiothymidine-5-triphosphate are $lambda_{255}$= 7,600 and $lambda_{276}$=16,600 respectively.

*Example 2: Incorporation of the 2-amino-2'-deoxyadenosine-5'-triphosphate and 2-thiothymidine-5-triphosphate into Polynucleotides by DNA Polymerases*

The ability of the *Bacillus sterothermophilus* (Bst) DNA polymerase (New England Biolabs), the *Thermus aquaticus* (Taq) DNA polymerase (Amersham) and the Moloney Murine Leukemia Virus reverse transcriptase (MMLV-RT) (Amersham) to incorporate the 2-amino-2'-deoxyadenosine-5'-triphosphate and 2-thiothymidine-5'-triphosphate into polynucleotides was tested using a synthetic 30-mer template and $^{32}P$-labeled 12-mer primer (S.A. 130 Ci/mmol). (Figure 2A). Extension reactions for each polymerase were performed in 0.65ml pre-siliconized microfuge tubes containing the following components: (Bst) 20 mM Tris-Cl (pH 8.8 @ 25° C), 10 mM KCl, 10 mM $(NH_4)_2SO_4$, 2 mM $MgSO_4$, 0.1% Triton-X100, 0.5 microM primer/template, 250 microM each dNTP and 0.15 units/microL Bst DNA polymerase; (Taq) 26 mM Tris-Cl (pH 9.5 @25° C), 6.5 mM $MgCl_2$, 0.5 microM primer/template, 250 microM each dNTP and 0.15 units/microL Taq DNA polymerase; (MMLV-RT) 50 mM Tris-Cl (pH 8.3 @ 25° C) 75 mM KCl, 3 mM $MgCl_2$, 1 M DTT, 0.5 microM primer/template, 250 microM each dNTP and 0.5 units/microL MMLV-RT. The reactions were incubated for 15 minutes at 65° C for the Bst and Taq reactions and 42° C for the MMLV-RT reaction. The reaction mixtures were separated by electrophoresis on 10% denaturing (7M urea) polyacrylamide gels and visualized by phosphorimaging methods.

In the reaction mixtures containing only dGTP, dCTP, dTTP or dATP, dGTP and dCTP, no full-length product was generated by any of the three polymerases (Figure 2B). However, when all four dNTPs were present or when 2-amino-2'-deoxyadenosine-5'-triphosphate was substituted for dATP or 2-thiothymidine-5'-triphosphate was substituted for dTTP, greater than 90% of the primer was converted to full-length 30-mer product. The incorporation of the 2-amino-2'-deoxyadenosine-5'-triphosphate by the Taq DNA polymerase is consistent with the results of Bailly and Waring *(Nucleic Acids Research,* (1995) 23:885). Importantly, full-length product was generated by all three polymerases when both the 2-aminoadenosine and 2-thiothymidine are substituted for A and T in a single reaction mixture.

To further assess the incorporation efficiency of the modified nucleotides by Bst DNA polymerase, the efficiency for a single nucleotide extension reaction was determined using two 6-mer primers in the presence of varying concentrations of dATP, 2-amino-2'-deoxyadenosine-5'-triphosphate, dTTP or 2-thiothymidine-5'-triphosphate (illustrated as 2-thioTTP) (Figure 3A and 4A). For this study, the reaction mixtures contained: 20 mM Tris-Cl (pH 8.8 @ 25° C), 10 mM KCl, 10 mM $(NH_4)_2SO_4$, 2 mM $MgSO_4$, 0.1% Triton-X 100, 500 nanoM 6-mer primer, 20 nanoM 30-mer DNA template, 0.8 units/microL (~70 nanoM) Bst DNA polymerase and a dNTP concentration ranging from 0.5 microM to 130 microM. The reaction mixtures were incubated at 45° C for 8 hours and separated by electrophoresis on 20% denaturing (7M urea) polyacrylamide gels.

The GACTGA 6-mer primer is extended with dATP and 2-amino-2'-deoxyadenosine-5'-triphosphate with approximately equal efficiencies (Figure 3B & 3C). Likewise, the extension efficiency of the GCTCTG 6-mer primer with the dTTP and 2-thiothymidine-5'-triphosphate (illustrated as S) are also very similar and exhibit a 50% incorporation at about 4 and 6 microM respectively (Figure 4B & 4C). These results indicate that the 2-amino-2'-deoxyadenosine-5'-triphosphate and 2-thiothymidine-5'-triphosphate are both very good substrates for the Bst DNA polymerase and possess $k_{cat}/K_m$ values near that of their natural counterparts.

To establish that the extension products generated in the presence of the 2-amino-2'-deoxyadenosine-5'-triphosphate and 2-thiothymidine-5'-triphosphate were not due to contaminating dATP and dTTP in the 2-amino-2'-deoxyadenosine-5'-triphosphate and 2-thiothymidine-5'-triphosphate preparations, a mass spectroscopic analysis was performed on the extension reaction mixtures using the 6-mer primers (Figure 5A). The extension reactions were performed in 0.65ml pre-siliconized microfuge tubes containing the following components: 20 mM Tris-Cl (pH 8.8 @ 25° C), 10 mM KCl, 10 mM $(NH_4)_2SO_4$, 2 mM $MgSO_4$, 0.1 % Triton-X100, 500 nM 6-mer primer, 20 nM 30-mer DNA template, 0.8 units/microL (~70 nM) Bst DNA polymerase and 20 microM dNTP. The reaction mixtures were incubated at 45° C for 4 hours and quenched with EDTA. 5 microL of the reaction mixture was mixed with 15 microL of distilled $H_2O$ and 20 microL of matrix solution (0.2 M 2,6-dihydroxyacetophenone, 0.2 M diammonium hydrogen citrate). One microliter samples were spotted and dried on a MALDI sample grid plate analyzed by Matrix Assisted Laser Desorption Ionization Time-of-Flight (MALDI-TOF) mass spectrometry.

The reaction mixtures containing the 6-mer primer GACTGA and either dATP or 2-amino-2'-deoxyadenosine-5'-triphosphate give single extension products having m/z values of 2130.2 and 2146.0 respectively (Figure 5B). This results in a 15.8 amu difference between the two 7-mer extension products which is consistent with the mass difference between the adenosine and the 2-aminoadenosine nucleotides or nucleosides (Figure 5C). Likewise, the reaction mixtures containing the 6-mer primer GCTCTG and either dTTP or 2-thiothymidine-5'-triphosphate (illustrated as S) give single extension reaction products having m/z values of 2089.7 and 2106.1 respectively. The resulting 16.4 amu difference between these two 7-mer extension products is consistent with the mass difference between the thymidine and 2-thiothymidine nucleotides or nucleosides (Figure 5C). These results conclusively show that both the 2-aminoadenosine and 2-thiothymidine nucleotides triphosphates are indeed incorporated by the Bst DNA polymerase and that the 2-amino-2'-deoxyadenosine-5'-triphosphate and 2-thiothymidine-5'-triphosphate preparations do not contain any contaminating dATP and dTTP respectively.

*Example 3: Synthesis of Single-stranded Polynucleotides*

Unstructured single-stranded UNA was generated by incorporating 2-aminoadenosine and 2-thiothymidine nucleotides using a 14-mer primer and 56-mer template (5'-phosphorylated) and Bst DNA polymerase followed by digestion of the DNA template with lambda exonuclease (Figure 6). Ten microliter extension reactions were performed in 0.65ml pre-siliconized microfuge tubes containing the following components: 20 mM Tris-Cl (pH 8.8 @ 25° C), 10 mM KCl, 10 mM $(NH_4)_2SO_4$, 2 mM $MaSO_4$, 0.1% Triton-X100, 1.0 microM primer/template, 500 microM dGTP, 500 microM dATP (or 2-amino-2'-deoxyadenosine-5'-triphosphate), 500 microM dTTP (or 2-thiothymidine-5'-triphosphate ), 200 microM$^{32}$P- -CTP and 0.8 units/microL Bst DNA polymerase. The reactions were incubated at 65° C for 30 minutes, quenched with 5 mM EDTA, ethanol precipitated, dried and resuspended in 10 microL of 10 mM Tris-Cl pH 8.0. The 56-mertemplate was digested by incubating the resuspended samples with 10 units of lambda exonuclease (Strandase Kit$^{TM}$, (Novagen; Madison, WI)) for 30 minutes at 37° C. The reactions were quenched with 5 mM EDTA, ethanol precipitated, dried and resuspended in 10 microL of 10 mM Tris-Cl pH 8.0. The samples were then electrophoresed on 10% denaturing (7M urea) polyacrylamide gels and visualized using phosphorimaging methods.

As shown in Figure 7, full-length 56-mer product is generated in the presence of either the four standard dNTPs or when 2-amino-2'-deoxyadenosine-5'-triphosphate and 2-thiothymidine-5'-triphosphate (illustrated as S) are substituted for dATP and dTTP respectively. In addition, little if any premature termination products are generated in the reactions containing 2-amino-2'-deoxyadenosine-5'-triphosphate and 2-thiothymidine-5'-triphosphate. Thus because the DNA template sequence includes three tandem A and T residues (bold text in Figure 6), the results show that the Bst DNA polymerase can efficiently incorporate the 2-aminoadenosine and 2-thiothymidine nucleotides at adjacent sites in the polynucleotide product.

*Example 4*: *Preparative Synthesis and Spectral Characterization of Single-stranded Polynucleotide Sequences Containing the 2-aminoadenosine and 2-thiothymidine Nucleotides*

Three related 56-mer polynucleotide sequences were designed which are predicted to form hairpin stem-loop structures of various stability flanked by a common sequence which is predicted to be unstructured (Figure 8). The 56-mer HP21AT forms a 10 base-pair stem closed by a thermodynamically stable (C)GAAA(G) tetra-loop (Antao, *et al., Nucleic Acids Research,* 19; 5901-5905 (1991)) and has a predicted Tm of 91° C (SantaLucia, *Proc. Natl. Acad Sci. USA,* 95; 1460-1465 (1998)). The 56-mer polynucleotides HP26AT and HP28AT are two and three nucleotide substitution variants of the HP21AT sequence which disrupt the stem structure and have predicted Tms of 69° C and 36° C respectively. HP21DS, HP26DS and HP28DS are the corresponding targets having the modified nucleotides 2-aminoadenosine and 2-thiothymidine which are expected to destabilize the stem structure by preventing stable A-T base pairing.

Using the polymerase extension method described above, the HP21, HP26 and HP28 56-mer polynucleotide sequences were synthesized having either the four standard nucleotides or having the two modified nucleotides 2-aminoadenosine and 2-thiothymidine in place of A and T. The polymerase extension reactions (250 uL) were performed in 0.65ml pre-siliconized microfuge tubes containing the following components: 20 mM Tris-Cl (pH 8.8 @ 25° C), 10 mM KCl, 10 mM $(NH_4)_2SO_4$, 2 mM $MgSO_4$, 0.1 % Triton-X 100, 2.0 microM 14-mer primer/56-mer (5'-phosphorylated) template, 500 microM dGTP, 500 microM dATP (or 2-amino-2'-deoxyadenosine-5'-triphosphate), 500 microM dTTP (or 2-thiothymidine-5'-triphosphate ), and 0.8 units/microL Bst DNA polymerase. The reactions were incubated at 65° C for 45 minutes, quenched with 5 mM EDTA, ethanol precipitated, dried and resuspended in 50 microL of 10 mM Tris-Cl pH 8.0. The 56-mer template was digested by incubating the resuspended samples with 40 units of lambda exonuclease (Strandase kitTM; Novagen, Madison, WI) for 45 minutes at 37° C. The reactions were quenched with 5 mM EDTA, ethanol precipitated, dried and resuspended in 15 microL of 10 mM Tris-Cl pH 8.0. The samples were then electrophoresed on 10% semi-denaturing (3.5M urea) polyacrylamide gels. The polynucleotide bands were visualized by UV-shadowing, excised and eluted from the gel in buffer containing 20 mM Tris-Cl pH 7.6, 100 mM NaCl, precipitated with ethanol, and dried under vacuum. The polynucleotides were resuspended in 100 uL of 1.0 mM Tris-Cl (pH 7.6) and quantitated by UV absorbance assuming an extinction coefficient of $\varepsilon_{260}$ = 5.12 x $10^5$ $M^{-1}$ for all six 56-mer polynucleotides.

The three 56-mer polynucleotides containing the 2-aminoadenosine and 2-thiothymidine modified nucleotides show the expected red shift in $\lambda_{max}$ (Figure 9). This is due to the presence of the modified nucleotide or nucleoside 2-aminoadenosine which has two peaks; one at 259nm and one at 280nm and the 2-thiothymidine which has a $\lambda_{max}$ at about 276 nm. A small amount (0.1 microgram) of the six purified 56-mer polynucleotide samples were separated by denaturing PAGE and stained with Stains-AllTM (Figure 10). The results show that all six 56-mer products are doublets that co-migrate with a 56 nucleotide single-stranded DNA marker. These analyses confirm that the polymerase efficiently incorporates the two modified nucleotides into the polynucleotide sequences.

The UNA polynucleotides can be further purified using ion-exchange or reverse-phase chromatography. It was found that the polynucleotides containing the 2-aminoadenosine and 2-thiothymidine modified nucleotides do not efficiently elute from ion-exchange columns such as ElutipTM Minicolumns (Schleicher & Schuell) under high salt conditions (e.g. 1M NaCl) unless a small amount (10%) of an organic solvent such as acetonitrile is present in the elution buffer (data not shown). Consistent with this result, it was found that the modified polynucleotides elute at higher acetonitrile concentrations than their natural counterpart when analyzed by HPLC using C-18 reverse-phase columns and triethylammonium acetate buffers (data not shown). This overall increase in hydrophobic character could be due to either direct chemical properties of the 2-aminoadenosine and 2-thiothymidine nucleotides or an increased exposure of the hydrophobic nucleotide resulting from the disruption of the natural secondary structure of the modified polynucleotides. Regardless of the specific mechanism responsible for this effect, it is suggested that all reaction vessels used for the synthesis, purification and assays of these types of UNAs be treated with a siliconizing agent to prevent non-specific binding of the UNAs to the reaction vessels.

*Example 5*: *Effect of the 2-aminoadenosine and 2-thiothymidine Nucleotides on Polynucleotide Secondary Structure; Polymerase Extension Assay*

The effect of incorporating the 2-aminoadenosine and 2-thiothymidine nucleotides into the polynucleotides on the polynucleotide's structure was assessed by determining their relative ability to promote a single nucleotide polymerase extension reaction using short 6-mer and 7-mer oligonucleotides as the primers (Figure 11). The 6-mer-543 and 7-mer-2169 have their complementary binding site located within stem structure of each of the target polynucleotides HP21. HP26 and HP28 (see Figure 8, bold text). The complementary binding site for the 6-mer-2978 lies outside of the stem-loop structure and serves as a control primer. The single-stranded target polynucleotide TarZT, which is predicted to have no secondary structure, served as a control target. All target sequences have a thymidine residue directly 5' to the primer binding sites which will direct the incorporation of a single ddATP residue into the primer sequence during the polymerase extension reaction.

The polymerase extension reactions (40 uL) were performed in 0.65ml pre-siliconized microfuge tubes containing the following components: 10 mM Tris-Cl (pH 8.4 at 25 o C), 0.05% Triton X-100, 1.0 mM $MgCl_2$, 320 microM $MnCl_2$, 80 microM ddATP, 10 nM target polynucleotide, 100 nM primer ($^{32}P$-labeled on the 5' terminus at S.A. 750 Ci/mmole) and 3.5 nM *Bacillus stearothermophilus* (Bst) DNA polymerase. The reaction mixtures were incubated at 45° C, and 8 uL aliquots were removed at 3, 6 and 24 hours, quenched with EDTA, separated by electrophoresis on 20% denaturing PAGE, and visualized using phosphorimaging methods.

As shown in Figures 12 and 13, the 6-mer-543 is efficiently extended to the 7-mer product in the presence of the control polynucleotide TarZT. In contrast, no extension product is produced after 24 hours in the presence of the polynucleotide HP21AT. This is expected since the 6-mer-543 binding site is buried within the secondary structure of the polynucleotide and not available for hybridization with the 6-mer primer. Importantly however, a detectable level of 7-mer product is generated in the presence of the related polynucleotide HP21DS, which contains the modified nucleotides 2-aminoadenosine and 2-thiothymidine. This result is even more dramatic for the single-nucleotide or -nucleoside extension reactions using the 7-mer-2169. In the presence of the polynucleotide HP21AT, no 8-mer product is generated after 24 hours whereas in the presence of the modified polynucleotide HP21DS, greater than 60% of the 7-mer-2169 is converted to the 8-mer product after 24 hours. These same trends hold true for the other two pairs of polynucleotide targets having the two and three nucleotide substitutions. HP26DS is a better target than its related HP26AT polynucleotide and HP28DS is a slightly better target than HP28AT.

Interestingly, the 2-amionadenosine and 2-thiothymidine-containing polynucleotides are more efficient targets for the extension of the 6-mer-2978 whose binding site lies outside of the stem-loop structure. This could, be due to a greater stability of the primer/target duplex for the 2-aminoadenosine and 2-thiothymidine-containing polynucleotides. It has been shown that A/2-thiothymidine and T/2-aminoadenosine base pairs are more stable than the standard A/T and T/A base pairs (Kutyavin *et al.* 1996). Importantly however, there is a correlation between the predicted stability of the polynucleotide's secondary structure and its efficiency as a target in the single-nucleotide or -nucleoside extension reaction. HP28 is a more efficient target than HP26 which, in turn, is a more efficient target than HP21 suggesting that intramolecular target structures near a primer binding site can effect the polymerase extension efficiency at that site. Thus because this same trend is exaggerated for the 2-aminoadenosine and 2-thiothymidine-containing polynucleotides, the results support the conclusion that the modifications do indeed alter the secondary structure of the polynucleotide targets. Regardless of the exact mechanism, these results clearly demonstrate that incorporating the 2-aminoadenosine and 2-thiothymidine nucleotide pair into a polynucleotide sequence increases the utility of the polynucleotide in hybridization-based assays.

*Example 6: Thermal Melting Profiles of UNA Compared to DNA*

Nucleic acids having secondary structure (hairpins) were synthesized enzymatically by primer extension in accordance with the teachings of the present invention to compare the thermal stability of a DNA hairpin compared to the same hairpin synthesized with modified nucleotides to reduce secondary structure (UNA). Figure 26 shows the nucleotide sequence of DNA having naturally occurring nucleotides A, T, C, and G (HP51 DNA) and the nucleotide sequence of a UNA (HP51 UNA) having the same nucleotide sequence except with 2-aminoadenosine substituted for A and 2-thiothymidine substituted for T at positions indicated within the hairpin structure.

The thermal melting profiles of the DNA and the UNA hairpins were obtained and are shown in Figure 27 (panels A and B). The absorbance of nucleic acid samples were measured at temperatures ranging from 20 degrees C to over 90 degrees C. Without limitations to theory, as duplex nucleic acids denature, the absorbance of the nucleic acid sample increases due to the exposure of the nucleotides which absorb UV light (for a representative study, see Sheardy *et al.* "A thermodynamic investigation of the melting of B-Z junction forming DNA oligomers." *Biochemistry* 33:1385-91, (1994.) The first derivatives of the profiles from Figures 27 (panels A and B) are provided in Figure 28 (panels A and B). A table in Figure 29 summarizes the data and shows that the enzymatic incorporation of 2-aminoadenosine and 2-thiothymidine into a nucleic acid molecules reduced the ability of the nucleic acid to form intramolecular hydrogen bond base pairs and thus decreased the thermal stability of the molecule by over 23 degrees C.

*Example 7: Incorporation Specificity of 2-thio-2'-deoxycytidine by DNA Polymerase*

UNAs of the present invention may contain the 2-thiocytidine/guanosine pair (2-thiocytosine/G) where the 2-thiocytosine and G are unable to form a stable base pair due to a steric clash within the minor groove. However, 2-thiocytosine and inosine (I) should form a stable base pair through two hydrogen bonds since the absence of the 2-amino group of guanine in inosine effectively removes the steric clash between the C2 Sulfur atom of 2-thiocytosine and the 2-amino group of guanine.

To demonstrate this base-pairing property, we tested the specificity of 2-thio-2'-deoxycytidine triphosphate incorporation by Bst DNA polymerase when either a single G or I was presented in the DNA template directly downstream of the primer binding site (see Figure 30 (panel A). The primer extension reactions (40.0 uL) were performed in 0.65 mL pre-siliconized microfuge tubes containing the following components: 20 mM Tris-Cl (pH 8.8), 10 mM KCl, 10 mM $(NH_4)_2SO_4$, 2 mM $MgSO_4$, 0.1% Triton-X100, 0.50 uM $^{32}$P-12mer primer, 0.75 uM template G or I, 5.0 uM dCTP or 2-thio-dCTP, and 0.4 units of Bst DNA polymerase. The reactions were incubated at 45°C. At 5, 10, 20 and 40 minutes, 7.0 uL aliquots were removed and the reaction mixture was separated by electrophoresis on 20%/7M urea polyacrylamide gels. The products were visualized by phosphorimaging.

As shown in Figure 30 (panel B), efficient conversion of the 12-mer primer to the 13-mer extension product is obtained with template G in the presence of dCTP but not in the presence of 2-thio-2'-deoxycytidine triphosphate (illustrated as dsCTP). For template I, however, the primer extension rate for dCTP is equivalent to that of 2-thio-2'-deoxycytidine triphosphate. Together, these data support the contention that the 2-thiocytidine does not form a stable base pair with guanosine yet is able to form a base pair with inosine with a stability similar to that of the natural cytidine/inosine base pair.

*Example 8: Incorporation of 2'-deoxyinosine-5'triphosphate, 2-amino-2'-deoxyadenosine-5'-triphosphate, 2-thiothymidine-5'-triphosphate and 2-thio-2'-deoxycytidine-5'-triphosphate into Polynucleotides by DNA Polymerase*

In order to synthesize a UNA having all natural base-pairing interactions destabilized, at least three modified nucleotides must be co-incorporated into the polynucleotide chain. This includes polynucleotides containing, but not limited to the set 2-aminoadenosine, I, C, and 2-thiothymine and the set 2-aminoadenosine, G, 2-thiocytosine and 2-thiothymine. The ability to generate these types of UNAs was tested using two primed DNA templates, one containing G at multiple positions and the other containing I at the analogous position (see Figure 31 (panel A)).

The extension reactions (20.0 uL) were performed in 0.65 mL pre-siliconized microfuge tubes containing the following components: 20 mM Tris-Cl (pH 8.8), 10 mM KCl, 10 mM $(NH_4)_2SO_4$, 2 mM $MgSO_4$, 0. 1% Triton-X100, 5% DMSO, 1.0 uM $^{32}$P-10mer primer, 1.0 uM Template G2 or 12, 0.2 mM dGTP (or dITP (I)), 0.2 mM dATP (or 2-amino-2'-deoxyadenosine-5'-triphosphate), 0.2 mM dCTP (or 2-thio-2'-deoxycytidine triphosphate), 0.2 mM TTP (or 2-thiothymidine-5'-triphosphate) and 8.0 units of Bst DNA polymerase. The reactions were incubated at 45°C. At 10 and 60 minutes, aliquots were removed and the reaction mixture was separated by electrophoresis on 20%/7M urea polyacrylamide gels. The products were visualized by phosphorimaging.

As shown in Figure 31 (panel B), full-length product is generated for template G2 in the presence of the four natural nucleotides (AGCT), the single I for G substitution (AICT) and the triple substitution containing 2-aminoadenosine (illustrated as D), I and 2-thiothymidine (illustrated as S) (DICS). The synthesis rate for the latter, however, is slightly reduced when compared to that for the product containing all natural nucleotides (AGCT). For template I2, full-length product is generated in the presence of the four natural nucleotides (AGCT), the single 2-thio-2'-deoxycytidine triphosphate (illustrated as sC in Figure 31 (panel B)) for C substitution (AGsCT) and the triple substitution containing 2-aminoadenosine for A, 2-thio2'-deoxycytidine triphosphate for C and 2-thiothymidine for T(DGsCS). While the rate of synthesis for the latter is the slowest of all that were tested, it is important to note that the rates for all products generated with the I2 template are slower than all those obtained with the G2 template. This suggests that the main contributing factor to the observed reduced rates is not attributable to the nucleotide analogues but more likely the presence of inosine in the template strand.

*Example 9: Synthesis of UNAs from Polyadenylated mRNAs*

The ability to synthesize full-length UNA copies (cUNAs) of polyadenylated RNA was tested using various reverse transcriptase enzymes and three T7-RNA transcripts having 3'-polyadenylated tails as templates. The three T7-RNA transcripts correspond to the genes for the penicillin binding protein from *E. coli* (pBp-1), the chlorophyll A/B binding protein from *Arabidopsis thaliana* (Cab), and nucleotides 5-335 of the Hepatitis C virus (HCV) genome (See Figure 32 (panel A)). The expected length of the cUNAs generated from the three polyadenylated RNAs using an oligo-dT primer is 1194, 729 and 360 nucleotides respectively (see Figure 32 (panel B)).

The reverse transcriptase reactions (15.0 uL) were performed in 0.65 mL pre-siliconized microfuge tubes containing the following components: 50 mM Tris-Cl (pH 8.3), 75 mM KCl, 3.0 mM $MjCl_2$, 4.5 mM DTT, 500 uM dGTP (or dITP), 500 uM dATP (or 2-amino-2'-deoxyadenosine-5'-triphosphate), 500 uM TTP (or 2-thiothymidine-5'-triphosphate), 100 uM dCTP, 0.33 uCi of $^{32}$P-$\alpha$-dCTP, 20 nM each T7-RNA template, 1.0 uM $T_{16}N_2$ primer, 1.0 unit of RNase Out (In Vitrogen), and 200 units of Superscript II MMLV reverse transcriptase (In Vitrogen). The buffer components, RNA template and primer were annealed by heating to 75°C for 4 minutes and quickly cooled to room temperature. The dNTPs and RNase inhibitor were then added and the reactions were initiated by the addition of the reverse transcriptase. After incubation at 42°C for 1 1/2 hours, the reactions were quenched with EDTA and 8 M urea. The reaction mixture was separated by electrophoresis on 5% polyacrylamide gels (19:1) containing 1x TBE and 9M urea and visualized by phosphorimaging methods.

As shown in Figure 33, full-length products are generated for all three template RNAs in the presence of the four natural dNTPs (A,G,C,T). Importantly, a similar amount of full-length product is generated for the three template RNAs when the UNA nucleotide triphosphate analogues 2-amino-2'-deoxyadenosine (illustrated as D) and 2-thiothymidine (illustrated as S) are substituted for dATP and TTP respectively. Full-length product is also generated in the reaction mixtures that contain the additional substitution of ITP (I) for GTP. However, in this case, the yield of product is reduced by about 2-fold relative to the natural dNTP or 2-amino-2'-deoxyadenosine and 2-thiothymidine substituted reactions. Moreover, full-length cUNA product is generated when all three templates are present in the reaction mixture. Similar results were also obtained for the ThermoScript RT (In Vitrogen) enzyme (data not shown). Thus, these data show that full length cUNA can be generated from complex RNA mixtures with reasonable yields using commercially available enzymes and standard reaction conditions.

The ability to generate fluorescently labeled cUNAs was tested using the pBp-1 T7-RNA template and dCTP-Cy5 fluorescently labeled nucleotide analogue (Perkin-Elmer/NEN). The reverse transcriptase reactions (10.0 uL) were performed in 0.65 mL pre-siliconized microfuge tubes containing the following components: 50 mM Tris-Cl (pH 8.3), 75 mM KCl, 3.0 mM $MgCl_2$, 4.5 mM DTT, 500 uM dGTP (or dITP), 500 uM dATP (or 2-amino-2'-deoxyadenosine-5'-triphosphate), 500 uM TTP (or 2-thiothymidine-5'-triphosphate), the indicated amounts of dCTP and dCTP-Cy5, 40 nM pBp-1 RNA template, 1.0 uM $T_{16}N_2$ primer, 1.0 unit of RNase Out (In Vitrogen), and 100 units of Superscript II MMLV reverse transcriptase (InVitrogen). The buffer components, RNA template, and primer were annealed by heating to 75°C for 4 minutes and quickly cooled to room temperature. The dNTPs and RNase inhibitor were then added and the reactions were initiated by the addition of the reverse transcriptase and incubated at 42°C. At one and three hours, 5.0 uL aliquots were removed, mixed with 0.07 ug of RNase A, and incubated at room temperature for 30 minutes. The ribonucleased reaction mixtures were quenched with EDTA and 8 M urea and separated by electrophoresis on 5% polyacrylamide gels (19:1) containing 1x TBE and 9M urea. The fluorescently-labeled products were visualized using a phosphorimager.

As shown in Figure 34, full-length fluorescently-labeled product is generated in one hour in the presence of either the four natural dNPTs (A,G,C,T) or when the UNA nucleotide triphosphate analogues 2-amino-2'-deoxyadenosine and 2-thiothymidine are substituted for dATP and TTP respectively. However, as observed above, the rate of full length cUNA synthesis is reduced about two to three-fold for the reaction mixtures that contain the additional substitution of ITP (I) for GTP. Nevertheless, these data clearly show that full-length fluorescently-labeled cUNAs can be easily generated using commercially available enzymes and standard reaction conditions.

*Example 10: Synthesis of Ribo-UNAs Using T7 RNA Polymerase*

The ability to transcribe longer ribo-UNAs (rUNAs) using T7 RNA polymerase and the corresponding modified ribonucleotide triphosphates was tested using a linear double-stranded DNA template. A plasmid DNA containing the human retinoblastoma gene under control of a T7 RNA polymerase promoter was digested with the restriction endonucleases XbaI and AflII. The resulting linearized plasmid templates for a 138 nucleotide transcript when transcribed with T7 RNA polymerase (see Figure 34A).

The transcription reactions (20 uL) contained 1x transcription buffer (Ambion Inc.), 0.5 mM GTP, 0.5 mM ATP (or 2-aminoadenosine-5'-triphosphate (rD)), 0.5 mM UTP (or 2-thiouridine-5'-triphosphate (rS), 1.6 uM $\alpha$-$^{32}$P-CTP, 1 ug of the linearized DNA template and 10 units of T7 RNA polymerase (Ambion Inc.). The reaction mixtures were incubated at 37°C for one hour and then separated by electrophoresis on a 6%/7M urea polyacrylamide gel.

As shown in Figure 34B, full-length product is generated in the presence of either the four natural nucleotide triphophates or when the 2-aminoadenosine-5'-triphosphate and 2-thiouridine-5'-triphosphate are substituted for ATP and UTP respectively. These data therefore show that relatively long rUNAs having a high specific activity can be generated using commercially available enzymes and standard protocols.

SEQUENCE LISTING

<110> AGILENT TECHNOLOGIES, INC

<120> Method of Producing Nucleic Acid Molecules with Reduced Secondary Structure

<130> IT/ED/N14452

<140> 03257914.6
<141> 2003-12-16

<150> US 10/324,409
<151> 2002-12-18

<160> 33

<170> PatentIn version 3.3

<210> 1
<211> 12
<212> DNA
<213> Artificial Sequence

<220>
<223> DNA primer and

template sequence used for the polymerase

extension reaction.

<400> 1
cgataggctc tg 12

<210> 2
<211> 30
<212> DNA
<213> Artificial Sequence

<220>
<223> Description of Artificial Sequence:DNA primer and

template sequence used for the polymerase

extension reaction.

<400> 2
ttgtccacag ttcagtccca gagcctatcg 30

<210> 3
<211> 30
<212> DNA
<213> Artificial Sequence

<220>
<223> Description of Artificial Sequence: The 6-mer DNA primer and template sequence used to test the

incorp of the -2'-deoxyadenosine-5'-triphosphates

in PCR.

<400> 3
ttgtccacag ttcagtcaca gagcctatcg 30

<210> 4
<211> 14
<212> DNA
<213> Artificial Sequence

<220>
<223> Description of Artificial Sequence:The Scheme for generating single-stranded polynucleotides using a primer template-dependent polymerase ext. reaction.

<400> 4
ctacctagcc tatc 14

<210> 5
<211> 56
<212> DNA
<213> Artificial Sequence

<220>
<223> Description of Artificial Sequence:The Scheme for generating single-stranded polynucleotides using a primer template-dependent polymerase ext. reaction.

<400> 5
tacgctgtgg acaaatgttg agactgtttc gagactgaac ttgatggatc ggatag 56

<210> 6
<211> 56
<212> DNA
<213> Artificial Sequence

<220>
<223> Description of Artificial Sequence:The Scheme for generating single-stranded polynucleotides using a primer template-dependent polymerase ext. reaction.

<400> 6
ctatccgatc catcaagttc agtctcgaaa cagtctcaac atttgtccac agcgta 56

<210> 7
<211> 28
<212> DNA

<213> Artificial Sequence

<220>
<223> Description of Artificial Sequence:Predicted secondary structures for three related 56-polynucleotide seqs containing either the four natural (A,G,C,T) nucleotides

<400> 7
ctatccgatc catcaagttc agtctcga 28

<210> 8
<211> 28
<212> DNA
<213> Artificial Sequence

<220>
<223> Description of Artificial Sequence:Predicted secondary structures for three related 56-polynucleotide seqs containing either the four natural (A,G,C,T) nucleotides.

<400> 8
ctatccgatc catcddgssc dgscscgd 28

<210> 9
<211> 28
<212> DNA
<213> Artificial Sequence

<220>
<223> Description of Artificial Sequence: Predicted secondary structures for three related 56-polynucleotide seqs containing either the four natural (A,G,C,T) nucleotides.

<400> 9
aagagtctca acatttgtcc acagcgta 28

<210> 10
<211> 28
<212> DNA
<213> Artificial Sequence

<220>
<223> Description of Artificial Sequence: Predicted secondary structures for three related 56-polynucleotide seqs containing either the four natural (A,G,C,T) nucleotides.

<400> 10
ddgdgscscd dcdsssgscc dcdgcgsd 28

<210> 11
<211> 28
<212> DNA
<213> Artificial Sequence

<220>
<223> Description of Artificial Sequence: Predicted seconday structures for three related 56-polynucleotide seqs containing either the four naTural (A,G,C,T) nucleotides

<400> 11
aacagtctca acatttgtcc acagcgta 28

<210> 12
<211> 28
<212> DNA
<213> Artificial Sequence

<220>
<223> Description of Artificial Sequence: Predicted secondary structures for three related 56-polynucleotide seqs containing either the four natural (A,G,C,T) nucleotides.

<400> 12
ddcdgscscd dcdsssgscc dcdgcgsd 28

<210> 13
<211> 56
<212> DNA
<213> Artificial Sequence

<220>
<223> Description of Artificial Sequence:The DNA primer and temp seqs used to test the effect of the polynucleotide secondary structure on the polymerase ext reaction.

<400> 13
ctatccgatc catcaagttc agtctcgaaa gagactgaac atttgtccac agcgta 56

<210> 14
<211> 56
<212> DNA
<213> Artificial Sequence

<220>
<223> Description of Artificial Sequence:The DNA primer and temp seqs used to test the effect of the polynucleotide secondary structure on the polymerase extension reaction

<400> 14

ctatccgatc catcaagttc agtctcgaaa gagtctcaac atttgtccac agcgta 56

<210> 15
<211> 13
<212> DNA
<213> Artificial Sequence

<220>
<223> Description of Artificial Sequence: The DNA primer and temp seqs used to test the effect of the polynucleotide secondary structure on the polymerase ext reaction.

<400> 15
tttttttttt ttt 13

<210> 16
<211> 17
<212> DNA
<213> Artificial Sequence

<220>
<223> Description of Artificial Sequence: Anneal Primer

<400> 16
aaaaaaaaaa aaaaaaa 17

<210> 17
<211> 14
<212> DNA
<213> Artificial Sequence

<220>
<223> Description of Artificial Sequence: Incubate with DNA Polymerases.

<400> 17
tttttttttt tttt 14

<210> 18
<211> 22
<212> DNA
<213> Artificial Sequence

<220>
<223> Description of Artificial Sequence: Incubate with DNA Polymerases.

<220>
<221> misc_feature
<222> (1)..(4)
<223> n is a, c, g, or t

<400> 18
nnnngagctt tttttttttt tt 22

<210> 19
<211> 18
<212> DNA
<213> Artificial Sequence

<220>
<223> Description of Artificial Sequence: Heat Kill SP and add BstNB I Nicking Enzyme.

<220>
<221> misc_feature
<222> (1)..(4)
<223> n is a, c, g, or t

<400> 19
nnnngagctt tttttttt 18

<210> 20
<211> 24
<212> DNA
<213> Artificial Sequence

<220>
<223> Description of Artificial Sequence: Predicted Thermodynamic Parameters.

<400> 20
ccgatccatc aagttcagtc tcga 24

<210> 21
<211> 14
<212> DNA
<213> Artificial Sequence

<220>
<223> Description of Artificial Sequence: Predicted Thermodynamic Parameters.

<400> 21
aagagactga acac 14

<210> 22
<211> 24
<212> DNA
<213> Artificial Sequence

<220>
<223> Description of Artificial Sequence: Predicted Thermodynamic Parameters.

<400> 22

ccgatccatc ddgsscdgsc scgd 24

<210> 23
<211> 14
<212> DNA
<213> Artificial Sequence

<220>
<223> Description of Artificial Sequence: Predicted

Thermodynamic Parameters.

<400> 23
ddgdgdcsgd dcdc 14

<210> 24
<211> 12
<212> DNA
<213> Artificial Sequence

<220>
<223> Description of Artificial Sequence: Depicts is the specificity of 2-thio-2'-deoxycytidine triphosphate incorp. by Bst DNA polymerase when either a single G or I was presented in the DNA template

<400> 24
ttgtccacag tt 12

<210> 25
<211> 30
<212> DNA
<213> Artificial Sequence

<220>
<223> Description of Artificial Sequence: Depicts is the specificity of 2-thio-2'-deoxycytidine triphosphate incorporation by Bst DNA polymerase when either a single G or I was presented in the DNA template

<400> 25
cgataggctc tgagactgaa ctgtggacaa 30

<210> 26
<211> 30
<212> PRT
<213> Artificial sequence

<220>
<223> Description of Artificial Sequence: Depicts is the specificity of 2-thio-2'-deoxycytidine triphosphate incorporation by Bst DNA polymerase when either a single G or I was presented in the DNA template

<400> 26

Cys Ile Ala Thr Ala Ile Ile Cys Thr Cys Thr Ile Ala Ile Ala Cys
1 5 10 15

Thr Ile Ala Ala Cys Thr Ile Thr Ile Ile Ala Cys Ala Ala
20 25 30

<210> 27
<211> 10
<212> DNA
<213> Artificial Sequence

<220>
<223> Description of Artificial Sequence: Synthesis of a UNA having all natural base pairing interactions destablized, using two primed DNA templates.

<400> 27
ccgatccatc 10

<210> 28
<211> 47
<212> DNA
<213> Artificial Sequence

<220>
<223> Description of Artificial Sequence: Synthesis of a UNA having all natural base pairing interactions destablized, using two primed DNA templates.

<400> 28
gtgttcagtc tctttcgaga ctgaacttga tggatcggat agttttt 47

<210> 29
<211> 47
<212> PRT
<213> Artificial Sequence

<220>
<223> Description of Artificial Sequence: Synthesis of a UNA having all natural base pairing interactions destablized, using two primed DNA templates

<400> 29

Ile Thr Ile Thr Thr Cys Ala Ile Thr Cys Thr Cys Thr Thr Thr Cys
1 5 10 15

Ile Ala Ile Ala Cys Thr Ile Ala Ala Cys Thr Thr Gly Ala Thr Gly
20 25 30

Gly Ala Thr Cys Gly Gly Ala Thr Ala Gly Thr Thr Thr Thr Thr
35 40 45

<210> 30
<211> 1194
<212> RNA
<213> Artificial Sequence

<220>
<223> Description of Artificial Sequence: Penicillin-binding protein 1C from E.

Coli

<400> 30
ggcgcaggau gguacgccgc ucuggcgcuu cgccgaugcu gacggcaucu ggcguuaucc 60
gguaacaauc gaagauguuu cuccacguua ccuugaagcg cugaucaauu augaagaucg 120
cugguucugg aagcauccgg gggugaaucc auucucggug gcgcgcgcag cauggcaaga 180
ucucacuucg ggacggguua uuuccggugg cagcacgcuc acuaugcagg uugcucgucu 240
gcuugauccu caccccaaaa cauuuggcgg caaaauucgc cagcucuggc gcgcguugca 300
acuggaaugg caucugucua agcgugaaau ucugaccuug uaucuuaacc gcgcuccguu 360
uggcgguacg uugcagggga ucggugcggc aaguugggcu uaucucggaa aaucgccugc 420
gaauuuaagc uauuccgagg cggcaaugcu ggcgguuuug ccgcaagcgc ccagccgucu 480
ucgcccggau cguuggccgg agcgugccga agccgcgcgu aauaaagugc ucgaacggau 540
ggccgugcaa ggugugugu cccgugagca gguaaaagag ucaagggaag aacccaucug 600
gcuggccccc cgacaaaugc cgcaacuggc accgcuguuu ucgcgcauga ugcucgguaa 660
aagcaaaagc gacaaaauca cuacuacguu ggaugccggu cuucaacgac gucuggaaga 720
acuggcgcaa aacuggaaag ggcgguugcc accgcgcagc ucacuggcga ugaucguggu 780
ugaucauacc gauaugcgug uucgcggcug ggugggaucg guugaucuca acgaugauuc 840
acgcuuuggu cauguugaua uggucaauuc gauccgaucg ccaggaucag ugcucaaacc 900
guuuguuuau ggucuggcgc uggaugaagg cuugauccac ccggcaucac ugcugcaaga 960
cguccccggg cgcaccggug auuaucgacc agguaacuuu gauagcgguu uucauggccc 1020
gaucagcaug agcgaggcgc uggugcgcuc gcugaacuua ccugcugugc aggugcugga 1080
agccuaugga ccgaaacggu uugcggcaaa guuacgcaau guuggauugc cguuauauuu 1140
gcccaacggu gcugcgccga aucuuucacu caaaaaaaaa aaaaaaaaaa aaaa 1194

<210> 31
<211> 736
<212> RNA
<213> Artificial Sequence

<220>
<223> Description of Artificial Sequence: Chlorophyll

A/B-binding protein from Arabidopsis Thalana.

<400> 31
cuuccaaguc uaaauucgua uccgccggag uuccacuccc aaacgccggg aauguugguc 60
guaucagaau ggcugcucac uggaugccug gcgagccacg accagcuuac cuugacgguu 120
cugcuccugg ugacuuuggg uuugacccac uuggacuugg agaaguucca gcgaaccuug 180
agagauacaa agagucagag cucauccacu guagaugggc uaugcucgcu guuccuggga 240
uuuugguacc agaagcauua ggauauggaa acuggguuaa ggcucaggaa ugggcagcac 300
uaccaggggg ucaagccacu uacuugggaa acccaguccc guggguacu uugcccacaa 360
ucuuggccau ugaguucuua gccauugcau uuguugagca ccagagaagu auggagaaag 420
acccugagaa gaagaaguac ccgggaggcg cauuugaccc ucuuggauac ucgaaggacc 480
ccaagaagcu cgaggaauug aaaguuaaag agaucaagaa cgggcggcuu gcgcuguugg 540
cguuuguagg auucugugug caacagucgg cuuacccggg gacaggacca uuggagaacu 600
uggcaacuca cuuggcggau ccauggcaca acaacauugg cgauauuguu aucccuuuca 660
acuaaugaau guaaaaauag aaauaugugu accuuaugag cuuuaugugu aucaaaaaaa 720
aaaaaaaaaa aaaaaa 736

<210> 32
<211> 374
<212> DNA
<213> Artificial Sequence

<220>
<223> Description of Artificial Sequence: Nucleotides 1-335 for the Hepatitis C Virus Genome.

<400> 32
ttgggggcga cactccacca tagatcactc ccctgtgagg aactactgtc ttcacgcaga 60
aagcgtctag ccatggcgtt agtatgagtg ttgtgcagcc tccaggaccc cccctcccgg 120
gagagccata gtggtctgcg gaaccggtga gtacaccgga attgccagga cgaccgggtc 180
ctttcttgga tcaacccgct caatgcctgg agatttgggc gtgcccccgc gagactgcta 240
gccgagtagt gttgggtcgc gaaaggcctt gtggtactgc ctgatagggt gcttgcgagt 300
gccccgggag gtctcgtaga ccgtgcatca tgagctcgac gagctcccaa aaaaaaaaaa 360
aaaaaaaaaa aaaa 374

<210> 33
<211> 16
<212> DNA
<213> Artificial Sequence

<220>
<223> Description of Artificial Sequence: Poly T Prime

<220>
<221> misc_feature
<222> (15)..(16)
<223> n is a, c, g, or t

<400> 33
tttttttttt ttttnn 16

SEQUENCE LISTING

<110> Sampson, et al.

<120> Method of Producing Nucleic Acid Molecules with Reduced Secondary Structure

<130> 2003309-0028

<140> 10/324,409
<141> 2002-12-18

<160> 33

<170> PatentIn Ver. 2.1

<210> 1
<211> 12
<212> DNA
<213> Artificial Sequence

<220>
<223> Description of Artificial Sequence:DNA primer and template sequence used for the polymerase extension reaction.

<220>
<223> Description of Combined DNA/RNA Molecule:hkj

<400> 1
cgataggctc tg 12

<210> 2
<211> 30
<212> DNA
<213> Artificial Sequence

<220>
<223> Description of Artificial Sequence:DNA primer and template sequence used for the polymerase extension reaction.

<400> 2
ttgtccacag ttcagtccca gagcctatcg 30

<210> 3

<211> 30
<212> DNA
<213> Artificial Sequence

<220>
<223> Description of Artificial Sequence: The 6-mer DNA primer and template sequence used to test the incorp of the -2'-deoxyadenosine-5'-triphosphates in PCR..

<400> 3
ttgtccacag ttcagtcaca gagcctatcg 30

<210> 4
<211> 14
<212> DNA
<213> Artificial Sequence

<220>
<223> Description of Artificial Sequence:The Scheme for generating single-stranded polynucleotides using a primer template-dependent polymerase ext. reaction.

<400> 4
ctacctagcc tatc 14

<210> 5
<211> 56
<212> DNA
<213> Artificial Sequence

<220>
<223> Description of Artificial Sequence:The Scheme for generating single-stranded polynucleotides using a primer template-dependent polymerase ext. reaction.

<400> 5
tacgctgtgg acaaatgttg agactgtttc gagactgaac ttgatggatc ggatag 56

<210> 6
<211> 56
<212> DNA
<213> Artificial Sequence

<220>
<223> Description of Artificial Sequence:The Scheme for generating single-stranded polynucleotides using a primer template-dependent polymerase ext. reaction.

<400> 6
ctatccgatc catcaagttc agtctcgaaa cagtctcaac atttgtccac agcgta 56

<210> 7
<211> 28
<212> DNA
<213> Artificial Sequence

<220>
<223> Description of Artificial Sequence:Predicted secondary structures for three related 56-polynucleotide seqs containing either the four natural (A,G,C,T) nucleotides.

<400> 7
ctatccgatc catcaagttc agtctcga 28

<210> 8
<211> 28
<212> DNA
<213> Artificial Sequence

<220>
<223> Description of Artificial Sequence:Predicted secondary structures for three related 56-polynucleotide seqs containing either the four natural (A,G,C,T) nucleotides.

<400> 8
ctatccgatc catcddgssc dgscscgd 28

<210> 9
<211> 28
<212> DNA
<213> Artificial Sequence

<220>
<223> Description of Artificial Sequence:Predicted

secondary structures for three related 56-polynucleotide seqs containing either the four natural (A,G,C,T) nucleotides.

<400> 9
aagagtctca acatttgtcc acagcgta 28

<210> 10
<211> 28
<212> DNA
<213> Artificial Sequence

<220>
<223> Description of Artificial Sequence:Predicted secondary structures for three related 56-polynucleotide seqs containing either the four natural (A,G,C,T) nucleotides.

<400> 10
ddgdgscscd dcdsssgscc dcdgcgsd 28

<210> 11
<211> 28
<212> DNA
<213> Artificial Sequence

<220>
<223> Description of Artificial Sequence:Predicted secondary structures for three related 56-polynucleotide seqs containing either the four natural (A,G,C,T) nucleotides.

<400> 11
aacagtctca acatttgtcc acagcgta 28

<210> 12
<211> 28
<212> DNA
<213> Artificial Sequence

<220>
<223> Description of Artificial Sequence:Predicted secondary structures for three related 56-polynucleotide seqs containing either the four natural (A,G,C,T) nucleotides.

<400> 12
ddcdgscscd dcdsssgscc dcdgcgsd 28

<210> 13
<211> 56
<212> DNA
<213> Artificial Sequence

<220>
<223> Description of Artificial Sequence:The DNA primer and temp seqs used to test the effect of the polynucleotide secondary structure on the polymerase ext reaction.

<400> 13
ctatccgatc catcaagttc agtctcgaaa gagactgaac atttgtccac agcgta 56

<210> 14
<211> 56
<212> DNA
<213> Artificial Sequence

<220>
<223> Description of Artificial Sequence:The DNA primer and temp seqs used to test the effect of the polynucleotide secondary structure on the polymerase ext reaction.

<400> 14
ctatccgatc catcaagttc agtctcgaaa gagtctcaac atttgtccac agcgta 56

<210> 15
<211> 13
<212> DNA
<213> Artificial Sequence

<220>
<223> Description of Artificial Sequence:The DNA primer and temp seqs used to test the effect of the polynucleotide secondary structure on the polymerase ext reaction.

<400> 15
tttttttttt ttt 13

<210> 16
<211> 17
<212> DNA
<213> Artificial Sequence

<220>
<223> Description of Artificial Sequence:Anneal Primer

<400> 16
aaaaaaaaaa aaaaaaa 17

<210> 17
<211> 14
<212> DNA
<213> Artificial Sequence

<220>
<223> Description of Artificial Sequence:Incubate with DNA Polymerases.

<400> 17
tttttttttt tttt 14

<210> 18
<211> 22
<212> DNA
<213> Artificial Sequence

<220>
<223> Description of Artificial Sequence:Incubate with DNA Polymerases.

<400> 18
nnnngagctt tttttttttt tt 22

<210> 19
<211> 18
<212> DNA
<213> Artificial Sequence

<220>
<223> Description of Artificial Sequence:Heat Kill SP and add BstNB I Nicking Enzyme.

<400> 19
nnnngagctt tttttttt 18

<210> 20
<211> 24
<212> DNA
<213> Artificial Sequence

<220>
<223> Description of Artificial Sequence:Predicted Thermodynamic Parameters.

<400> 20
ccgatccatc aagttcagtc tcga 24

<210> 21
<211> 14
<212> DNA
<213> Artificial Sequence

<220>
<223> Description of Artificial Sequence:Predicted Thermodynamic Parameters.

<400> 21
aagagactga acac 14

<210> 22
<211> 24
<212> DNA
<213> Artificial Sequence

<220>
<223> Description of Artificial Sequence:Predicted Thermodynamic Parameters.

<400> 22
ccgatccatc ddgsscdgsc scgd 24

<210> 23
<211> 14
<212> DNA
<213> Artificial Sequence

<220>
<223> Description of Artificial Sequence:Predicted Thermodynamic Parameters.

<400> 23
ddgdgdcsgd dcdc 14

<210> 24
<211> 12
<212> DNA
<213> Artificial Sequence

<220>
<223> Description of Artificial Sequence:Depicts is the specificity of 2-thio-2'-deoxycytidine triphosphate incorp. by Bst DNA polymerase when either a single G or I was presented in the DNA template.

<400> 24
ttgtccacag tt 12

<210> 25
<211> 30
<212> DNA
<213> Artificial Sequence

<220>
<223> Description of Artificial Sequence:Depicts is the specificity of 2-thio-2'-deoxycytidine triphosphate incorporation by Bst DNA polymerase when either a single G or I was presented in the DNA template.

<400> 25
cgataggctc tgagactgaa ctgtggacaa 30

<210> 26
<211> 30
<212> PRT
<213> Artificial Sequence

<220>
<223> Description of Artificial Sequence:Depicts is the

specificity of 2-thio-2'-deoxycytidine triphosphate incorporation by Bst DNA polymerase when either a single G or I was presented in the DNA template.

<400> 26
Cys Ile Ala Thr Ala Ile Ile Cys Thr Cys Thr Ile Ala Ile Ala Cys
1 5 10 15

Thr Ile Ala Ala Cys Thr Ile Thr Ile Ile Ala Cys Ala Ala
20 25 30

<210> 27
<211> 10
<212> DNA
<213> Artificial Sequence

<220>
<223> Description of Artificial Sequence:Synthesis of a UNA having all natural base pairing interactions destablized, using two primed DNA templates.

<400> 27
ccgatccatc 10

<210> 28
<211> 47
<212> DNA
<213> Artificial Sequence

<220>
<223> Description of Artificial Sequence:Synthesis of a UNA having all natural base pairing interactions destablized, using two primed DNA templates.

<400> 28
gtgttcagtc tctttcgaga ctgaacttga tggatcggat agttttt 47

<210> 29
<211> 47
<212> PRT
<213> Artificial Sequence

<220>
<223> Description of Artificial Sequence:Synthesis of a

UNA having all natural base pairing interactions destablized, using two primed DNA templates.

<400> 29
Ile Thr Ile Thr Thr Cys Ala Ile Thr Cys Thr Cys Thr Thr Thr Cys
1 5 10 15

Ile Ala Ile Ala Cys Thr Ile Ala Ala Cys Thr Thr Gly Ala Thr Gly
20 25 30

Gly Ala Thr Cys Gly Gly Ala Thr Ala Gly Thr Thr Thr Thr Thr
35 40 45

<210> 30
<211> 1194
<212> RNA
<213> Artificial Sequence

<220>
<223> Description of Artificial Sequence:Penicillin-binding protein 1C from E. Coli

<400> 30
ggcgcaggau gguacgccgc ucuggcgcuu cgccgaugcu gacggcaucu ggcguuaucc 60
gguaacaauc gaagauguuu cuccacguua ccuugaagcg cugaucaauu augaagaucg 120
cugguucugg aagcauccgg gggugaaucc auucucggug gcgcgcgcag cauggcaaga 180
ucucacuucg ggacggguua uuuccggugg cagcacgcuc acuaugcagg uugcucgucu 240
gcuugauccu caccccaaaa cauuuggcgg caaaauucgc cagcucuggc gcgcguugca 300
acuggaaugg caucugucua agcgugaaau ucugaccuug uaucuuaacc gcgcuccguu 360
uggcgguacg uugcagggga ucggugcggc aaguugggcu uaucucggaa aaucgccugc 420
gaauuuaagc uauuccgagg cggcaaugcu ggcgguuuug ccgcaagcgc ccagccgucu 480
ucgcccggau cguuggccgg agcgugccga agccgcgcgu aauaaagugc ucgaacggau 540
ggccgugcaa ggugugugggu cccgugagca gguaaaagag ucaagggaag aacccaucug 600
gcuggccccc cgacaaaugc cgcaacuggc accgcuguuu ucgcgcauga ugcucgguaa 660
aagcaaaagc gacaaaauca cuacuacguu ggaugccggu cuucaacgac gucuggaaga 720
acuggcgcaa aacuggaaag ggcgguugcc accgcgcagc ucacuggcga ugaucguggu 780
ugaucauacc gauaugcgug uucgcggcug ggugggaucg guugaucuca acgaugauuc 840
acgcuuuggu cauguugaua uggucaauuc gauccgaucg ccaggaucag ugcucaaacc 900
guuuguuuau ggucuggcgc uggaugaagg cuugauccac ccggcaucac ugcugcaaga 960
cguccccgg cgcaccggug auuaucgacc agguaacuuu gauagcgguu uucauggccc 1020
gaucagcaug agcgaggcgc uggugcgcuc gcugaacuua ccugcugugc aggugcugga 1080
agccuaugga ccgaaacggu uugcggcaaa guuacgcaau guuggauugc cguuauauuu 1140
gcccaacggu gcugcgccga aucuuucacu caaaaaaaaa aaaaaaaaaa aaaa 1194

<210> 31

<211> 736
<212> RNA
<213> Artificial Sequence

<220>
<223> Description of Artificial Sequence:Chlorophyll A/B-binding protein from Arabidopsis Thalana.

<400> 31
cuuccaaguc uaaauucgua uccgccggag uuccacuccc aaacgccggg aauguugguc 60
guaucagaau ggcugcucac uggaugccug gcgagccacg accagcuuac cuugacgguu 120
cugcuccugg ugacuuuggg uuugacccac uuggacuugg agaaguucca gcgaaccuug 180
agagauacaa agagucagag cucauccacu guagaugggc uaugcucgcu guuccuggga 240
uuuugguacc agaagcauua ggauauggaa acugguuaa ggcucaggaa ugggcagcac 300
uaccaggggg ucaagccacu uacuugggaa acccaguccc gugggguacu uugcccacaa 360
ucuuggccau ugaguucuua gccauugcau uuguugagca ccagagaagu auggagaaag 420
acccugagaa gaagaaguac ccgggaggcg cauuugaccc ucuuggauac ucgaaggacc 480
ccaagaagcu cgaggaauug aaaguuaaag agaucaagaa cgggcggcuu gcgcuguugg 540
cguuuguagg auucugugug caacagucgg cuuacccggg gacaggacca uuggagaacu 600
uggcaacuca cuuggcggau ccauggcaca acaacauugg cgauauuguu aucccuuuca 660
acuaaugaau guaaaaauag aaauaugugu accuuaugag cuuuaugugu aucaaaaaaa 720
aaaaaaaaaa aaaaaa 736

<210> 32
<211> 374
<212> DNA
<213> Artificial Sequence

<220>
<223> Description of Artificial Sequence:Nucleotides 1-335 for the Hepatitis C Virus Genome.

<400> 32
ttgggggcga cactccacca tagatcactc ccctgtgagg aactactgtc ttcacgcaga 60
aagcgtctag ccatggcgtt agtatgagtg ttgtgcagcc tccaggaccc cccctcccgg 120
gagagccata gtggtctgcg gaaccggtga gtacaccgga attgccagga cgaccgggtc 180
ctttcttgga tcaacccgct caatgcctgg agatttgggc gtgcccccgc gagactgcta 240
gccgagtagt gttgggtcgc gaaaggcctt gtggtactgc ctgatagggt gcttgcgagt 300
gccccgggag gtctcgtaga ccgtgcatca tgagctcgac gagctcccaa aaaaaaaaaa 360
aaaaaaaaaa aaaa 374

<210> 33
<211> 16
<212> DNA
<213> Artificial Sequence

```
<220>
<223> Description of Artificial Sequence:Poly T Prime.

<400> 33
tttttttttt ttttnn                                                    16
```

## Claims

1. A first unstructured nucleic acid molecule or mixture of molecules with reduced secondary structure relative to a nucleic acid molecule or mixture of molecules of substantially identical nucleotide sequence having naturally occurring nucleotides, wherein at least some of the molecules possess at least two sequence elements that are substantially complementary to one another, wherein said complementary sequence elements have reduced levels of base-pairing stability relative to a nucleic acid molecule of substantially identical nucleotide sequence having naturally occurring nucleotides, wherein said complementary sequence elements of the first unstructured nucleic acid molecule can form stable base-pairs with substantially complementary sequence elements present in a second nucleic acid molecule.

2. A mixture of first nucleic acid molecules having reduced levels of intermolecular hybridization stability relative to a nucleic acid molecule or mixture of molecules of substantially identical nucleotide sequence having naturally occurring nucleotides, wherein at least two sequence elements within said mixture of first nucleic acids molecules are substantially complementary to one another, wherein said complementary sequence elements have reduced levels of base-pairing stability relative to nucleic acid molecules of substantially identical nucleotide sequence having naturally occurring nucleotides, wherein said complementary sequence elements within said mixture of first nucleic acids molecules having reduced levels of intermolecular hybridization stability can form stable base-pairs with a substantially complementary sequence element present in a second nucleic acid molecule.

3. The first unstructured nucleic acid or mixture of molecules according to claim 1 or 2 comprising:

   (a) nucleotides selected from the group consisting of: 2-amino-2'-deoxyadenosine-monophosphate, 2-thio-2'-deoxythymidine-monophosphate, 2'-deoxyinosine-monophosphate, 2'-deoxypyrrolopyrimidine-monophosphate, 2-thio-2'-deoxycytidine-monophosphate, 2'-deoxyguanosine-monophosphate, 2'-deoxycytidine-monophosphate, 2'-deoxyadenosine-monophosphate, 2'-deoxythymidine-monophosphate, and modifications thereof; or
   (b) nucleotides selected from the group consisting of: 2-aminoadenosine-5'-monophosphate, 2-thiouridine-monophosphate, inosine-monophosphate, pyrrolopyrimidine-monophosphate, 2-thiocytidine-monophosphate, guanosine-monophosphate, cytidine-monophosphate, adenosine-monophosphate, uridine-monophosphate, and modifications thereof; or
   (c) the nucleotides A, A', G, G', T, T', C and C', wherein A' does not form a stable base pair with T', and G' does not form a stable base pair with C', wherein said first nucleic acid can form stable base pairs with said second nucleic acid comprising the nucleotides A, A*, G, G*, T, T*, C, and C*, wherein A* can form a stable base pair with T' and G* can form a stable base pair with C' and T* can form a stable base pair with A' and C* can form a stable base pair with G'; or
   (d) the nucleotides A', G', T', and C', wherein A' does not form a stable base pair with T', and G' does not form a stable base pair with C', wherein said first nucleic acid can form stable base pairs with said second nucleic acid comprising the nucleotides A*, G*, T*, and C*, wherein A* can form a stable base pair with T' and G* can form a stable base pair with C' and T* can form a stable base pair with A' and C* can form a stable base pair with G'; or
   (e) the nucleotides A', G, T', C, wherein A' does not form a stable base pair with T', wherein said first nucleic acid can form stable base pairs with said second nucleic acid comprising the nucleotides A*, G, T*, C, wherein A* can form a stable base pair with T' and T* can form a stable base pair with A'; or
   (f) the nucleotides A, G', T, C' wherein G' does not form a stable base pair with C', wherein said first nucleic acid can form stable base pairs with said second nucleic acid comprising the nucleotides A, G*, T, C*, wherein G* can form a stable base pair with C' and C* can form a stable base pair with G'.

4. A method of generating nucleic acid having a reduced ability to hybridize, comprising steps of:

providing at least one nucleic acid template strand;
providing a collection of nucleotide triphosphates sufficient to synthesize a first nucleic acid strand complementary to at least a portion of the template nucleic acid strand, wherein the collection of nucleotide triphosphates includes at least one complementary nucleotide pair, wherein said pair has a reduced ability to form stable base pairs but can form stable base pairs with nucleotides present in a second nucleic acid molecule; and
contacting the template nucleic acid strand and the nucleotide triphosphates with an enzyme **characterized by** the ability to incorporate the nucleotide triphosphates into a new strand complementary to the template nucleic acid strand.

5. The method of claim 4, wherein

(a) the resulting new strand that is complementary to the template nucleic acid strand has a reduced ability to form intramolecular base pairs; or
(b) a mixture of two or more nucleic acid template strands is provided and the resulting new strands that are complementary to the template nucleic acid strands have a reduced ability to form intermolecular base-pairs; or
(c) in the step of providing a template strand, the template strand comprises synthetic RNA, in vitro transcribed RNA, total cellular RNA, messenger RNA, ribosomal RNA, transfer RNA, synthetic DNA, genomic DNA, cDNA, plasmid DNA, virion DNA, phage DNA, PCR amplified DNA, rolling circle amplified DNA, or linear amplified DNA; or
(d) the template strand is in a single stranded, double stranded, or circular form; or
(e) in the step of providing a template strand, the template strand is polyadenylated messenger RNA; or
(f) the template strand is hybridized to at least one oligonucleotide primer sequence, and for example, in the oligonucleotide primer is a mixture of primer sequences; or
(g) a double stranded DNA template contains at least one nick having a 3'-terminal hydroxyl; or
(h) in the step of providing a template strand, the template strand contains a phage RNA polymerase promoter sequence; or
(i) the enzyme is selected from the group consisting of: a DNA dependent DNA polymerase, an RNA dependent DNA polymerase, or an RNA dependent reverse transcriptase; or
(j) the step of providing nucleotides comprises providing a mixture of nucleotides designated A, A', G, G', T, T', C and C', wherein A' does not form a stable base pair with T', and G' does not form a stable base pair with C', wherein said first nucleic acid can form stable base pairs with said second nucleic acid comprising the nucleotides A, A*, G, G*, T, T*, C, and C*, wherein A* can form a stable base pair with T' and G* can form a stable base pair with C' and T* can form a stable base pair with A' and C* can form a stable base pair with G'; or
(k) the step of providing nucleotides comprises providing at least one nucleotide selected from the group consisting of: 2-amino-2'-deoxyadenosine-5'-triphosphate, 2-thio-2'-deoxythymidine-5'-triphosphate, 2'-deoxyinosine-5'-triphosphate, 2'-deoxypyrrolopyrimidine-5'-triphosphate, 2-thio-2'-deoxycytidine-5'-triphosphate, 2'-deoxyguanosine-5'-triphosphate, 2'-deoxycytidine-5'-triphosphate, 2'-deoxyadenosine-5'-triphosphate, 2'-deoxythymidine-5'-triphosphate, and modifications thereof; or
(l) the step of providing nucleotides comprises providing at least one nucleotide selected from the group consisting of: 2-aminoadenosine-5'-triphosphate, 2-thiouridine-5'-triphosphate, inosine-5'-triphosphate, pyrrolopyrimidine-5'-triphosphate, 2-thiocytidine-5'-triphosphate, guanosine-5'-triphosphate, cytidine-5'-triphosphate, adenosine-5'-triphosphate, uridine-5'-triphosphate, and modifications thereof, and for example, wherein the enzyme comprises a promoter dependent RNA polymerase; or
(m) at least one nucleotide triphosphate is labeled, and for example, the label is selected from a group comprising fluorescent dyes, radioactive isotopes, and biotin; or
(n) in the step of providing nucleotide triphosphates, the nucleotide triphosphates contain A' and T' wherein A' and T' have a reduced ability to form a stable base pair, wherein A' can form a stable base pair with T* and wherein T' can form a stable base pair with A*; or
(o) in the step of providing nucleotide triphosphates, the nucleotide triphosphates are selected from the group consisting of: 2-aminodeoxyadenosine 5'-triphosphate, 2-thiodeoxythymidine 5'-triphosphate, deoxyinosine 5'-triphosphate, deoxypyrrolopyrimidine 5'-triphosphate, 2-thiodeoxycytidine 5'-triphosphate, deoxyguanosine 5'-triphosphate, deoxycytidine 5'-triphosphate, deoxyadenosine 5'-triphosphate, deoxythymidine 5'-triphosphate, and combinations thereof.

6. The method of claim 4, wherein the step of providing nucleotides comprises providing a mixture of nucleotides designated A', G', T', and C', wherein A' does not form a stable base pair with T', and G' does not form a stable base pair with C', wherein said first nucleic acid can form stable base pairs with said second nucleic acid comprising the nucleotides A*, G*, T*, and C*, wherein A* can form a stable base pair with T' and G* can form a stable base pair with C' and T* can form a stable base pair with A' and C* can form a stable base pair with G'.

**7.** The method of claim 6, wherein

(a) A' is 2-aminoadenosine triphosphate, G' is inosine triphosphate, T' is 2-thiouridine triphosphate, and C' is cytidine triphosphate, A* is adenine, G* is guanosine, T* is thymidine or uridine, and C* is cytidine; or
(b) A' is 2-amino-2'-deoxyadenosine triphosphate, G' is 2'-deoxyinosine triphosphate, T' is 2-thio-2' deoxyuridine triphosphate, and C' is 2'deoxycytidine triphosphate, A* is 2'deoxyadenine, G* is 2'-deoxyguanosine, T* is 2'-deoxythymidine, and C* is 2'-deoxycytidine; or
(c) A' is 2-aminoadenosine triphosphate, G' is guanosine triphosphate, T' is 2-thiouridine triphosphate, C' is 2-thiocytidine triphosphate, A* is adenine, G* is inosine, T* is thymidine or uridine, and C* is cytidine; or
(d) A' is 2-amino-2'-deoxyadenosine triphosphate, G' is 2'-deoxyguanosine triphosphate, T' is 2-thio-2'-deoxyuridine triphosphate, C' is 2-thio-2'-deoxycytidine triphosphate, A* is 2'-deoxyadenine, G* is 2'-deoxyinosine, T* is 2'-deoxythymidine, and C* is 2'-deoxycytidine.

**8.** The method of claim 4, wherein the step of providing nucleotides comprises providing a mixture of nucleotides designated A', G, T', and C wherein, A' does not form a stable base pair with T', wherein said first nucleic acid can form stable base pairs with said second nucleic acid comprising the nucleotides A*, G, T*, C, wherein A* can form a stable base pair with T' and T* can form a stable base pair with A'.

**9.** The method of claim 8, wherein

(a) A' is 2-aminoadenosine triphosphate, T' is 2-thiouridine triphosphate, A* is adenine, and T* is thymidine; or
(b) A' is 2-amino2'deoxyadenosine triphosphate, T' is 2-thio-2'-deoxyuridine triphosphate, A* is 2'-deoxyadenine, and T* is 2'-dexoythymidine.

**10.** The method of claim 4, wherein the step of providing nucleotides comprises providing a mixture of nucleotides designated A, G', T, and C', wherein G does not form a stable base pair with C', wherein said first nucleic acid can form stable base pairs with said second nucleic acid comprising the nucleotides A, G*, T, C*, wherein G* can form a stable base pair with C' and C* can form a stable base pair with G'.

**11.** The method of claim 10, wherein

(a) G' is inosine triphosphate, C' is pyrrolo-pyrimidine triphosphate, G* is guanosine, and C* is cytidine; or
(b) G' is 2'-deoxyinosine triphosphate, C' is 2'deoxypyrrolo-pyrimidine triphosphate, G* is 2'deoxyguanosine, and C* is 2'-deoxycytidine; or
(c) G' is guanosine triphosphate, C' is 2-thiocytidine triphosphate, G* is inosine, and C* is cytidine; or
(d) G' is 2'-deoxyguanosine triphosphate, C' is 2-thio-2'-deoxycytidine triphosphate, G* is 2'-deoxyinosine, and C* is 2'-deoxycytidine.

**12.** Nucleic acid obtainable by the method of any one of claims 4 to 11.

**13.** A first unstructured nucleic acid (UNA) molecule, or a mixture of nucleic acid molecules including at least a first UNA, having reduced levels of intramolecular cross hybridization and/or intermolecular hybridization, wherein the first UNA comprises at least a first sequence element that is substantially complementary to a second sequence element in the first and/or a second UNA, said first sequence element being unable to form stable base pairs with the second sequence element but being capable of forming stable base-pairs with a substantially complementary sequence element present in a target nucleic acid molecule.

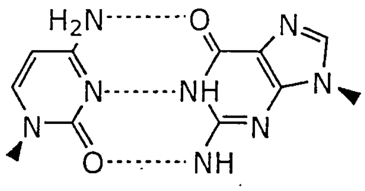

C - G

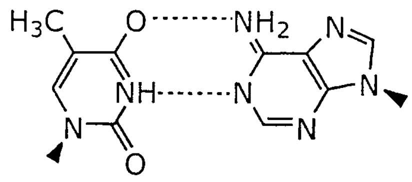

T (or U) - A

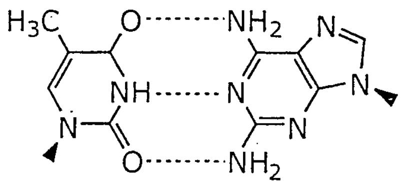

T (or U) - 2-amino A

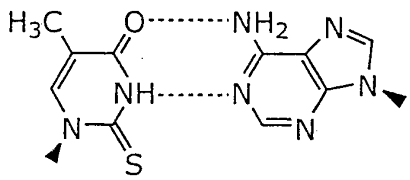

2-thio T (or U) - A

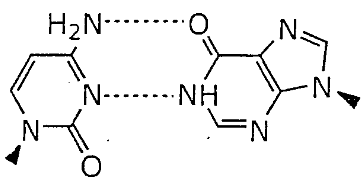

C - I

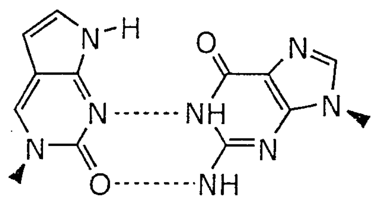

PyrroloPyr - G

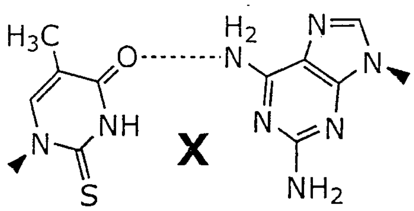

2-thio T (or U) - 2-amino A

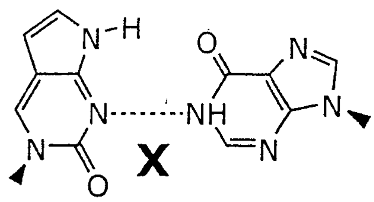

PyrroloPyr - I

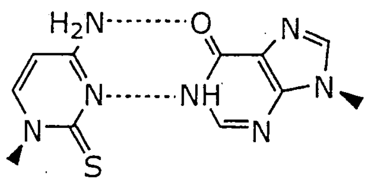

2-thio C - I

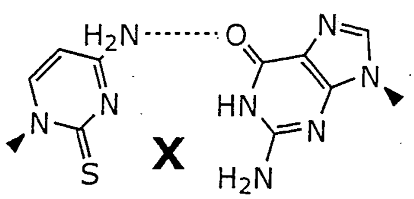

2-thio C - G

FIG. 1

5'-CGATAGGCTCTG →
3'-GCTATCCGAGACCCTGACTTGACACCTGTT-5'

FIG. 2A

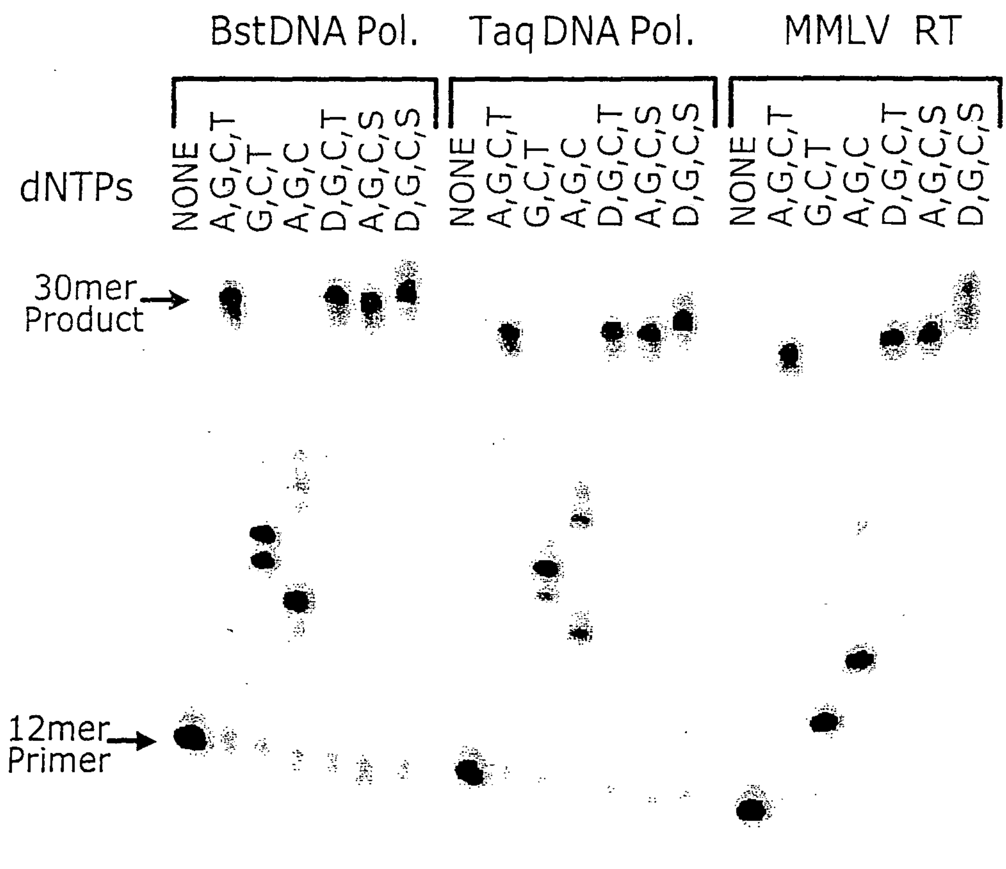


FIG. 2B

5'-GACTGA→
3'-GCTATCCGAGACACTGACTTGACACCTGTT-5'

FIG. 3A

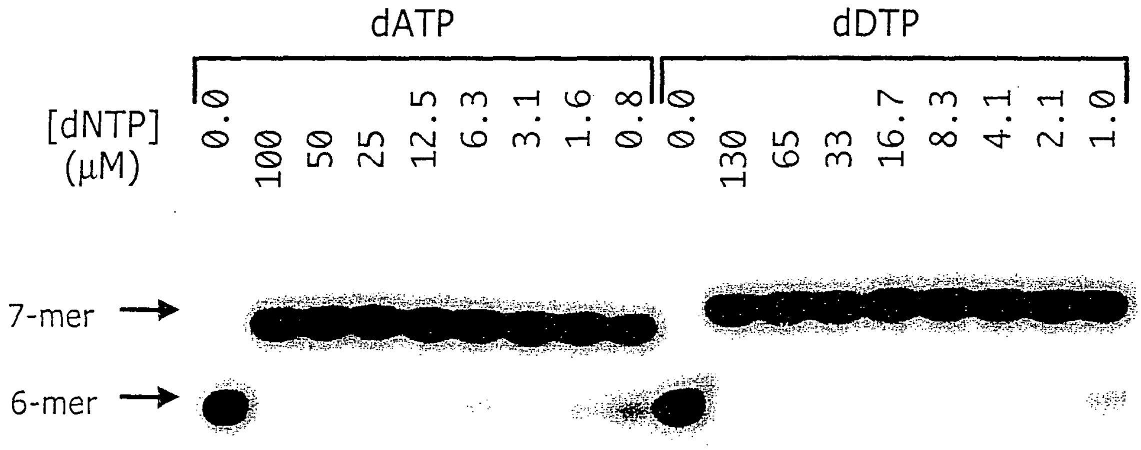


FIG. 3B

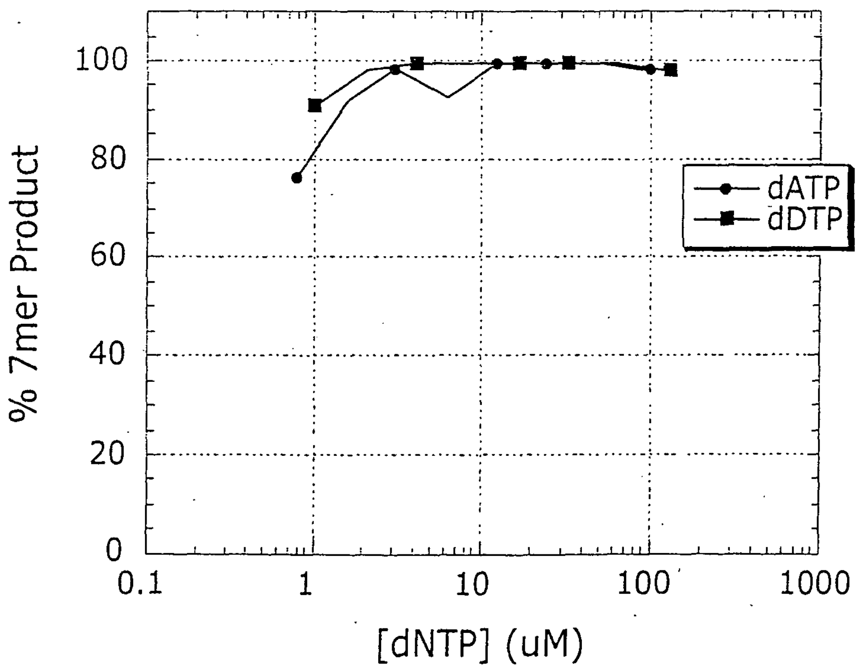


FIG. 3C

5'-GCTCTG →
3'-GCTATCCGAGACACTGACTTGACACCTGTT-5'

FIG. 4A

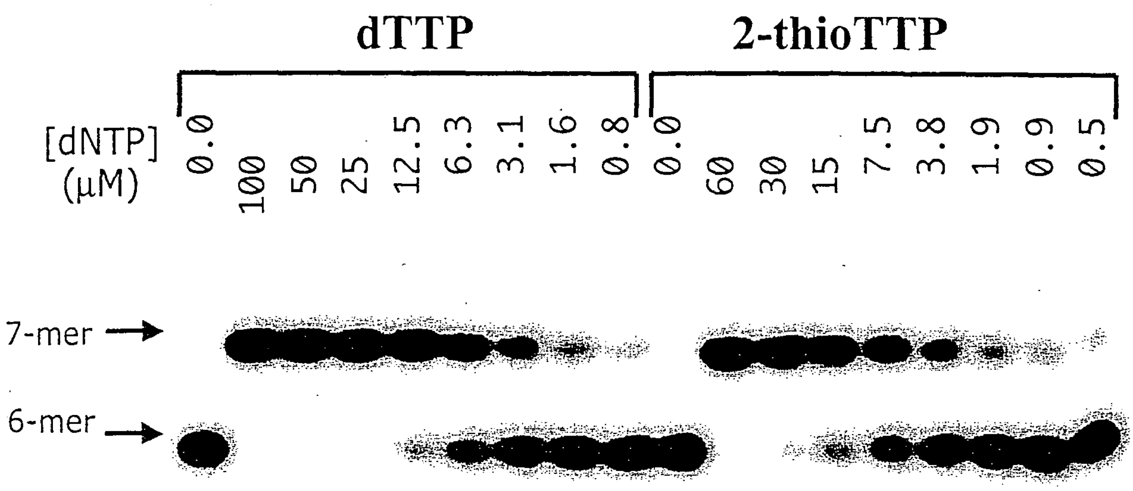


FIG. 4B

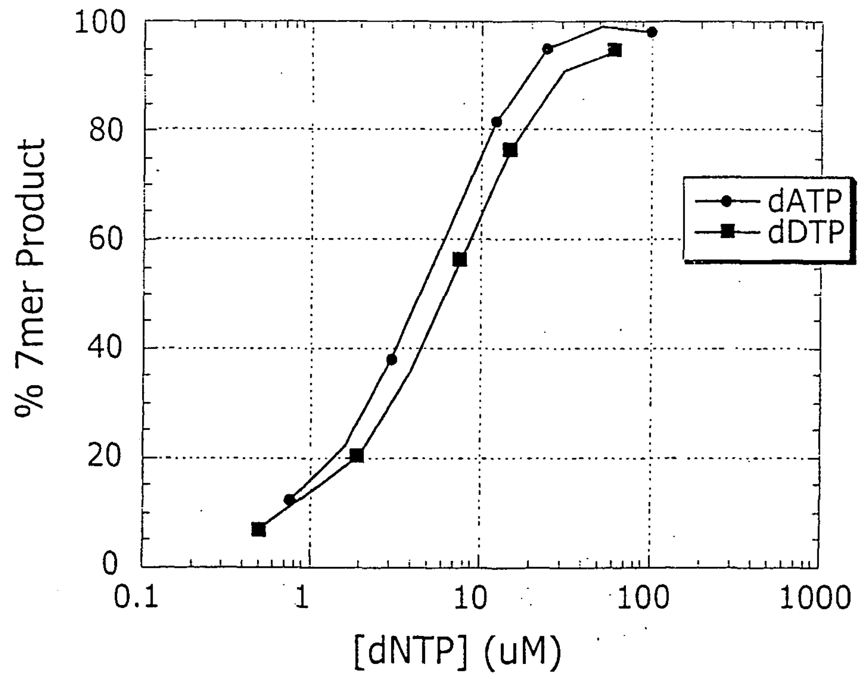


FIG. 4C

5'-GACTGA →
3'-GCTATCCGAGACACTGACTTGACACCTGTT-5'

5'-GCTCTG →
3'-GCTATCCGAGACACTGACTTGACACCTGTT-5'

FIG. 5A

| Extension Nucleotide | X-mer | Predicted m/z (Positive Ion)* | Measured m/z (Positive Ion) | Predicted △ m/z | Measured △ m/z |
|---|---|---|---|---|---|
| | | | | | |
| None | GACTGA | 1816.3 | nd | -- | -- |
| dATP | GACTGA**A** | 2129.5 | 2130.2 | | |
| dDTP | GACTGA**D** | 2144.5 | 2146.0 | **+15.0** | **+15.8** |
| | | | | | |
| None | GCTCTG | 1783.2 | 1785.4 ± 0.2 | -- | -- |
| dTTP | GCTCTG**T** | 2087.4 | 2089.7 | | |
| d-2-thio-TTP | GCTCTG**S** | 2103.4 | 2106.1 | **+16.0** | **+16.4** |

FIG. 5C

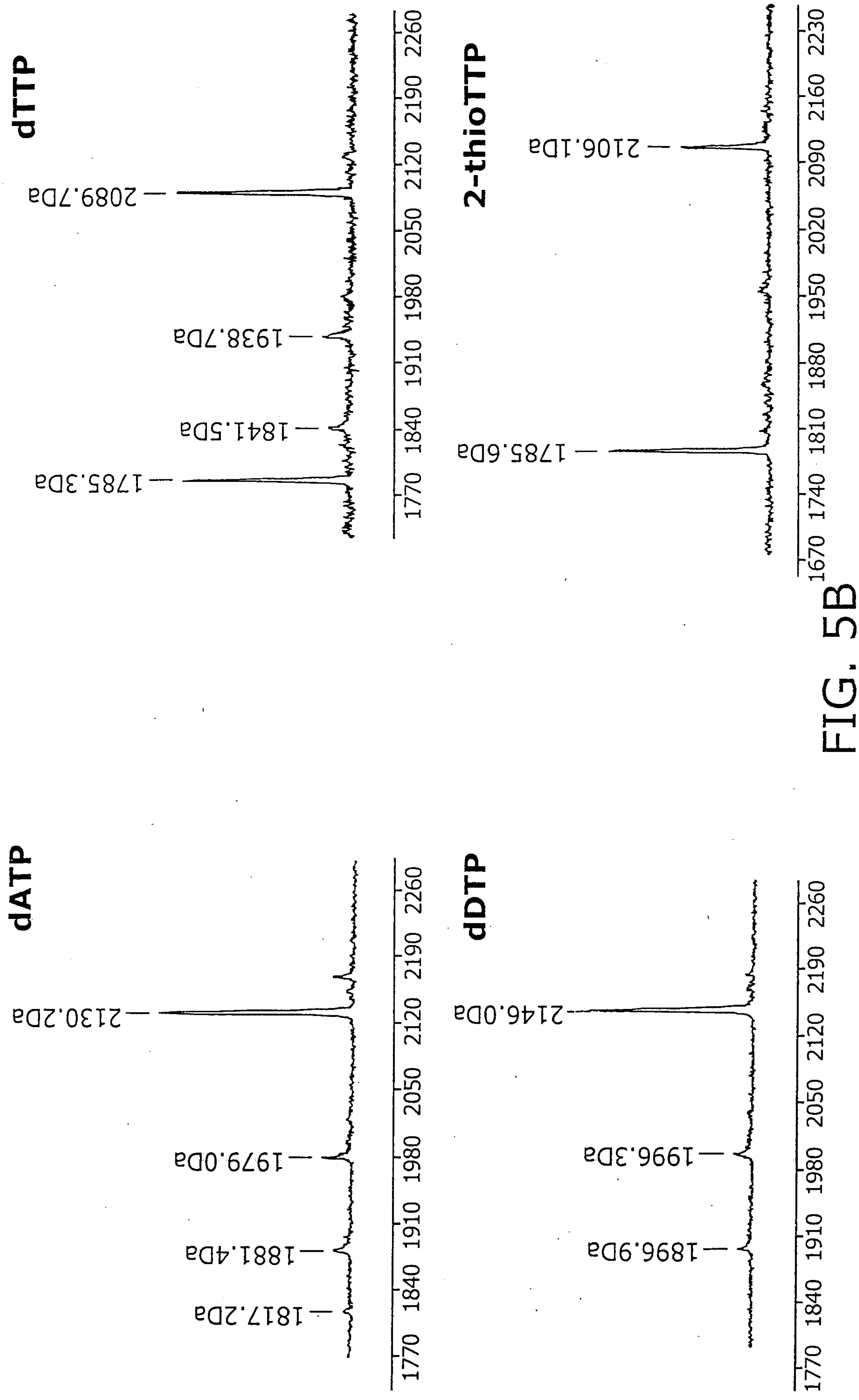
dTTP
1785.3Da
1841.5Da
1938.7Da
2089.7Da
1770 1840 1910 1980 2050 2120 2190 2260
2-thioTTP
1785.6Da
2106.1Da
1670 1740 1810 1880 1950 2020 2090 2160 2230
dATP
1817.2Da
1881.4Da
1979.0Da
2130.2Da
1770 1840 1910 1980 2050 2120 2190 2260
dDTP
1896.9Da
1996.3Da
2146.0Da
1770 1840 1910 1980 2050 2120 2190 2260

FIG. 5B

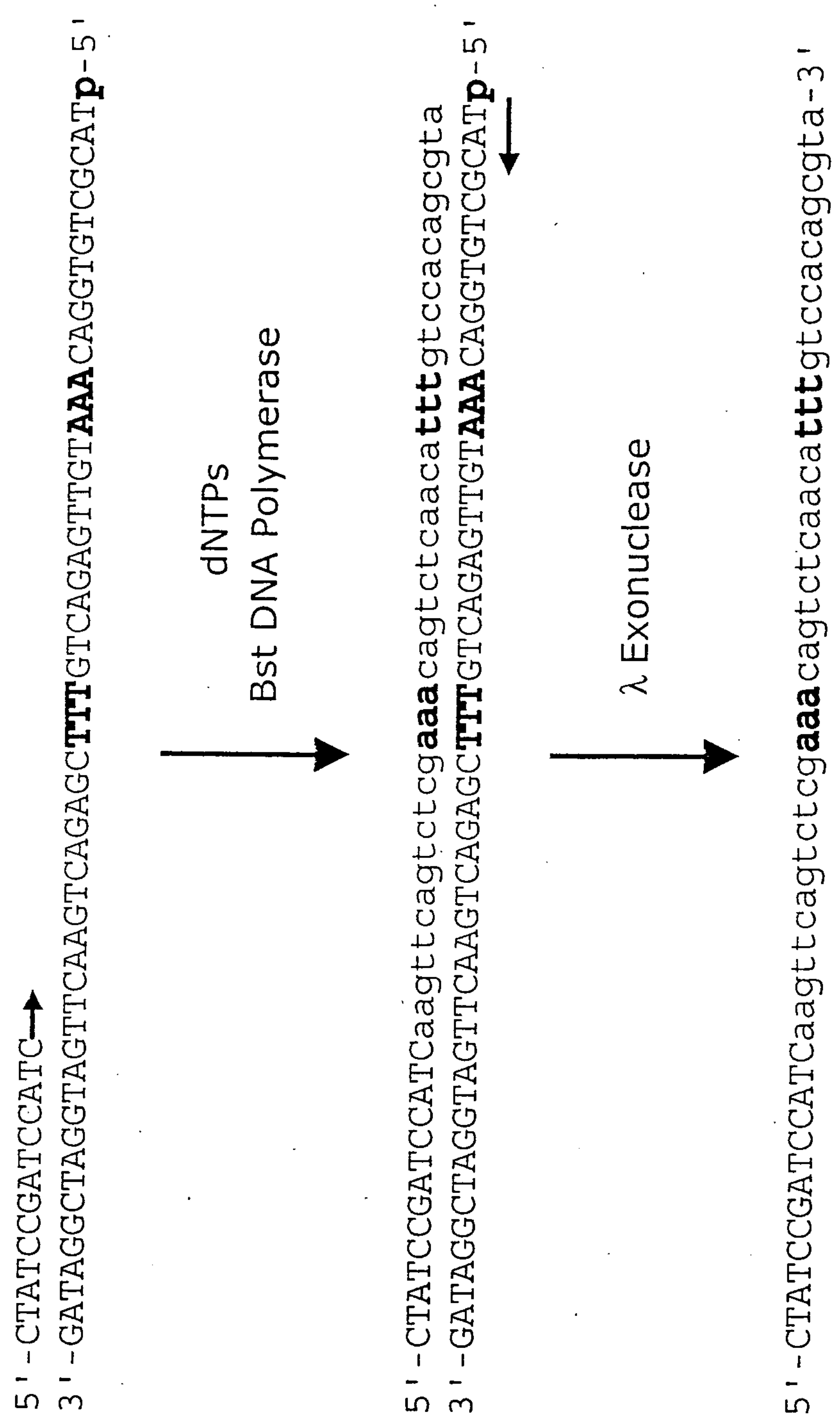
5'-CTATCCGATCCATC
3'-GATAGGCTAGGTAGTTCAAGTCAGAGCTTTGTCAGAGTTGTAAACAGGTGTCGCATp-5'
dNTPs
Bst DNA Polymerase
5'-CTATCCGATCCATCaagttcagtctcgaaacagtctcaacatttgtccacagcgta
3'-GATAGGCTAGGTAGTTCAAGTCAGAGCTTTGTCAGAGTTGTAAACAGGTGTCGCATp-5'
λ Exonuclease
5'-CTATCCGATCCATCaagttcagtctcgaaacagtctcaacatttgtccacagcgta-3'

FIG. 6

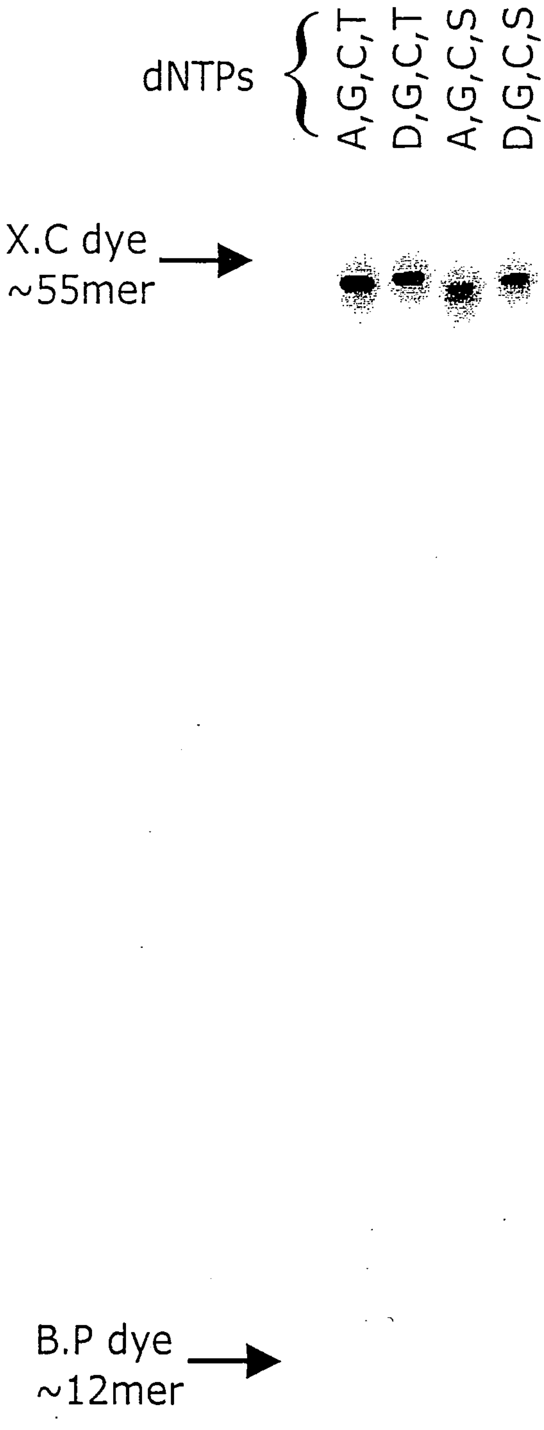


FIG. 7

**HP21AT**

$\Delta G^\circ$ = -12.3 kcal/mole at 37 °C
$\Delta H^\circ$ = -82.5 kcal/mole
$\Delta S^\circ$ = -226.3 cal/ (°K·mol)
Tm = 91.4°C

```
           10          20
5'-CTATCCGATCCATCAA           G
                   GTTCAGTCTC A
                   ||||||||||
                   CAAGTCAGAG A
3'-ATGCGACACCTGTTTA           A
        50         40         30
```

**HP21DS**

```
           10          20
5'-CTATCCGATCCATCDD           G
                   GSSCDGSCSC D
                   |..|.|.|.|
                   CDDGSCDGDG D
3'-DSGCGDCDCCSGSSSD           D
        50         40         30
```

**HP26AT**

$\Delta G^\circ$ = -3.8 kcal/mole at 37 °C
$\Delta H^\circ$ = -41.2 kcal/mole
$\Delta S^\circ$ = -120.5 cal/ (°K·mol)
Tm = 68.8°C

```
           10          20
5'-CTATCCGATCCATCAA    C  T   G
                   GT T AG CTC A
                   ||| || |||
                   CAA TC GAG A
3'-ATGCGACACCTGTTTA    C  T   A
        50         40         30
```

**HP26DS**

```
           10          20
5'-CTATCCGATCCATCDD    C  S   G
                   GSS DG CSC D
                   |.. .| |.|
                   CDD SC GDG D
3'-DSGCGDCDCCSGSSSD    C  S   D
        50         40         30
```

**HP28AT**

$\Delta G^\circ$ = 0.1 kcal/mole at 37°C
$\Delta H^\circ$ = -27.4 kcal/mole
$\Delta S^\circ$ = -88.6 cal/ (°K·mol)
Tm = 36.1°C

```
           10          20
5'-CTATCCGATCCATCAA    C  T  CG
                   GTT AG CT  A
                   ||| || ||
                   CAA TC GA  A
3'-ATGCGACACCTGTTTA    C  T  CA
        50         40        30
```

**HP28DS**

```
           10          20
5'-CTATCCGATCCATCDD    C  S  CG
                   GSS DG CS  D
                   |.. .| |.
                   CDD SC GD  D
3'-DSGCGDCDCCSGSSSD    C  S  CD
        50         40        30
```

FIG. 8

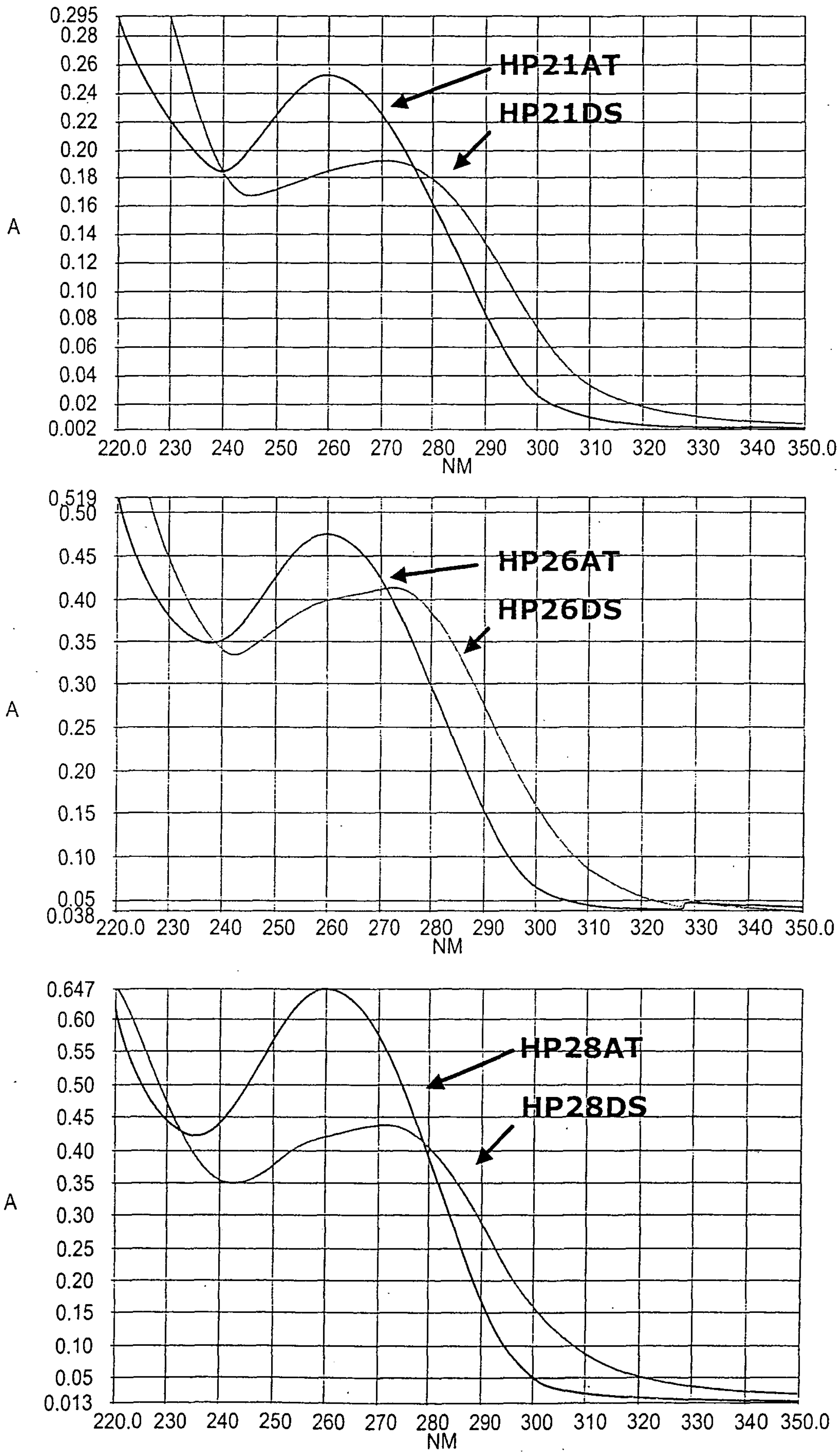
HP21AT
HP21DS
A
0.295
0.28
0.26
0.24
0.22
0.20
0.18
0.16
0.14
0.12
0.10
0.08
0.06
0.04
0.02
0.002
220.0 230 240 250 260 270 280 290 300 310 320 330 340 350.0
NM
HP26AT
HP26DS
A
0.519
0.50
0.45
0.40
0.35
0.30
0.25
0.20
0.15
0.10
0.05
0.038
220.0 230 240 250 260 270 280 290 300 310 320 330 340 350.0
NM
HP28AT
HP28DS
A
0.647
0.60
0.55
0.50
0.45
0.40
0.35
0.30
0.25
0.20
0.15
0.10
0.05
0.013
220.0 230 240 250 260 270 280 290 300 310 320 330 340 350.0
NM

FIG 9

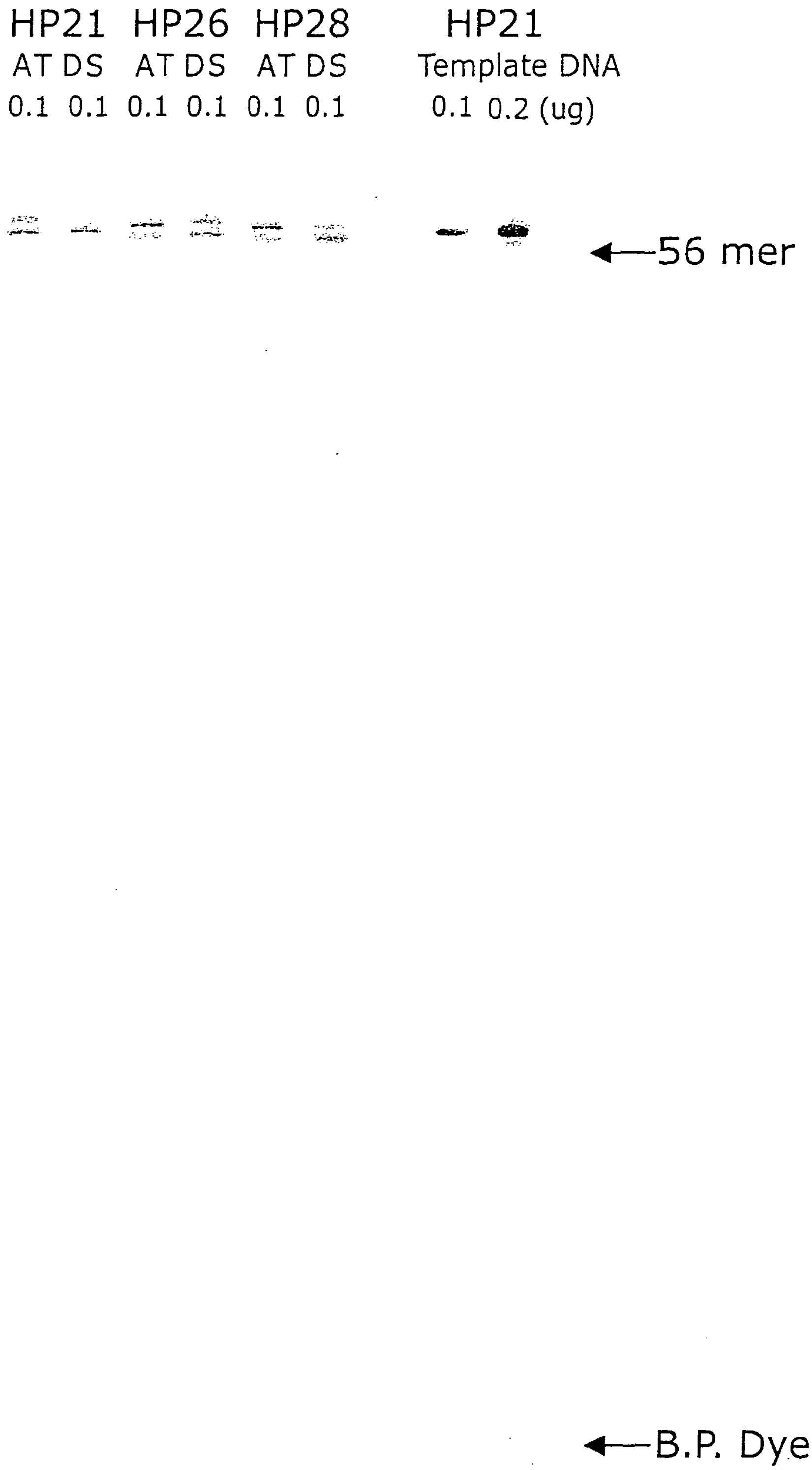


10% 7M Urea PAGE

FIG. 10

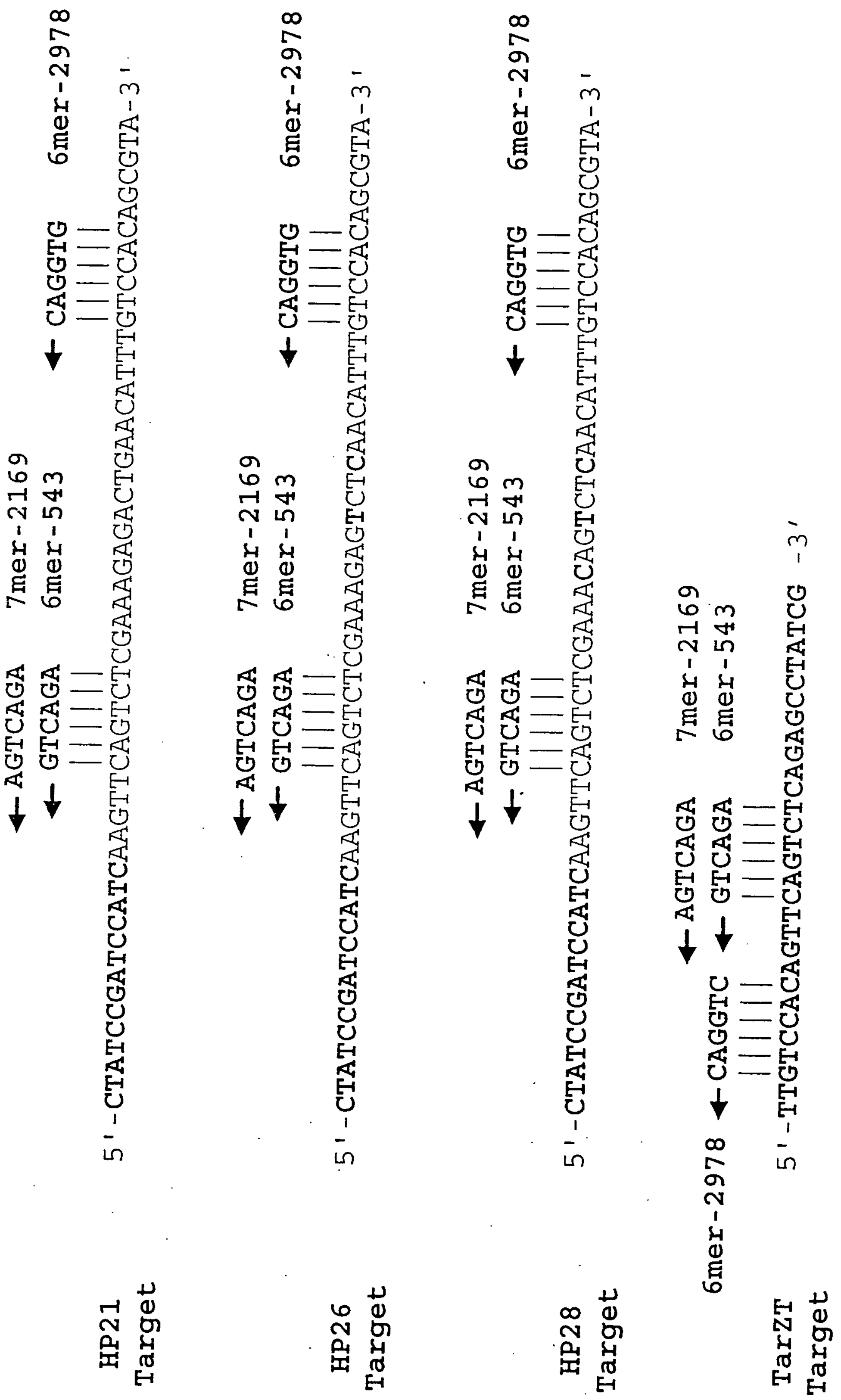
HP21 Target
5'-CTATCCGATCCATCAAGTTCAGTCTCGAAAGAGACTGAACATTTGTCCACAGCGTA-3'
AGTCAGA 7mer-2169
GTCAGA 6mer-543
CAGGTG 6mer-2978
HP26 Target
5'-CTATCCGATCCATCAAGTTCAGTCTCGAAAGAGTCTCAACATTTGTCCACAGCGTA-3'
AGTCAGA 7mer-2169
GTCAGA 6mer-543
CAGGTG 6mer-2978
HP28 Target
5'-CTATCCGATCCATCAAGTTCAGTCTCGAAACAGTCTCAACATTTGTCCACAGCGTA-3'
AGTCAGA 7mer-2169
GTCAGA 6mer-543
CAGGTG 6mer-2978
TarZT Target
5'-TTGTCCACAGTTCAGTCTCAGAGCCTATCG -3'
6mer-2978 CAGGTC
AGTCAGA 7mer-2169
GTCAGA 6mer-543

FIG. 11

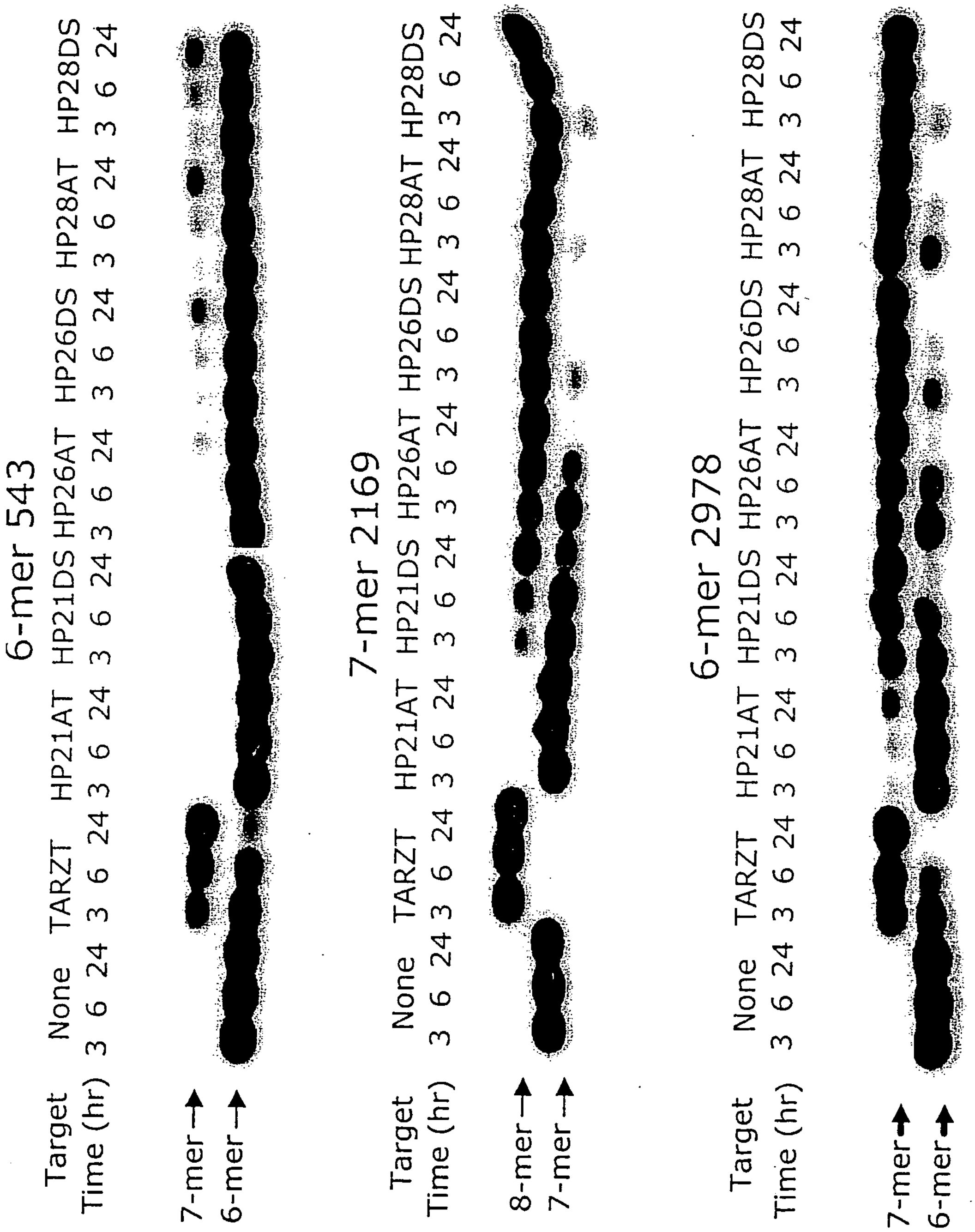
6-mer 543
Target None TARZT HP21AT HP21DS HP26AT HP26DS HP28AT HP28DS
Time (hr) 3 6 24 3 6 24 3 6 24 3 6 24 3 6 24 3 6 24 3 6 24 3 6 24
7-mer →
6-mer →
7-mer 2169
Target None TARZT HP21AT HP21DS HP26AT HP26DS HP28AT HP28DS
Time (hr) 3 6 24 3 6 24 3 6 24 3 6 24 3 6 24 3 6 24 3 6 24 3 6 24
8-mer →
7-mer →
6-mer 2978
Target None TARZT HP21AT HP21DS HP26AT HP26DS HP28AT HP28DS
Time (hr) 3 6 24 3 6 24 3 6 24 3 6 24 3 6 24 3 6 24 3 6 24 3 6 24
7-mer →
6-mer →

FIG. 12

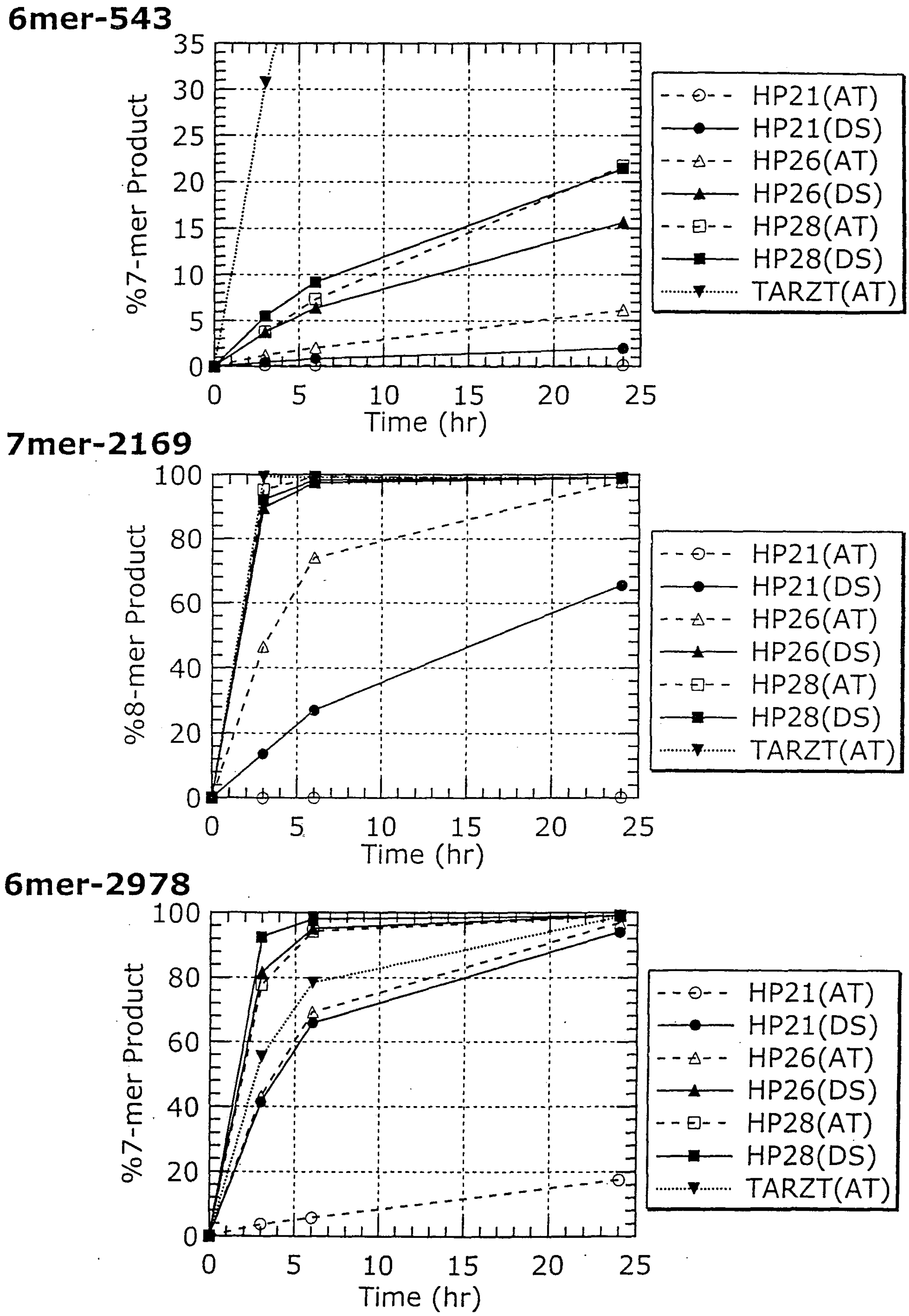
6mer-543
%7-mer Product
Time (hr)
HP21(AT)
HP21(DS)
HP26(AT)
HP26(DS)
HP28(AT)
HP28(DS)
TARZT(AT)
7mer-2169
%8-mer Product
Time (hr)
HP21(AT)
HP21(DS)
HP26(AT)
HP26(DS)
HP28(AT)
HP28(DS)
TARZT(AT)
6mer-2978
%7-mer Product
Time (hr)
HP21(AT)
HP21(DS)
HP26(AT)
HP26(DS)
HP28(AT)
HP28(DS)
TARZT(AT)

FIG. 13

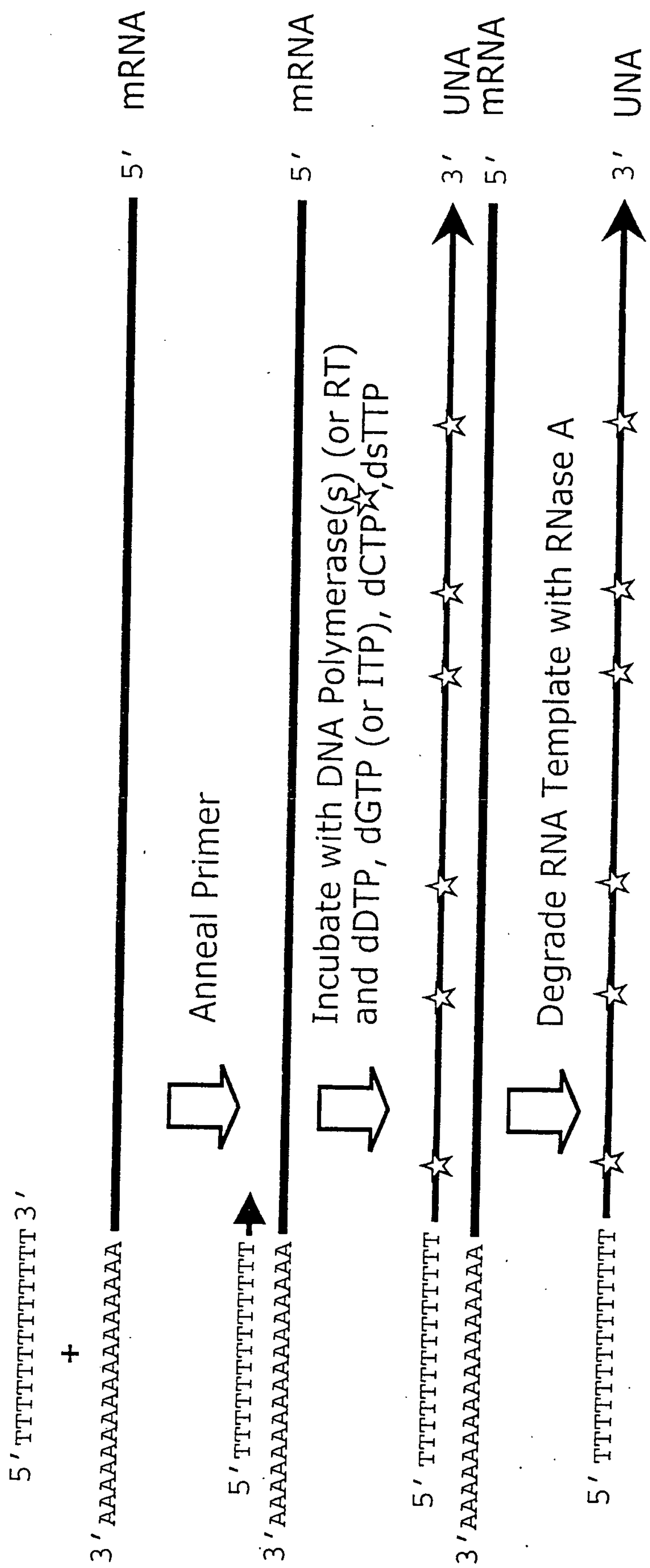
5′ TTTTTTTTTTTT 3′
+
3′ AAAAAAAAAAAAA
5′ mRNA
Anneal Primer
5′ TTTTTTTTTTTT
3′ AAAAAAAAAAAAA
5′ mRNA
Incubate with DNA Polymerase(s) (or RT)
and dDTP, dGTP (or ITP), dCTP☆, dsTTP
5′ TTTTTTTTTTTT
3′ UNA
3′ AAAAAAAAAAAAA
5′ mRNA
Degrade RNA Template with RNase A
5′ TTTTTTTTTTT
3′ UNA

FIG. 14

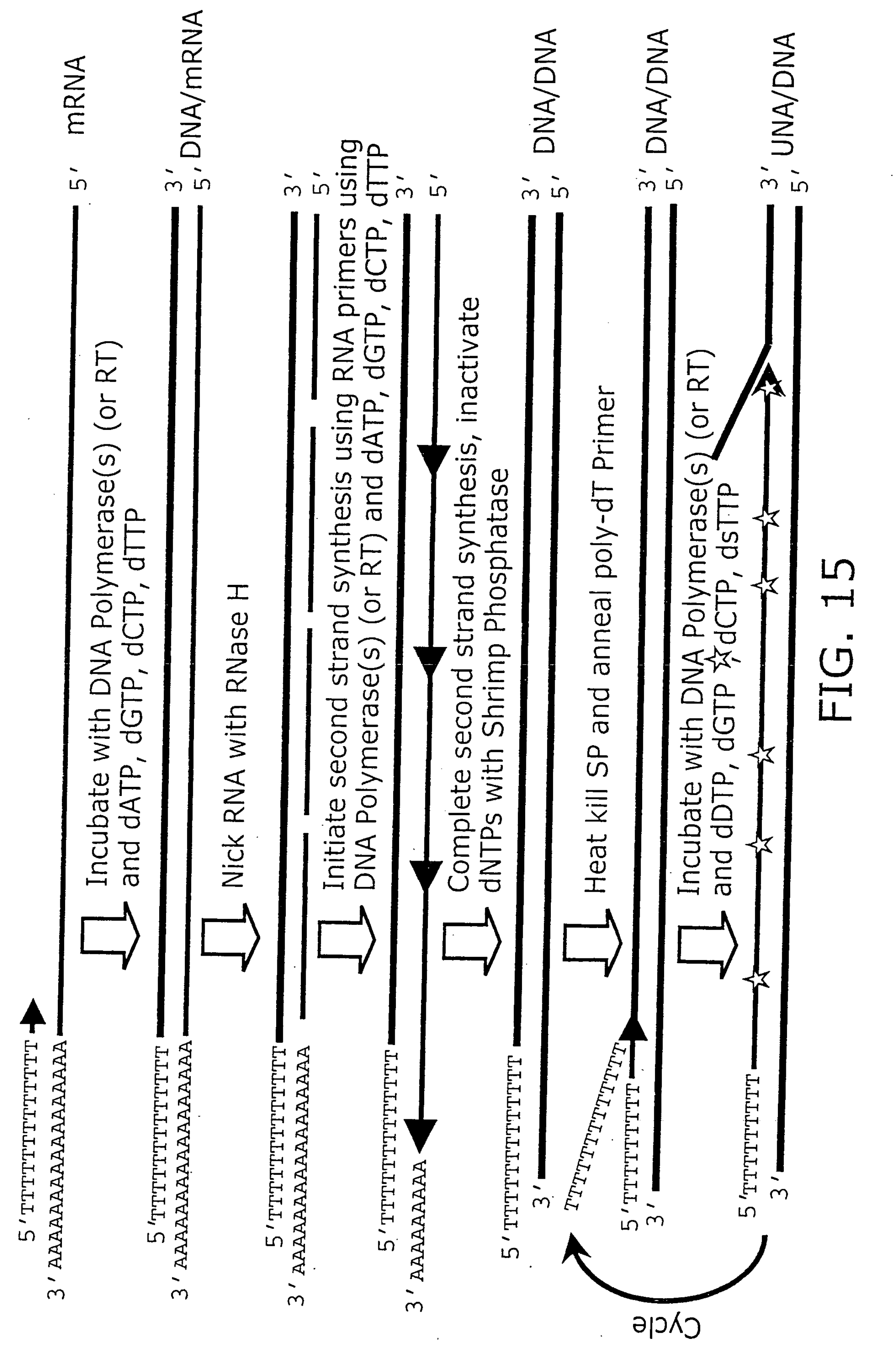
mRNA
DNA/mRNA
DNA/DNA
DNA/DNA
UNA/DNA
5'TTTTTTTTTTTT
3'AAAAAAAAAAAAAA
Incubate with DNA Polymerase(s) (or RT) and dATP, dGTP, dCTP, dTTP
Nick RNA with RNase H
Initiate second strand synthesis using RNA primers using DNA Polymerase(s) (or RT) and dATP, dGTP, dCTP, dTTP
Complete second strand synthesis, inactivate dNTPs with Shrimp Phosphatase
Heat kill SP and anneal poly-dT Primer
Incubate with DNA Polymerase(s) (or RT) and dDTP, dGTP ☆dCTP, dsTTP
Cycle

FIG. 15

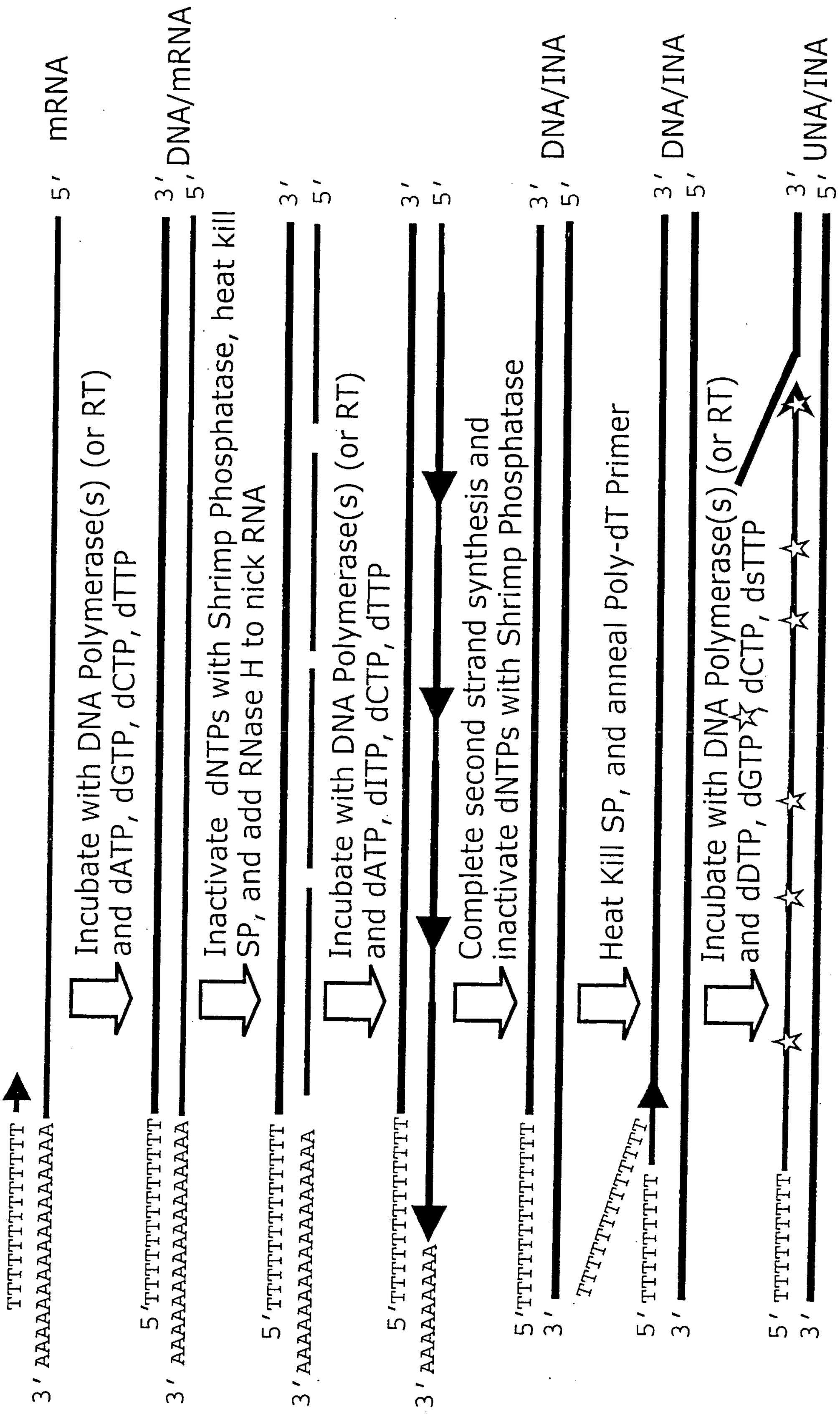
TTTTTTTTTTT
3' AAAAAAAAAAAAAA
5' mRNA
Incubate with DNA Polymerase(s) (or RT) and dATP, dGTP, dCTP, dTTP
5' TTTTTTTTTTTTT
3' AAAAAAAAAAAAAA
3' 5' DNA/mRNA
Inactivate dNTPs with Shrimp Phosphatase, heat kill SP, and add RNase H to nick RNA
5' TTTTTTTTTTTTT
3' AAAAAAAAAAAAAA
3' 5'
Incubate with DNA Polymerase(s) (or RT) and dATP, dITP, dCTP, dTTP
5' TTTTTTTTTTTTT
3' AAAAAAAA
3' 5'
Complete second strand synthesis and inactivate dNTPs with Shrimp Phosphatase
5' TTTTTTTTTTTTT
3'
3' 5' DNA/INA
Heat Kill SP, and anneal Poly-dT Primer
TTTTTTTTTT
5' TTTTTTTT
3'
3' 5' DNA/INA
Incubate with DNA Polymerase(s) (or RT) and dDTP, dGTP, dCTP, dsTTP
5' TTTTTTTT
3'
3' 5' UNA/INA

FIG. 16

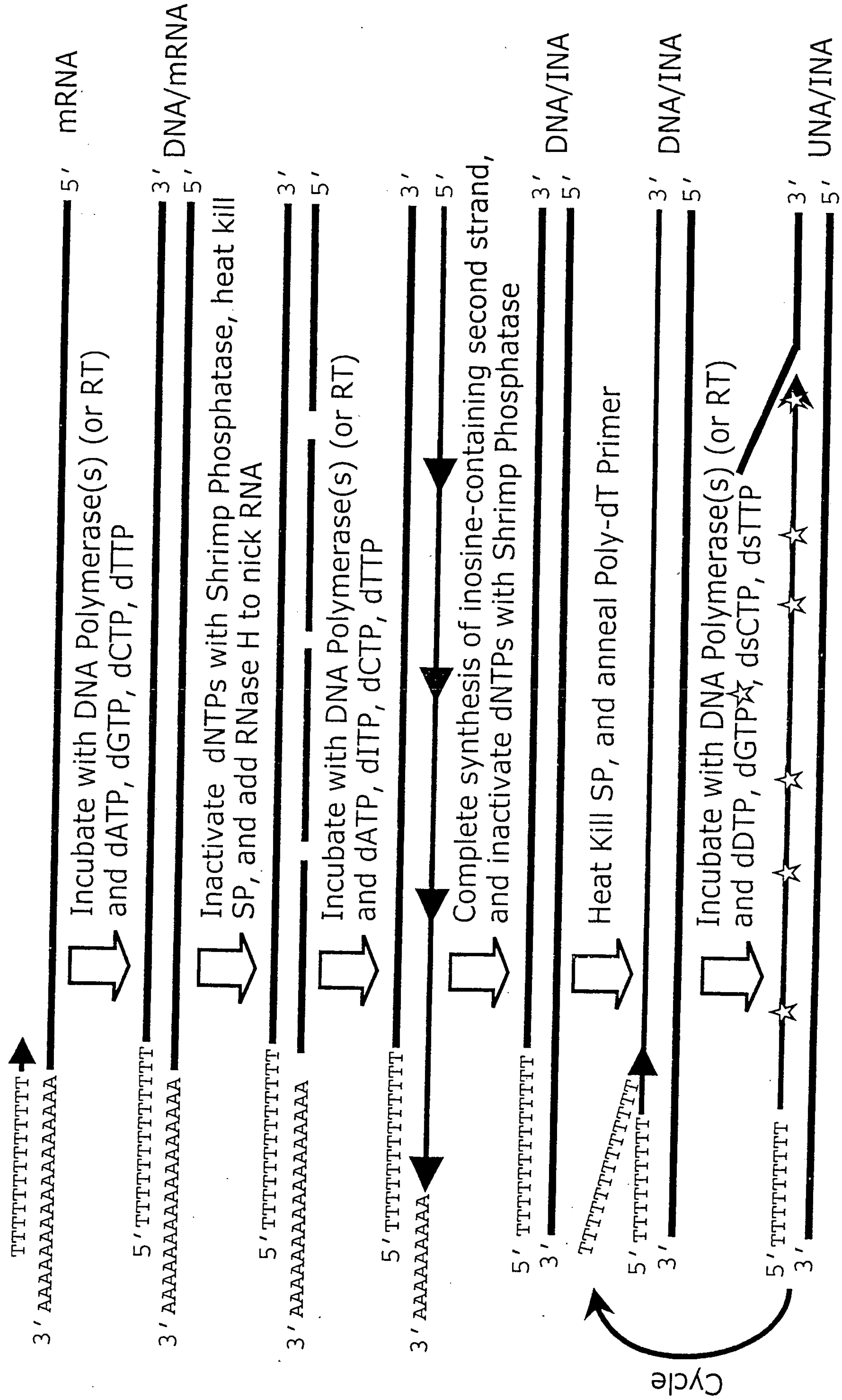
mRNA
3′ AAAAAAAAAAAAAA
TTTTTTTTTTTT
5′
Incubate with DNA Polymerase(s) (or RT) and dATP, dGTP, dCTP, dTTP
DNA/mRNA
5′ TTTTTTTTTTTT
3′ AAAAAAAAAAAAAA
Inactivate dNTPs with Shrimp Phosphatase, heat kill SP, and add RNase H to nick RNA
Incubate with DNA Polymerase(s) (or RT) and dATP, dITP, dCTP, dTTP
Complete synthesis of inosine-containing second strand, and inactivate dNTPs with Shrimp Phosphatase
DNA/INA
Heat Kill SP, and anneal Poly-dT Primer
Incubate with DNA Polymerase(s) (or RT) and dDTP, dGTP☆, dsCTP, dsTTP
UNA/INA
Cycle

FIG. 17

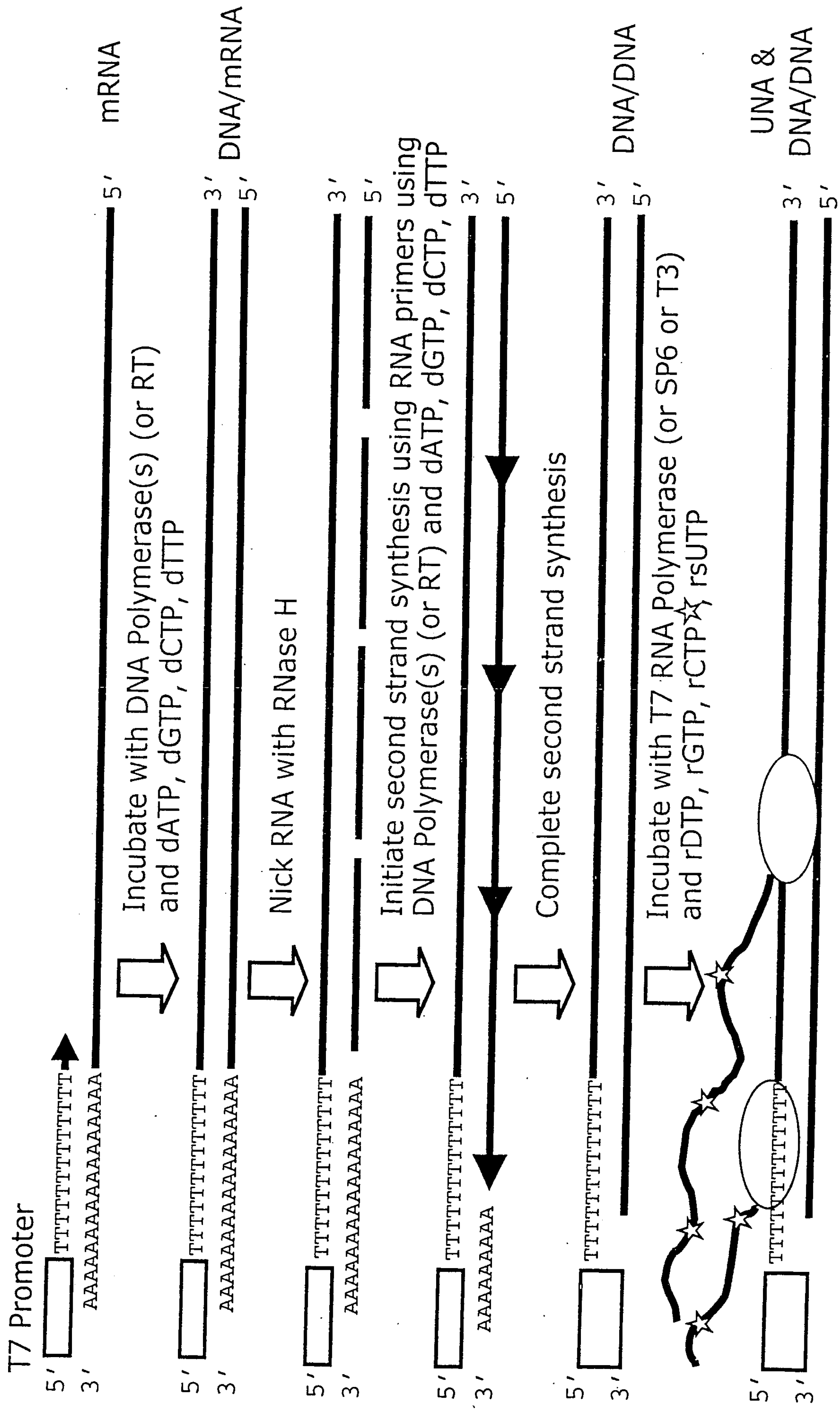
T7 Promoter
5′
3′
TTTTTTTTTTTTT
AAAAAAAAAAAAAA
5′
mRNA
Incubate with DNA Polymerase(s) (or RT)
and dATP, dGTP, dCTP, dTTP
3′
5′
DNA/mRNA
Nick RNA with RNase H
Initiate second strand synthesis using RNA primers using
DNA Polymerase(s) (or RT) and dATP, dGTP, dCTP, dTTP
AAAAAAAA
Complete second strand synthesis
DNA/DNA
Incubate with T7 RNA Polymerase (or SP6 or T3)
and rDTP, rGTP, rCTP☆, rsUTP
UNA &
DNA/DNA

FIG. 18

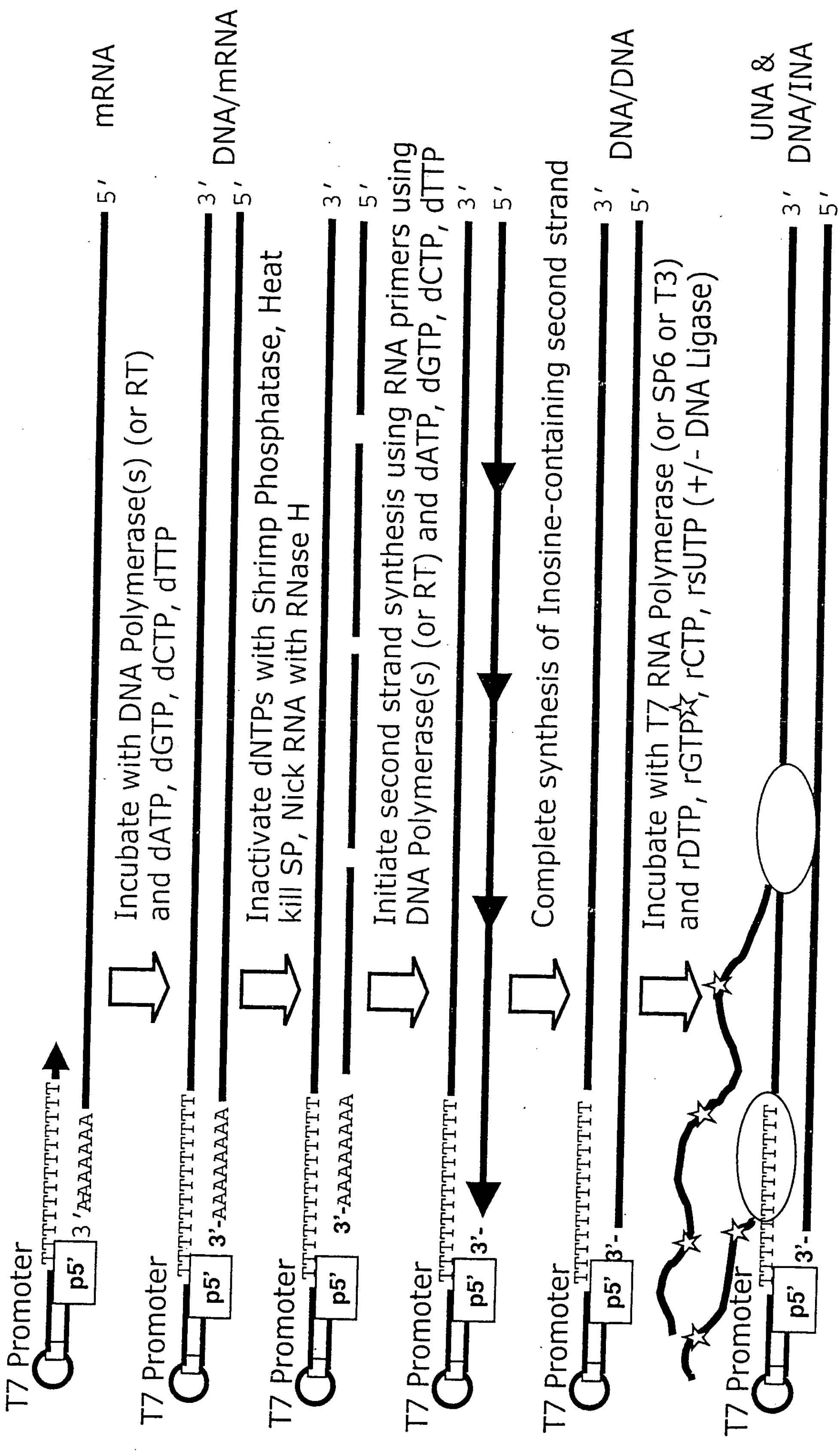
T7 Promoter
p5'
TTTTTTTTTTTT
3 'AAAAAAA
5'
mRNA
Incubate with DNA Polymerase(s) (or RT)
and dATP, dGTP, dCTP, dTTP
3'-AAAAAAA
3'
5'
DNA/mRNA
Inactivate dNTPs with Shrimp Phosphatase, Heat
kill SP, Nick RNA with RNase H
Initiate second strand synthesis using RNA primers using
DNA Polymerase(s) (or RT) and dATP, dGTP, dCTP, dTTP
3'-
Complete synthesis of Inosine-containing second strand
DNA/DNA
Incubate with T7 RNA Polymerase (or SP6 or T3)
and rDTP, rGTP☆, rCTP, rsUTP (+/- DNA Ligase)
UNA &
DNA/INA

FIG. 19

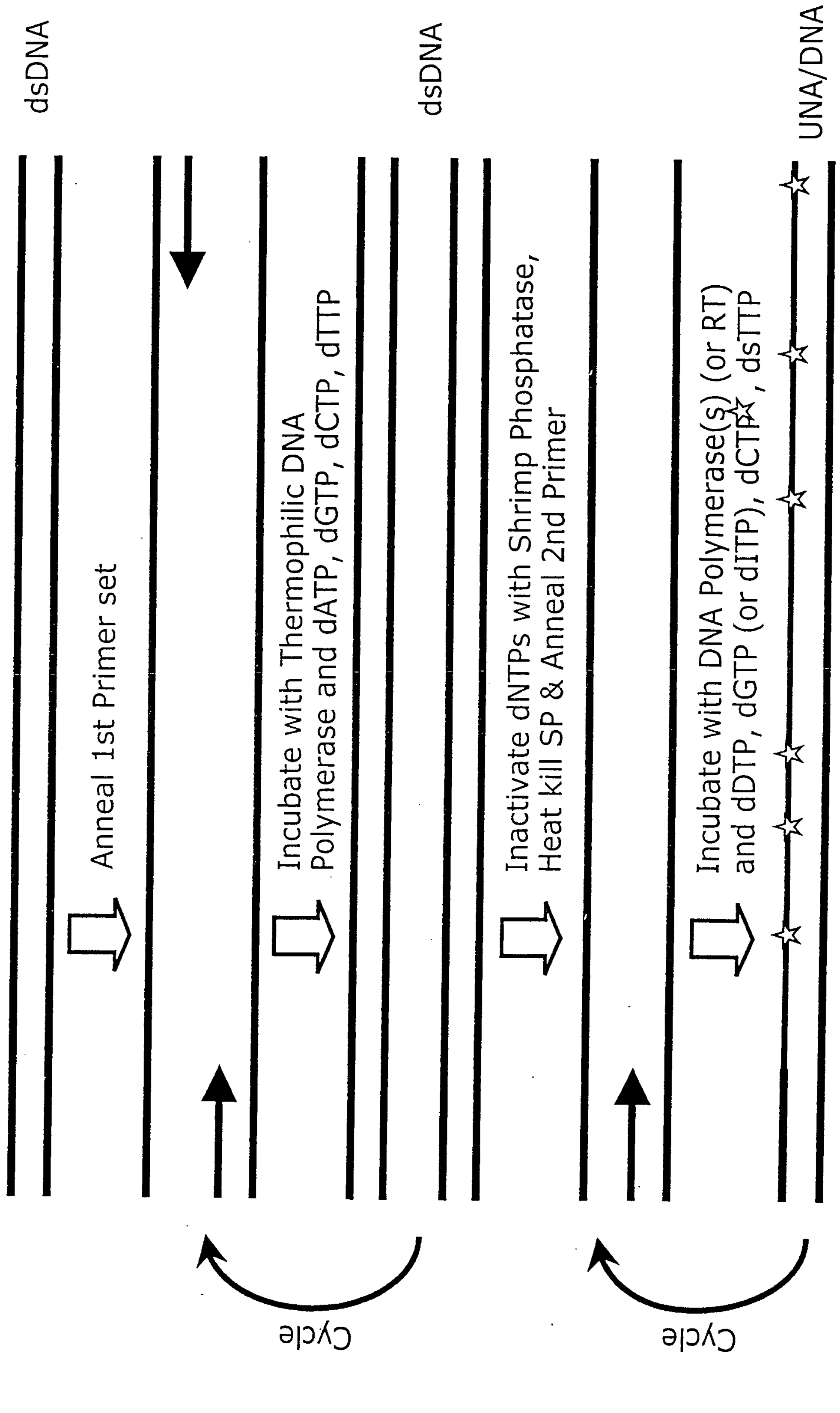
dsDNA
Anneal 1st Primer set
Incubate with Thermophilic DNA Polymerase and dATP, dGTP, dCTP, dTTP
dsDNA
Inactivate dNTPs with Shrimp Phosphatase, Heat kill SP & Anneal 2nd Primer
Incubate with DNA Polymerase(s) (or RT) and dDTP, dGTP (or dITP), dCTP☆, dsTTP
UNA/DNA
Cycle
Cycle

FIG. 20

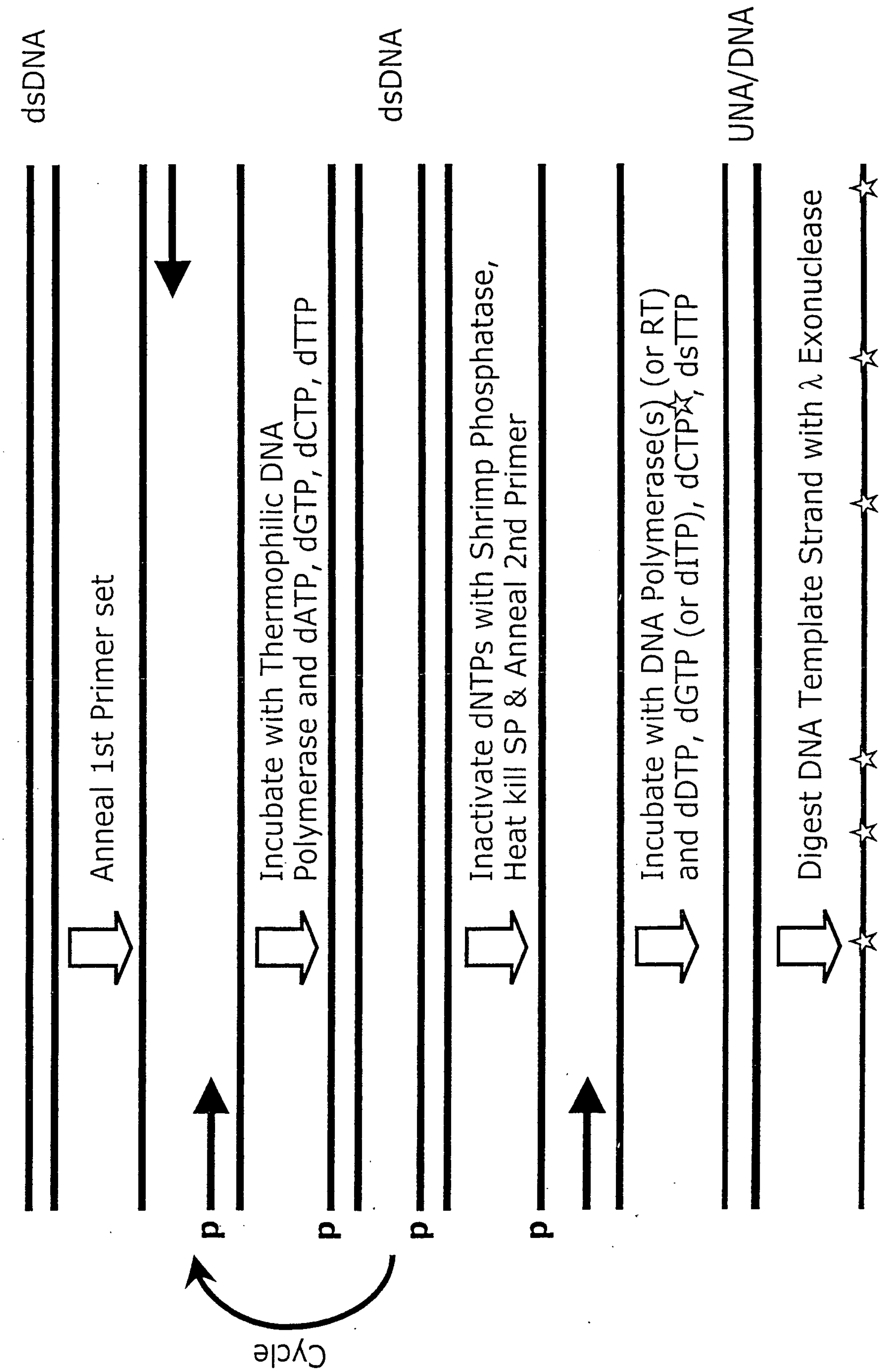
dsDNA
Anneal 1st Primer set
p
Incubate with Thermophilic DNA Polymerase and dATP, dGTP, dCTP, dTTP
p
dsDNA
p
Cycle
Inactivate dNTPs with Shrimp Phosphatase, Heat kill SP & Anneal 2nd Primer
p
Incubate with DNA Polymerase(s) (or RT) and dDTP, dGTP (or dITP), dCTP☆, dsTTP
UNA/DNA
Digest DNA Template Strand with λ Exonuclease

FIG. 21

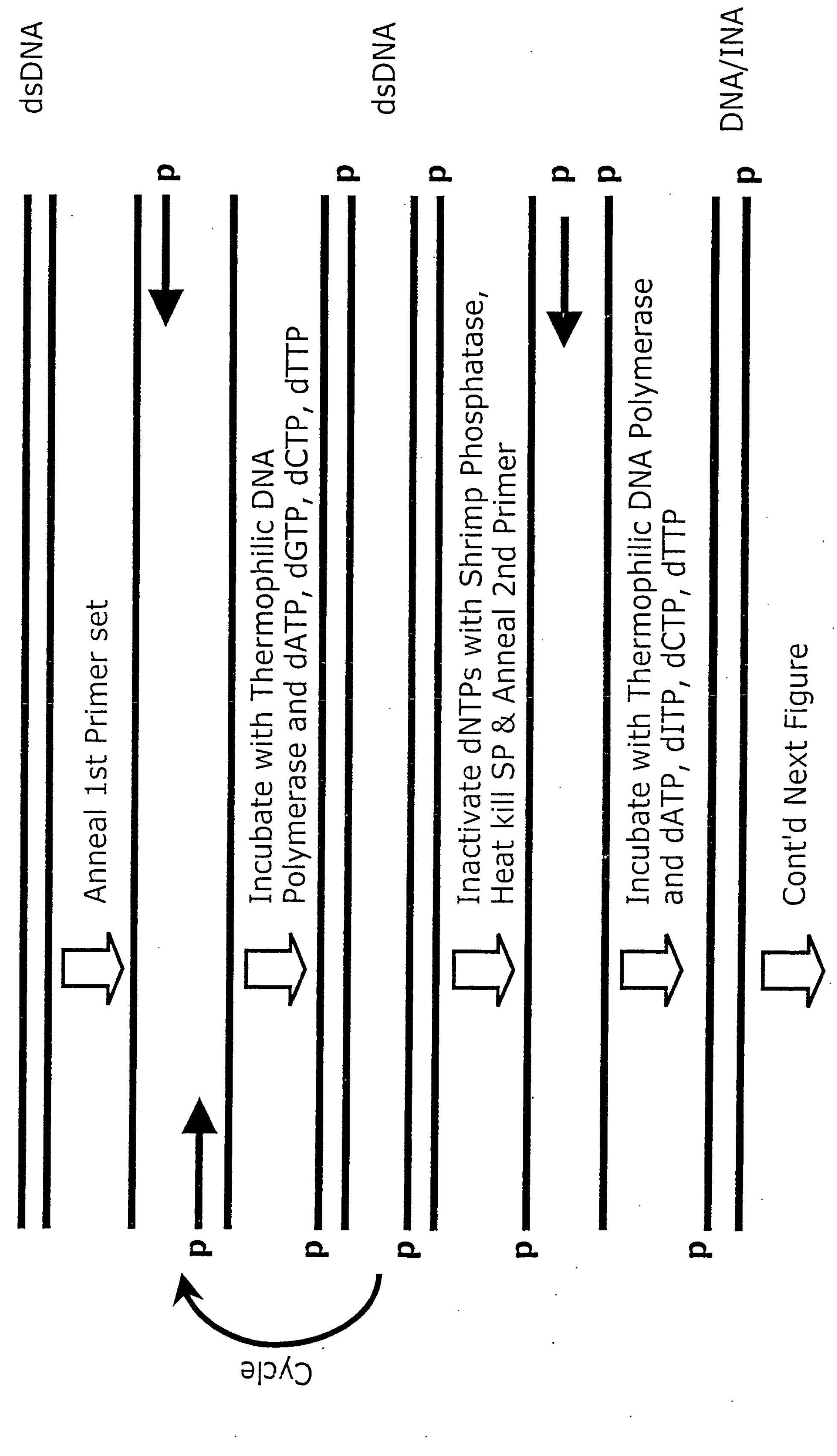
dsDNA
Anneal 1st Primer set
p
p
Incubate with Thermophilic DNA Polymerase and dATP, dGTP, dCTP, dTTP
p
p
dsDNA
p
Cycle
Inactivate dNTPs with Shrimp Phosphatase, Heat kill SP & Anneal 2nd Primer
p
p
p
Incubate with Thermophilic DNA Polymerase and dATP, dITP, dCTP, dTTP
p
p
DNA/INA
Cont'd Next Figure

FIG. 22A

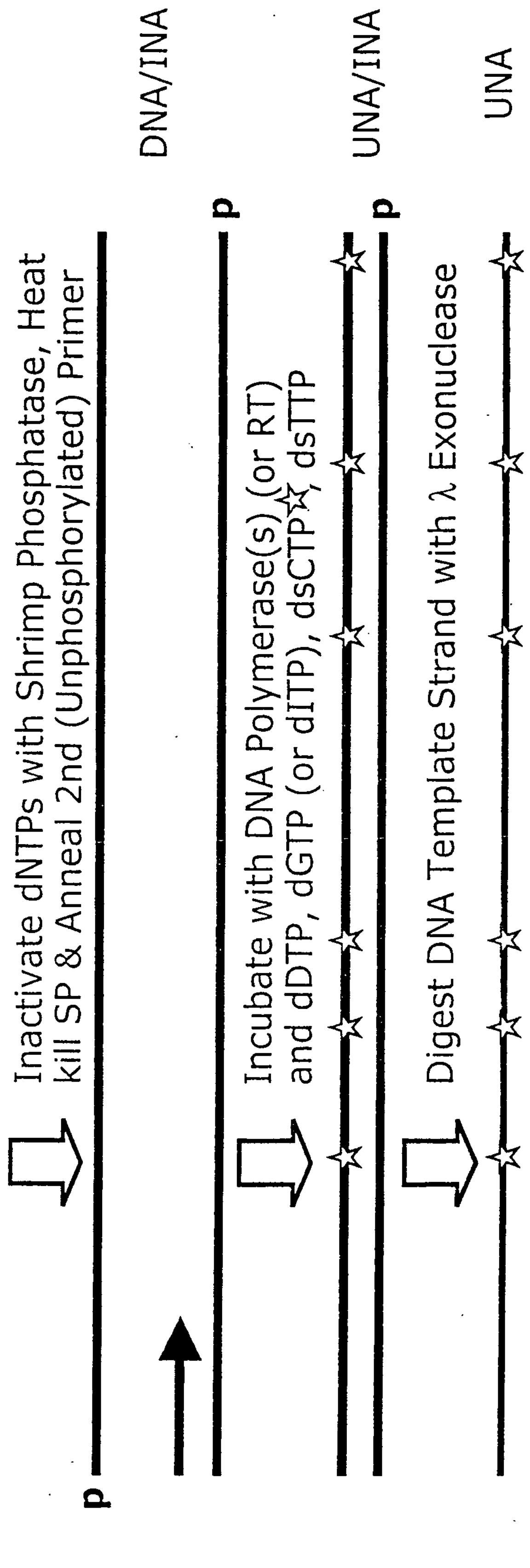
p
Inactivate dNTPs with Shrimp Phosphatase, Heat kill SP & Anneal 2nd (Unphosphorylated) Primer
DNA/INA
p
Incubate with DNA Polymerase(s) (or RT) and dDTP, dGTP (or dITP), dsCTP☆, dsTTP
UNA/INA
p
Digest DNA Template Strand with λ Exonuclease
UNA

FIG. 22B

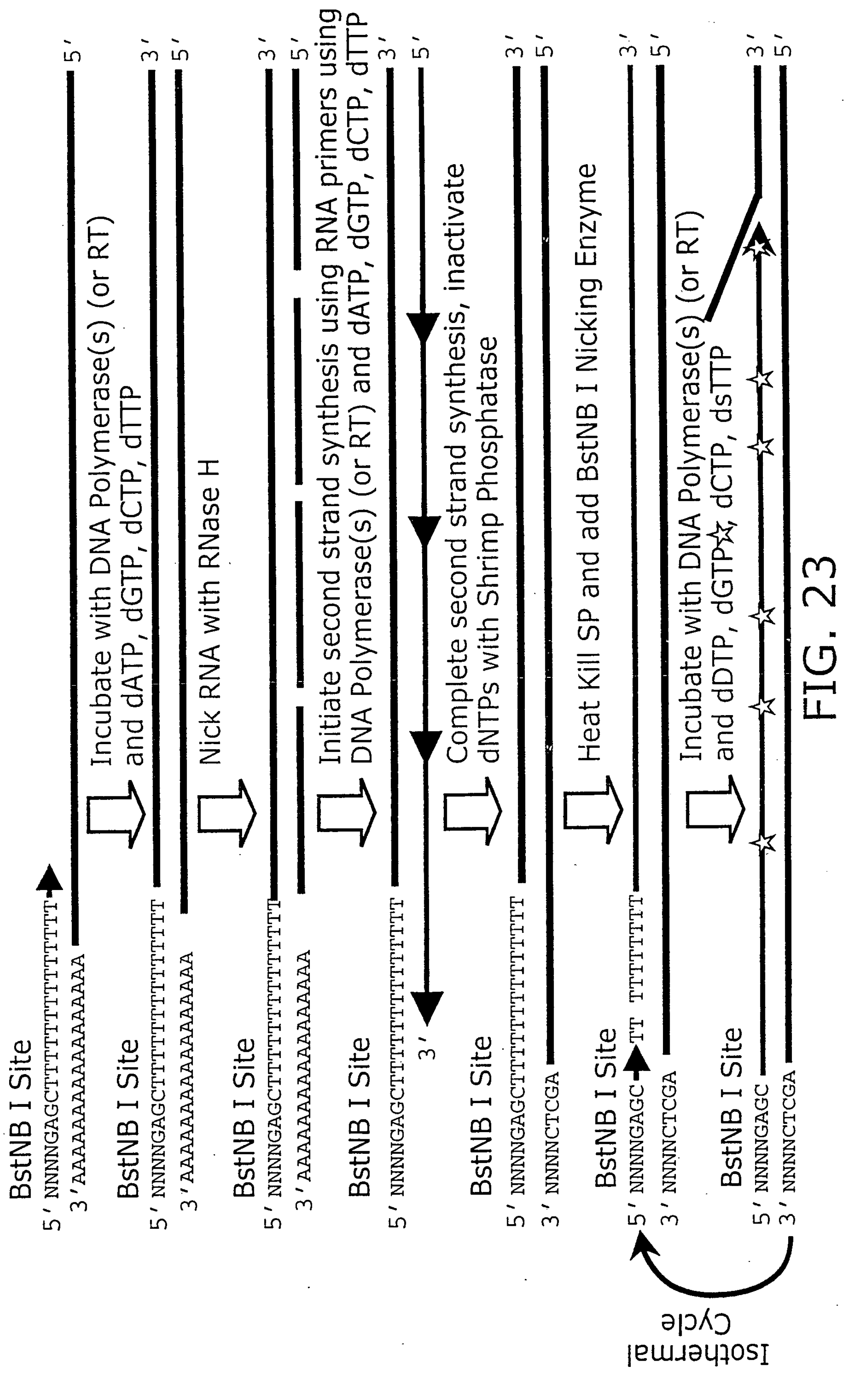
BstNB I Site
5' NNNNGAGCTTTTTTTTTTTTTT
3' AAAAAAAAAAAAAAAA
5'
Incubate with DNA Polymerase(s) (or RT)
and dATP, dGTP, dCTP, dTTP
BstNB I Site
5' NNNNGAGCTTTTTTTTTTTTTT
3' AAAAAAAAAAAAAAAA
3'
5'
Nick RNA with RNase H
BstNB I Site
5' NNNNGAGCTTTTTTTTTTTTTT
3' AAAAAAAAAAAAAAAA
3'
5'
Initiate second strand synthesis using RNA primers using
DNA Polymerase(s) (or RT) and dATP, dGTP, dCTP, dTTP
BstNB I Site
5' NNNNGAGCTTTTTTTTTTTTTT
3'
3'
5'
Complete second strand synthesis, inactivate
dNTPs with Shrimp Phosphatase
BstNB I Site
5' NNNNGAGCTTTTTTTTTTTTTT
3' NNNNCTCGA
3'
5'
Heat Kill SP and add BstNB I Nicking Enzyme
BstNB I Site
5' NNNNGAGC TT TTTTTTT
3' NNNNCTCGA
3'
5'
Incubate with DNA Polymerase(s) (or RT)
and dDTP, dGTP☆, dCTP, dsTTP
BstNB I Site
5' NNNNGAGC
3' NNNNCTCGA
3'
5'
Isothermal
Cycle

FIG. 23

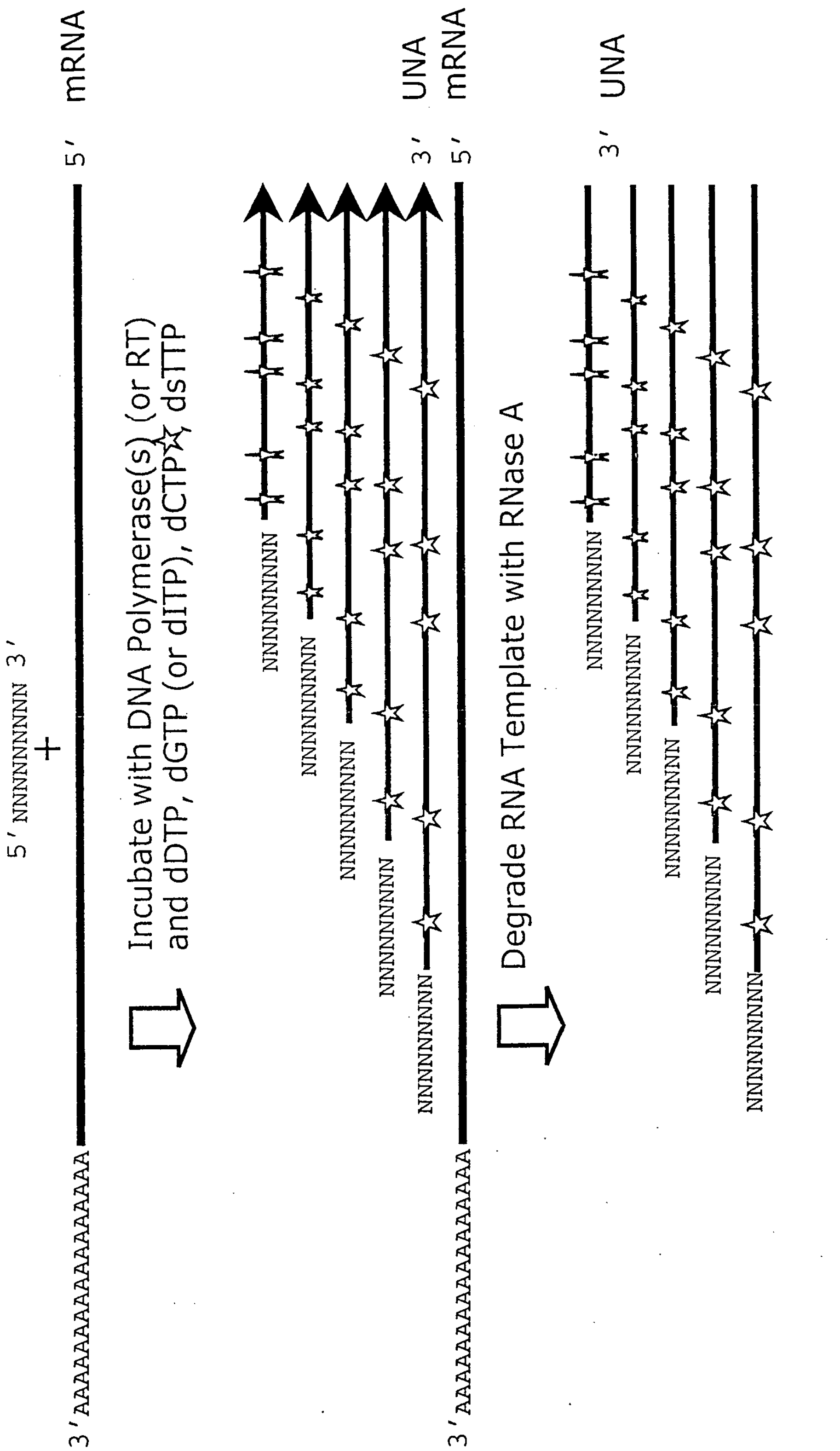
5′ NNNNNNNNN 3′
+
3′ AAAAAAAAAAAAAAA
5′ mRNA
Incubate with DNA Polymerase(s) (or RT) and dDTP, dGTP (or dITP), dCTP☆, dsTTP
NNNNNNNNN
3′ UNA
5′ mRNA
3′ AAAAAAAAAAAAAAAAA
Degrade RNA Template with RNase A
NNNNNNNNN
3′ UNA

FIG. 24

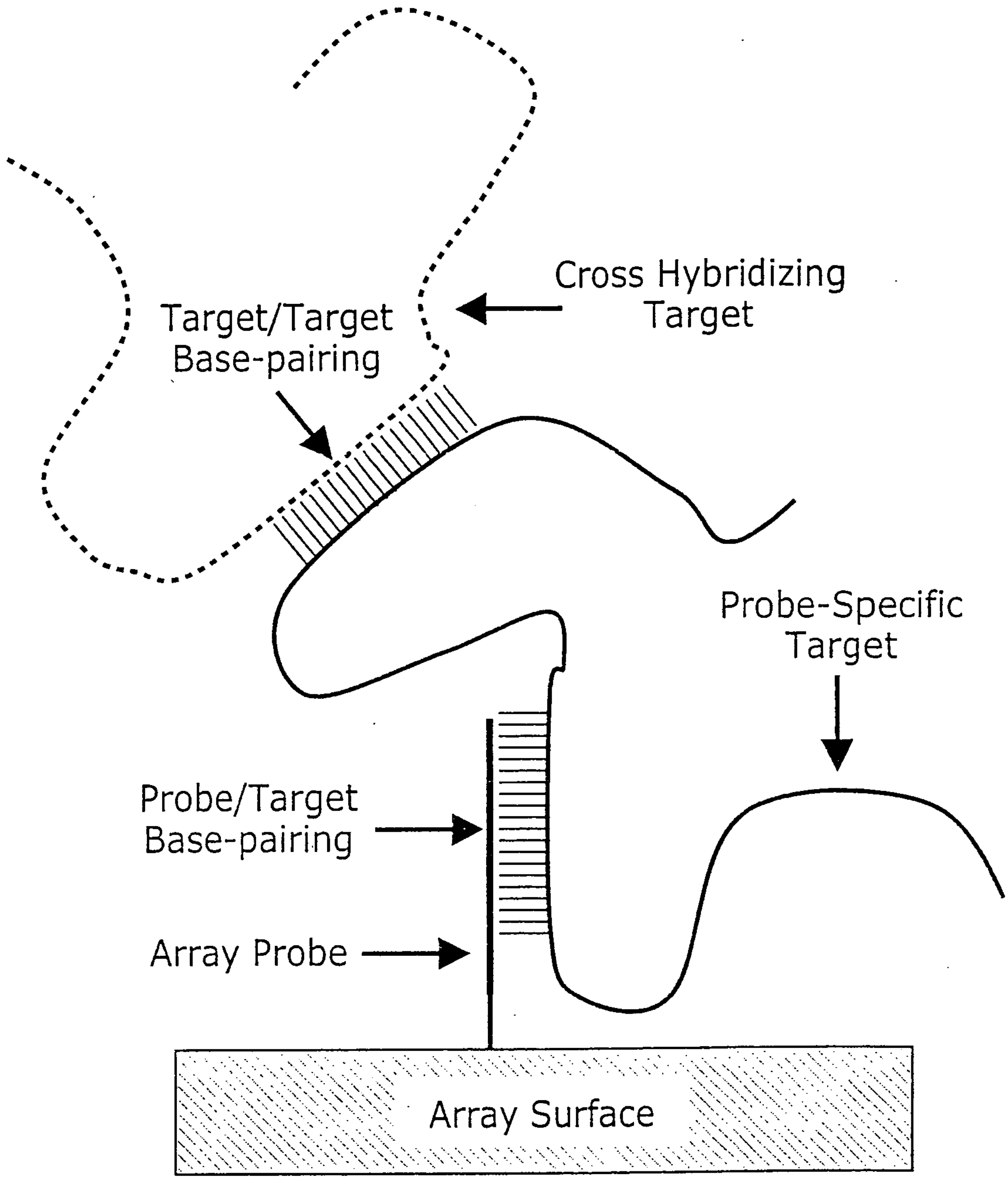
Cross Hybridizing
Target
Target/Target
Base-pairing
Probe-Specific
Target
Probe/Target
Base-pairing
Array Probe
Array Surface

FIG. 25

## HP51 DNA

(Predicted Thermodynamic parameters for 1M NaCl)

G° = -12.3 kcal/mole at 37 °C
H° = -82.5 kcal/mole
S° = -226.3 cal/ (°K ·mol)
**Tm = 91.3°C**

```
5'-CCGATCCATCAA          G
             GTTCAGTCTC A
             ||||||||||
             CAAGTCAGAG A
         3'-CA           A
```

## HP51 UNA

```
5'-CCGATCCATCDD          G
             GSSCDGSCSC D
             |  | | | |
             CDDGSCDGDG D
         3'-CD           D
```

FIG. 26

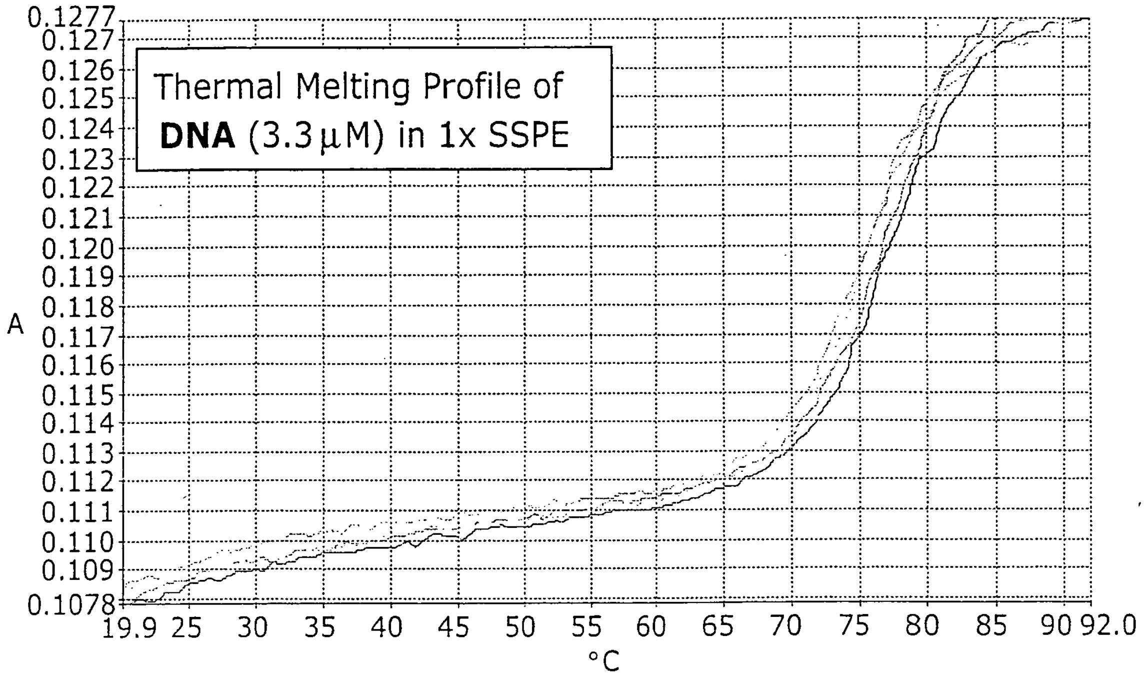


FIG. 27A

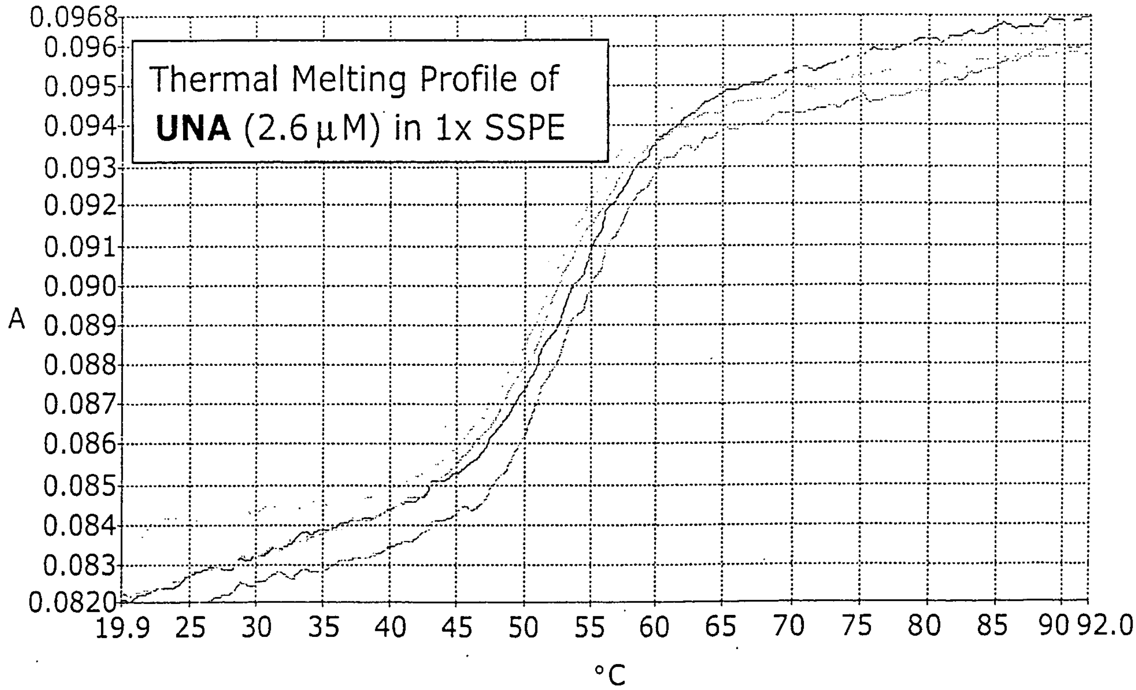


FIG. 27B

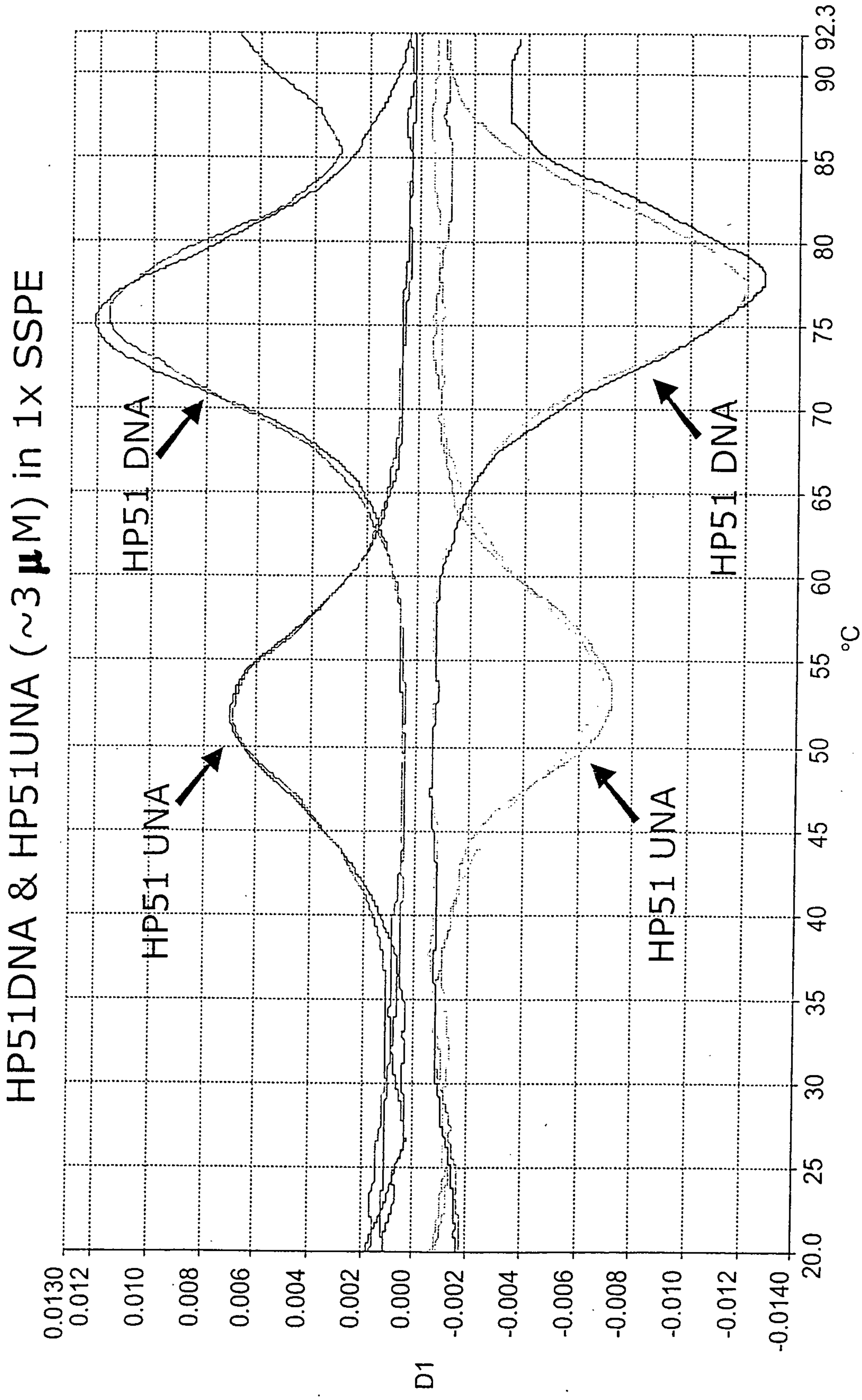
HP51DNA & HP51UNA (~3 μM) in 1x SSPE
HP51 DNA
HP51 UNA
HP51 DNA
HP51 UNA
D1
°C
0.0130
0.012
0.010
0.008
0.006
0.004
0.002
0.000
-0.002
-0.004
-0.006
-0.008
-0.010
-0.012
-0.0140
20.0
25
30
35
40
45
50
55
60
65
70
75
80
85
90
92.3

FIG. 28A

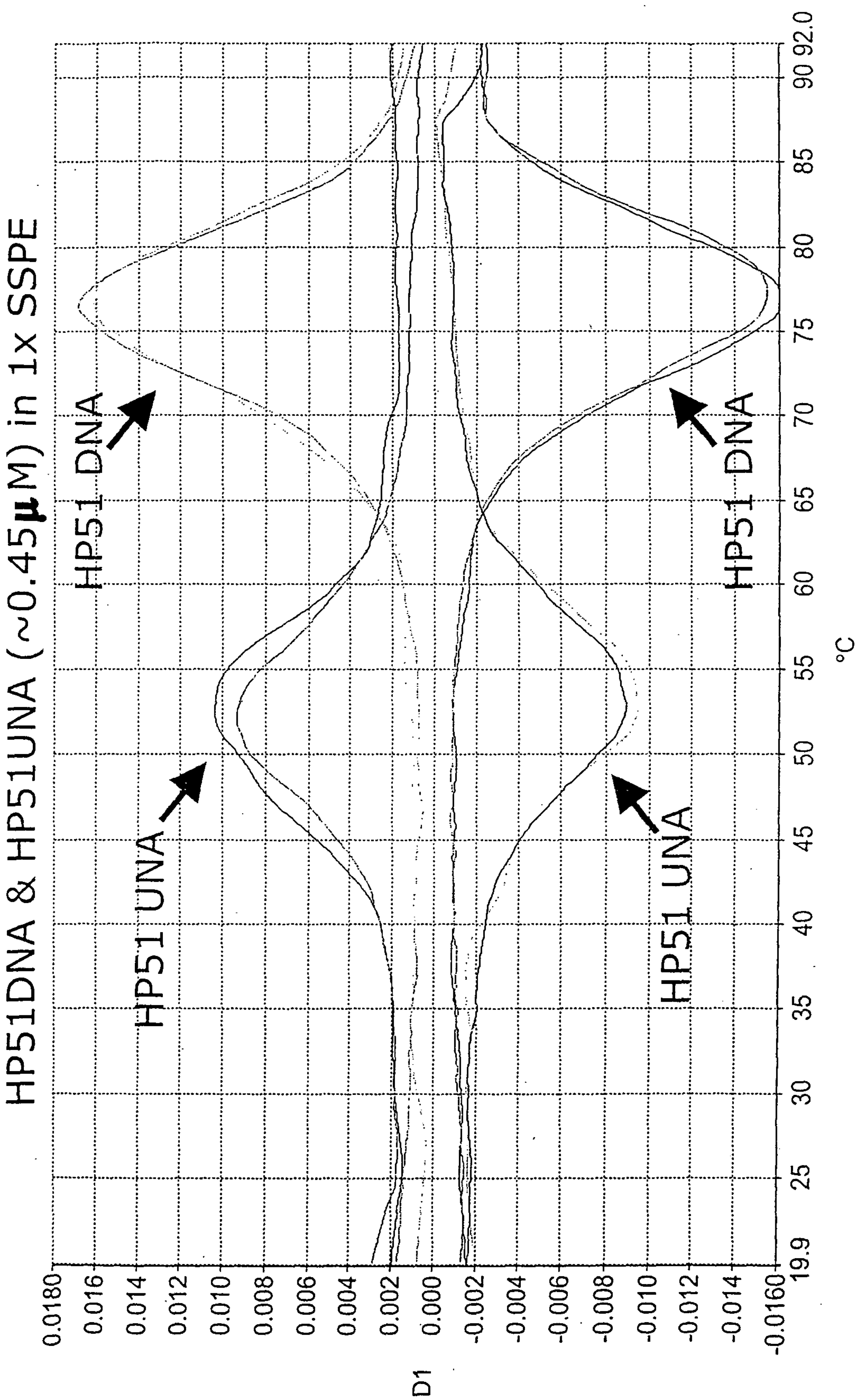
HP51DNA & HP51UNA (~0.45µM) in 1x SSPE
HP51 DNA
HP51 DNA
HP51 UNA
HP51 UNA
D1
0.0180
0.016
0.014
0.012
0.010
0.008
0.006
0.004
0.002
0.000
-0.002
-0.004
-0.006
-0.008
-0.010
-0.012
-0.014
-0.0160
19.9
25
30
35
40
45
50
55
60
65
70
75
80
85
90
92.0
°C

FIG. 28B

## Thermal Melting Profile Summary (~3 µM)

| Sample | Ramp Up 1 Tm | Ramp Up 2 Tm | Ramp Dwn 1 Tm | Ramp Dwn 2 Tm | Average Tm |
|---|---|---|---|---|---|
| HP51 DNA | 75.0°C | 75.5°C | 77.0°C | 78.0°C | **76.4°C** |
| HP51 UNA | 52.0°C | 52.0°C | 53.0°C | 54.0°C | **52.8°C** |
| ΔTm | 23.0°C | 23.5°C | 24.0°C | 24.0°C | **23.6°C** |

## Thermal Melting Profile Summary (~0.5 µM)

| Sample | Ramp Up 1 Tm | Ramp Up 2 Tm | Ramp Dwn 1 Tm | Ramp Dwn 2 Tm | Average Tm |
|---|---|---|---|---|---|
| HP51 DNA | 76.5°C | 76.5°C | 77.0°C | 77.5°C | **76.9°C** |
| HP51 UNA | 53.5°C | 53.5°C | 53.5°C | 54.0°C | **53.6°C** |
| ΔTm | 23.0°C | 23.0°C | 23.5°C | 23.5°C | **23.3°C** |

FIG. 29

```
12mer Primer   5'-TTGTCCACAGTT⇒3'
                  ||||||||||||
Template G     3'-AACAGGTGTCAAGTCAGAGTCTCGGATAGC-5'
Template I     3'-AACAIITITCAAITCAIAITCTCIIATAIC-5'
```

FIG. 30A

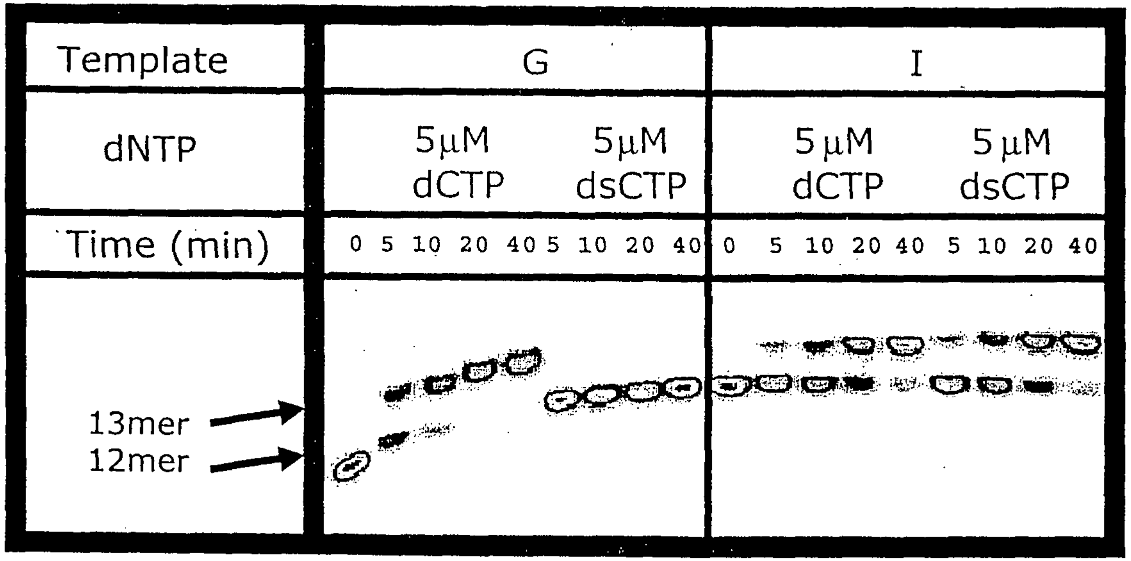


FIG. 30B

**10mer Primer** 5'-CCGATCCATC-3'

**Template G2** 3'-TTTTTGATAGGCTAGGTAGTTCAAGTCAGAGCTTTCTCTGACTTGTG-5'
**Template I2** 3'-TTTTTGATAGGCTAGGTAGTTCAAITCAIAICTTTCTCTIACTTITI-5'

FIG. 30A

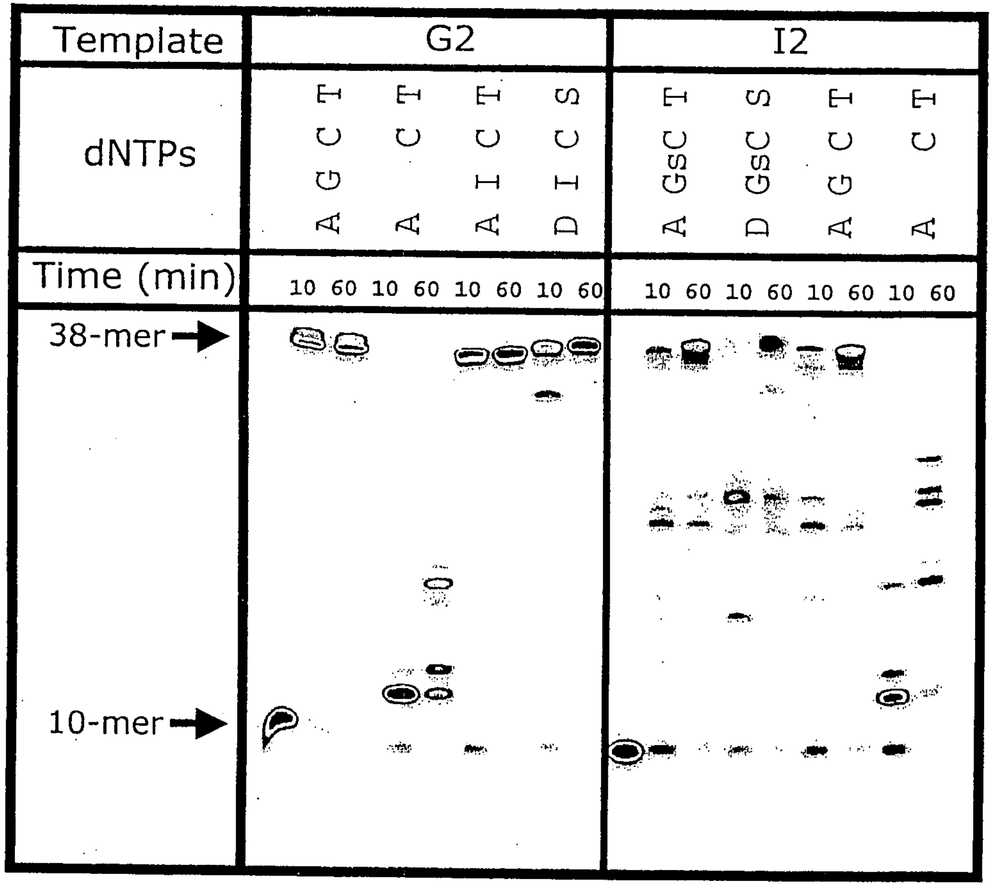


FIG. 30B

**pBp-1 (Penicillin-binding protein 1C from *E. coli*; U88571)**

```
   1 GGCGCAGGAU GGUAcgccgc ucuggcgcuu cgccgaugcu gacggcaucu ggcguuaucc
  61 gguaacaauc gaagauguuu cuccacguua ccuugaagcg cugaucaauu augaagaucg
 121 cugguucugg aagcauccgg gggugaaucc auucucggug gcgcgcgcag cauggcaaga
 181 ucucacuucg ggacggguua uuuccggugg cagcacgcuc acuaugcagg uugcucgucu
 241 gcuugauccu caccccaaaa cauuuggcgg caaaauucgc cagcucuggc gcgcguugca
 301 acuggaaugg caucugucua agcgugaaau ucugaccuug uaucuuaacc gcgcuccguu
 361 uggcgguacg uugcagggga ucggugcggc aaguugggcu uaucucggaa aaucgccugc
 421 gaauuuaagc uauuccgagg cggcaaugcu ggcgguuuug ccgcaagcgc ccagccgucu
 481 ucgcccggau cguuggccgg agcgugccga agccgcgcgu aauaaagugc ucgaacggau
 541 ggccgugcaa ggugugggu cccgugagca gguaaaagag ucaagggaag aacccaucug
 601 gcuggccccc cgacaaaugc cgcaacuggc accgcuguuu ucgcgcauga ugcucgguaa
 661 aagcaaaagc gacaaaauca cuacuacguu ggaugccggu cuucaacgac gucuggaaga
 721 acuggcgcaa aacuggaaag ggcgguugcc accgcgcagc ucacuggcga ugaucguggu
 781 ugaucauacc gauaugcgug uucgcggcug ggugggaucg guugaucuca acgaugauuc
 841 acgcuuuggu cauguugaua uggucaauuc gauccgaucg ccaggaucag ugcucaaacc
 901 guuuguuuau ggucuggcgc uggaugaagg cuugauccac ccggcaucac ugcugcaaga
 961 cgucccccgg cgcaccggug auuaucgacc agguaacuuu gauagcgguu uucauggccc
1021 gaucagcaug agcgaggcgc uggugcgcuc gcugaacuua ccugcugugc aggugcugga
1081 agccuaugga ccgaaacggu uugcggcaaa guuacgcaau guuggauugc cguuauauuu
1141 gcccaacggu gcugcgccga aucuuuCACU Caaaaaaaaa aaaaaaaaaa aaaa
```

**Cab (Chlorophyll A/B-binding protein from *Arabidopsis thalana;* X56062)**

```
   1 cuuccaaguc uaaauucgua uccgccggag uuccacuccc aaacgccggg aauguugguc
  61 guaucagaau ggcugcucac uggaugccug gcgagccacg accagcuuac cuugacgguu
 121 cugcuccugg ugacuuuggg uuugacccac uuggacuugg agaaguucca gcgaaccuug
 181 agagauacaa agagucagag cucauccacu guagaugggc uaugcucgcu guuccuggga
 241 uuuugguacc agaagcauua ggauauggaa acugguuaa ggcucaggaa ugggcagcac
 301 uaccaggggg ucaagccacu uacuugggaa acccaguccc guggguacu uugcccacaa
 361 ucuuggccau ugaguucuua gccauugcau uuguugagca ccagagaagu auggagaaag
 421 acccugagaa gaagaaguac ccgggaggcg cauuugaccc ucuuggauac ucgaaggacc
 481 ccaagaagcu cgaggaauug aaaguuaaag agaucaagaa cgggcggcuu gcgcuguugg
 541 cguuuguagg auucugugug caacagucgg cuuacccggg gacaggacca uuggagaacu
 601 uggcaacuca cuuggcggau ccauggcaca acaacauugg cgauauuguu aucccuuuca
 661 acuaaugaau guaaaaauag aaauaugugu accuuaugag cuuuaugugu aucaaaaaaa
 721 aaaaaaaaaa aaaaaa
```

FIG. 32A

```
HCV  (Nucleotides 1-335 for the Hepatitis C Virus genome; NC_001433)
       1 TTGGgggcga cactccacca tagatcactc ccctgtgagg aactactgtc ttcacgcaga
      61 aagcgtctag ccatggcgtt agtatgagtg ttgtgcagcc tccaggaccc cccctcccgg
     121 gagagccata gtggtctgcg gaaccggtga gtacaccgga attgccagga cgaccgggtc
     181 ctttcttgga tcaacccgct caatgcctgg agatttgggc gtgcccccgc gagactgcta
     241 gccgagtagt gttgggtcgc gaaaggcctt gtggtactgc ctgatagggt gcttgcgagt
     301 gccccgggag gtctcgtaga ccgtgcatca tgagcTCGAC GAGCTCCCAA AAAAAAAAAA
         AAAAAAAAAA AAAA
```

Note: Nucleotides in Capital letters indicate vector sequence.

FIG. 32B

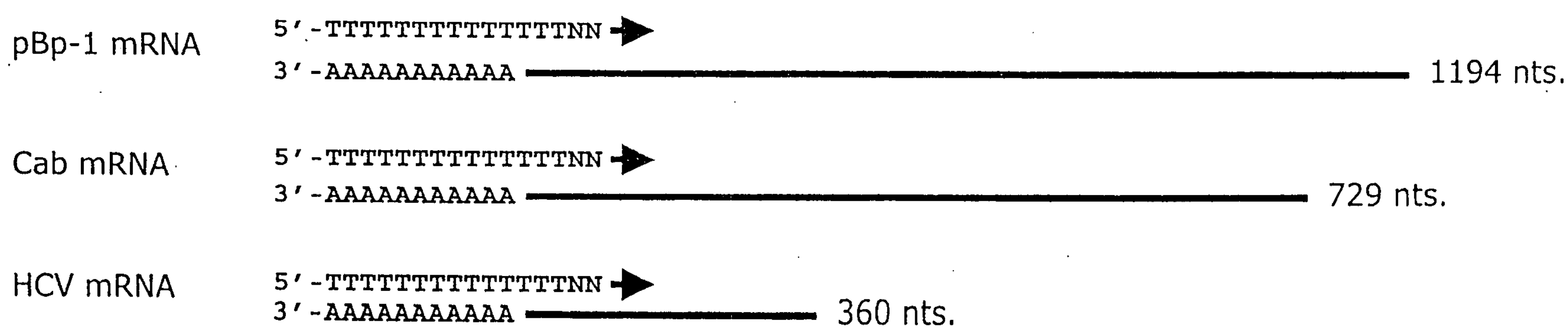


FIG. 32C

| mRNA Template | pBp-1 | | | | Cab | | | | HCV | | | | pBP-1 Cab HCV | | | |
|---|---|---|---|---|---|---|---|---|---|---|---|---|---|---|---|---|
| dNTPs | AGCT | AGCT | DGCS | DICS | AGCT | AGCT | DGCS | DICS | AGCT | AGCT | DGCS | DICS | AGCT | AGCT | DGCS | DICS |
| Primer | - | + | + | + | - | + | + | + | - | + | + | + | - | + | + | + |

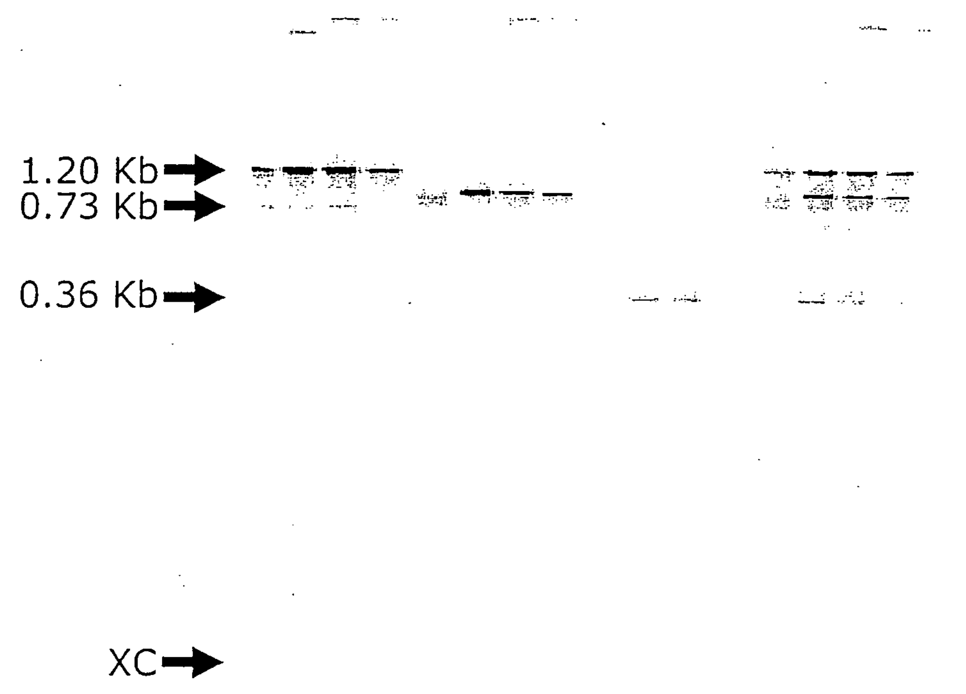


FIG. 33

| mRNA Template | pBp-1 | | | | pBp-1 | | | | pBp-1 | | | |
|---|---|---|---|---|---|---|---|---|---|---|---|---|
| dNTPs | AGCT | | | | DGCS | | | | DICS | | | |
| [dCTP] µM | 25 | | 75 | | 25 | | 75 | | 25 | | 75 | |
| [dCTP-Cy5] µM | 25 | | 25 | | 25 | | 25 | | 25 | | 25 | |
| Time(hr) | 1 | 3 | 1 | 3 | 1 | 3 | 1 | 3 | 1 | 3 | 1 | 3 |

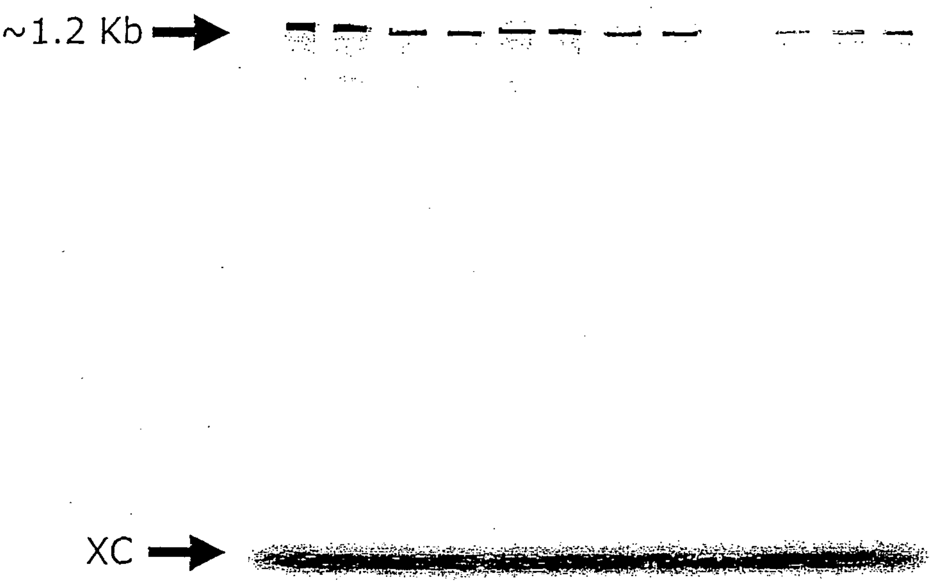


FIG. 34

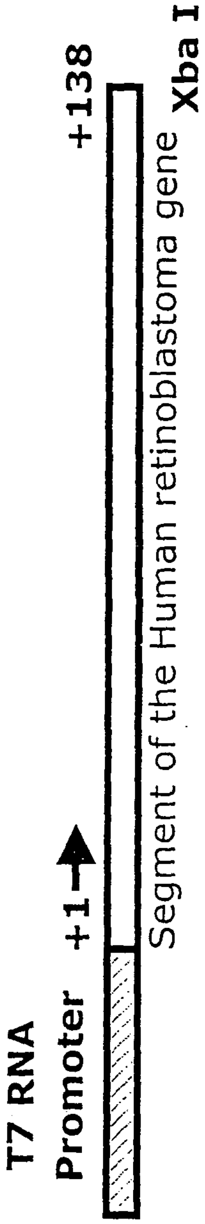


FIG. 35A

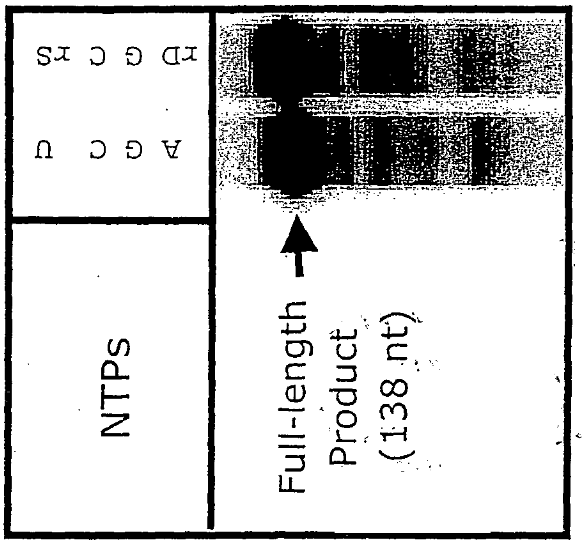


FIG. 35B